(19) Europäisches Patentamt
European Patent Office
Office européen des brevets

(11) EP 0 277 270 B1

(12) EUROPÄISCHE PATENTSCHRIFT

(45) Veröffentlichungstag und Bekanntmachung des Hinweises auf die Patenterteilung: **27.12.1996 Patentblatt 1996/52**

(21) Anmeldenummer: **87111385.8**

(22) Anmeldetag: **06.08.1987**

(51) Int. Cl.$^6$: **A61K 9/10**, A61K 47/00, C07D 213/00, C07D 239/00, C07D 241/00, C07D 209/00, C07D 473/00, C07D 231/00, C07D 233/00, C07D 235/00, C07D 277/00

(54) **N-alkylierte quartäre stickstoffhaltige Heterozyklen, Verfahren zu deren Herstellung und deren Verwendung in Arzneimitteln**

N-alkylated quaternary nitrogen-containing heterocycles, process for their preparation and their use in pharmaceutical compositions

Héterocycles azotés N-alkylés quaternaires, procédé pour leur préparation et utilisation en compositions pharmaceutiques

(84) Benannte Vertragsstaaten:
**AT BE CH DE ES FR GB GR IT LI LU NL SE**

(30) Priorität: **07.08.1986 DE 3626700**

(43) Veröffentlichungstag der Anmeldung: **10.08.1988 Patentblatt 1988/32**

(73) Patentinhaber: **MEDICE Chem.-Pharm. Fabrik Pütter GmbH & Co. KG 58638 Iserlohn (DE)**

(72) Erfinder: **Paradies, Henrich Hasko, Prof. Dr. med.Dr.rer.nat. D-5860 Iserlohn (DE)**

(74) Vertreter: **Reinhard - Skuhra - Weise & Partner Postfach 44 01 51 80750 München (DE)**

(56) Entgegenhaltungen:

| | |
|---|---|
| EP-A- 0 159 638 | DE-A- 2 112 644 |
| DE-A- 2 225 229 | DE-B- 1 293 767 |
| DE-C- 820 949 | FR-A- 2 576 306 |
| GB-A- 870 415 | US-A- 2 485 256 |
| US-A- 2 643 967 | US-A- 2 728 775 |

- **Journal of Pharmaceuticl Sciences, Band 71, Nr. 12, Dezember 1982, Seiten 1313-1318; Am. Pharm. Ass., Washington, US; A. Tsuji et al.: "Effects of surfactants on the aqueous stability and solubility of beta-lactam antibiotics"**
- **Accounts of Chemical Research, Band 12, Nr. 4, April 1979, Seiten 111-118; Am. Chem. Soc., Washington, US, F.M. MENGER: "On the structure of micelles"**
- **Journal of the American Chemical Society, Band 108, Nr. 11, Mai 1986, Seiten 1980-1984; Am. Chem. Soc., Washington, US, F.M. Menger et al.: "A microscopic hydrophobicity parameter"**
- **Journal of the Chemical Society, Faraday Transactions I, Band 78, Nr. 3, März 1982, Seiten 947-955; Chem. Soc., Letchworth, GB; H.D. Burrows et al.: "Dodecyl-pyrazinium halides: their synthesis, characterisation and use in the micellar catalysis of the aquation of tris(1,10-phenanthroline)iron(II-perchlorate in aqueous perchloric acid medium"**
- **Journal of the American Chemical Society, Band 73, Nr. 8, August 1951, Seiten 3959-3963; Am. Chem. Soc., Wahsington, US; G.H. Harris et al.: "Quaternary ammonium salts as germicides. IV. Quaternary ammonium salts derived from substituted pyridines"**
- **Journal of the American Chemical Society, Band 104, Nr. 4, 24. Februar 1982, Seiten 1069-1072; Am. Chem. Soc., Washington, US; E.J.R. SUDHÖLTER et al.: "Rhodopsin reconstitution in vesicles formed from simple, fully synthetic amphiphiles"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 277 270 B1

Printed by Rank Xerox (UK) Business Services
2.13.10/3.4

(Forts. nächste Seite)

- Patent Abstracts of Japan, Band 6, Nr. 15 (C-089), 28. Januar 1982, Seite 91 & JP-A-56 139 462 (KOGYO GIJUTSUIN) 30-10-1981
- Tethahedron Letters, Nr. 26, 1977, Seiten 2277-2280; Pergamon Press, Oxford, GB; V. Gani: "Inhibition de l'hydrolyse alcaline de l'acétate de p-nitrophényle par des micelles hydroxylées dérivées d'un sel de pyridinium"
- Journal of the American Pharmaceutical Association, Band 37, Nr. 1, Januar 1948, Seiten 99-101; Am. Pharm. Ass., Washington, US; M.F. ZIENTY: "Quaternary salts of pyridine-3-carboxamides"
- Journal of Physical Chemistry, Band 74, Nr. 5, 5 März 1970, Seiten 1152-1153; Am. Chem. Soc., Easton, US; J. BAUMRUCKER et al.: "Secondary valence force catalysis. XI. Enhanced reactivity and affinity of cyanide ion toward N-substituted 3-carbamoyl-pyridinium ions elicited by ionic surfactants"
- Chemical Abstracts, Band 72, Nr. 5, 2 Februar 1970, Seiten 251-252, Ref. Nr. 21101k; Colombus, Ohio, US; V.A. VIL'SHANSKII et al.: "Decomposition of cumyl hydroperoxide in aqueous solutions of N-alkylpyridinium compounds" & USP. KHIM. ORG. PEREKISNYKH SOEDIN. AUTOOKISLENIYA, DOKL. VSES. KONF. 3rd 1965 (Pub. 1969), 176-84
- Journal of the American Chemical Society, Band 102, Nr. 7, 26 März 1980, Seiten 2467-2468; Am. Chem. Soc., Washington, US: E.J.R. SUDHÖLTER et al.: "Vesicle formation by two novel synthetic amphiphiles carrying micropolarity reporter head groups"
- Journal of Organic Chemistry, Band 49, Nr. 17, 1984, Seiten 3088-3091, American Chemical Society, Easton, US; F.J.A. HUNDSCHEID et al.: "Aggregation behavior of a series of structurally related single-chain amphiophiles. Structural effects on aggregate morphology as studied by electron microscopy"
- Journal of Heterocyclic Chemistry, Band 14, April 1977, Seiten 321-323, Hetero Corp., Provo, US; H.J.M. DOU et al.: "Alkylation d'anions ambidents en catalyse par transfert de phase. Cas des hydroxypyridines"
- Journal of Organic Chemistry, Band 32, 1967, Seiten 4040-4044, American Chemical Society, Easton, US; G.C. Hopkins et al.: "Alkylations of heterocyclic ambident anions. II. Alkylation of 2-pyridone salts"
- Monatshefte für Chemie, Band 85, Nr. 3, 1954, Seiten 655-659, Springer Verlag, Wien, AT; F. DRAHOWZAL et al.: "Die Beeinflussung der Grignardschen Alkylierungsreaktion durch Pyridinbasen"
- Collection of Czechoslowak Chemical Communications, Band 34, 1969, Seiten 427-441, Academica, Prag, CS; J. PALECEK et al.: "On dihydropyridines. XVI. Reactions of esters of dinicotinic acid with sodium borohydride, lithium aluminum hydride and methylmagnesium iodide"
- Angewandte Chemie, Band 81, Nr. 8, 1969, Seite 293, Verlag Chemie, Weinheim, DE; H.H. PARADIES et al.: "Neue Methode zur Darstellung von Organomagnesium-Verbindungen des Pyridins"
- Angewandte Chemie, Band 77, Nr. 13, 7. Juli 1965, Seiten 557-571, Verlag Chemie, Weinheim, DE; TH. KAUFFMANN: "Die Hetarine"
- Journal of the American Chemical Society, Band 79, 5. März 1957, Seiten 1245-1249, American Chemical Society, Washington, US; H. GILMAN et al.: "Allylmagnesium bromide as a selective nucleophile toward aza-aromatic heterocycles"
- Recueil des travaux chimiques des Pays-Bas, Band 71, 1952, Seiten 798-804, Sigma Chemie Publishing Foundation, Den Haag, NL; J.P. Wibaut et al.: "The preparation of pyridine derivatives from halogenopyridines by means of the Grignard reaction V"
- Tetrahedron, Band 38, Nr. 22, 1982, Seiten 3347-3354, Pergamon Press Ltd.; Oxford, GB; K. TAMAO et al.: "Nickel-phosphine complex-catalyzed Grignard coupling-II"
- Journal of Organic Chemistry, Band 31, 1966, Seiten 3449-3451, American Chemical Society, Easton, US; N. Kornblum et al.: "The reaction of triethyloxonium of alpha-pyridone"
- Helvetica Chimica Acta, Band 57, Nr. 130, Fasc 4, Seiten 1174-1177, Verlag Helvetica Chimica Acta, Basel, CH; H. Balli et al.:"Struktur-reaktivitäts-Beziehungen in Serien quartärer N-heteroaromaten: Aciditäts-Konstanten von 2-Amino-cyclimonium-Ionen als SigmaHet-Konstanten"
- Journal of Organic Chemistry, Band 47, 1982, Seiten 2923-2928, American Chemical Society, Easton, US; N.W. FADNAVIS et al.: "Solubilization of picric acid by reversed micelles of a double-chained N-methylpyridinium chloride amphiphile"
- Chemical Abstracts, Band 95, Nr. 4, 27. Juli 1981, Seite 114, Zusammenfassung Nr. 27174g, Colombus, Ohio, US; & SU-A-819 096 (DONETSK STATE UNIVERSITY; MOSCOW STATE UNIVERSITY) 07-04-1981
- Analytica Chimica Acta, Band 169, März 1985, Seiten 1-29, Elseviers Science Publishers, Amsterdam, NL; E. PELIZZETTI et al.: "Analytical applications of organized molecular assemblies"

- Chemical Abstracts, Band 101, Nr. 26, 24 Dezember 1984, Seite 576, Zusammenfassung Nr. 239534t, Colombus, Ohio, US; W.S. SELIG et al.: "The potentiometric titration of mercury (II) with cetylpyridinium chloride", & Mikrochim. Acta 1984, 2(5-6), 455-61
- Chemical Abstracts, Band 104, Nr. 8, 24. Februar 1986, Seite 682, Zusammenfassung Nr. 61153q, Colombus, Ohio, US; W.S. SELIG et al.: "The microdetermination of cyanide complexes of Group VIII, IB, and IIB elements" & MICROCHEM. J. 1985, 32(1), 18-23

## Beschreibung

Die Erfindung betrifft neue N-alkylierte quaternäre Pyridiniumverbindungen, Verfahren zur Herstellung dieser Verbindungen und Verwendungen hierfür. Weiterhin offenbart die Erfindung pharmazeutische Zubereitungen, die N-alkylierte quaternäre Pyridiniumverbindungen in micellarer oder vesikulärer Form enthalten, in die pharmazeutische Wirkstoffe eingeschlossen sind. Weiterhin offenbart die Erfindung antiphlogistisch wirksame pharmazeutische Zubereitungen, die N-alkylierte quaternäre Pyridiniumverbindungen als eigenständige pharmazeutische Wirkstoffe enthalten.

<u>Stand der Technik:</u>

Bekannt sind die quartären Ammoniumbasen mit tensidartiger Wirkung der allgemeinen Struktur (I)

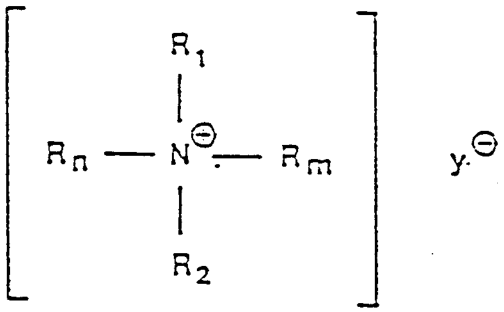

wobei im allgemeinen

| | |
|---|---|
| $R_1$ = | ein Alkylrest mit 1 - 12 C-Atomen |
| $R_2$ = | ein Alkylrest mit 1 - 12 C-Atomen |
| $R_n$ = | ein geradkettiger oder verzweigter Alkylrest mit 10 - 20 C-Atomen oder ein Alkenylrest mit 8 - 10 C-Atomen oder ein 5- oder 6-gliedriger aromatischer Heterozyklus mit einem oder 2 Stickstoffatomen und |
| $R_m$ = | ein geradkettiger oder verzweigter Alkylrest mit 10 - 20 C-Atomen oder ein Alkenylrest mit 8-10 C-Atomen oder ein 5- oder 6-gliedriger aromatischer Heterozyklus mit einem oder 2 Stickstoffatomen |
| $y^-$ = | ein einwertiges Anion |

ist.

Verbindungen dieser allgemeinen Formel sind zum Teil im Tensid-Taschenbuch, herausgegeben von Dr. H. Stache, Carl-Hauser-Verlag, München Wien, 1981, Seite 8/9, beschrieben worden.

Auch sind Teile dieser Verbindung Gegenstand einer europäischen Patentanmeldung, Anmelde-Nr. 83810338.0 vom 24.07.1983, wobei diese Tenside zur Herstellung von unilamellaren Liposomen in wäßriger Phase zur Dispergierung angewendet werden.

Hierbei ist zu berücksichtigen, daß diese bekannten $N^+$-Tenside sowohl micellare als auch vesikuläre Strukturen in wäßrigen und unpolaren Lösungsmitteln bilden (siehe z.B. J. Fendler, Acc. Chem. Res. 1976 <u>9</u>, 153; H.H. Paradies, J.Phys.Chem.1986, <u>90</u>, 5956; H.H. Paradies, 1982, Angew. Chem <u>10</u>, 737; Angew. chem. Int. Ed. Engl., 1982, 21, 765, Supplement 1982, 1670-1681) und hier auch definierte, je nach Aufgabenstellung, bestimmte chemische und biophysische Reaktionen micellar katalysieren.

Dagegen sind kationische Tenside mit einem quartären Stickstoff innerhalb eines $\pi$-Überschuß oder $\pi$ -Mangel-Aromaten, substituiert oder nicht im Kern substituiert, weniger gut bekannt. Es liegen Beschreibungen, z.B. für Hexadecyl-pyridinium-Halogenid (Cetylpyridinium-Halogenid), siehe u.a. Merck-Index 9, Chinolin-, (siehe K. Lindner, in Tenside-Textilhilfsstoffe-Waschrohstoffe (1964, Bd. 1, 987) und für Benzthiazolium-salze (Demande de Brevet Europeen 85400876.0 vom 06.05.1987 sowie BE 660.802 vom 08.03.1965) vor, und zwar mit verschiedenen Alkylkettenlängen und Gegenionen mit Anwendungen in der Photographie und Elektronenübertragung durch geeignete Bildung von Charge-Transfer-Komplexen. Es handelt sich hier allerdings um 2-Methyl bzw. 2-substituierte Benz-thiazolium-Verbindungen mit variabler hydrophober Alkylkettenlänge von 12 - 30 Kohlenstoffatomen am Heterozyklus des ankondensierten Benzolringes.

Für vesikuläre Verbindungen mit einem Pyridinring als Aromaten sind nur 4- bzw. 3,5 Alkyl bzw. Alkoxyl-Verbindungen beschrieben worden, welche am quartären Stickstoff eine Methylgruppe enthalten (siehe z.B. Sudhölter et al. 1982, J. Amer. Chem. Soc. 104, 1069, Sudhölter et al. 1979, J. Amer. Chem. Soc. 102, 2467).

Im "PATENT ABSTRACTS OF JAPAN", Band 6, Nr. 15 (C-089), 28. Jan. 1982, Seite 91, wird 1-Dodecyl-3-hydroxypyridiniumbromid offenbart.

In der US-A-2 728 775 wird 1-n-Cetyl-3-hydroxypyridiniumchlorid offenbart.

Im CHEMICAL ABSTRACTS, Band 101, Nr. 26, 24. Dez. 1984, Seite 756, Zusammenfassung Nr. 239534t, wird Cetylpyridiniumchlorid offenbart.

Im JOURNAL OF THE AMERICAN CHEMICAL SOCIETY, Band 73, Nr. 8, August 1951, S. 3959-3963; Am. Chem. Soc., Washington, USA, wird N-Alkyl-3-carbamoylpyridiniumbromid offenbart.

Im JOURNAL OF THE AMERICAN PHARMACEUTICAL ASSOCIATION, Band 37, Nr. 1, Januar 1948, S. 99-101; Am. Pharm. Ass., Washington, USA, werden N-Alkyl-3-carbamoylpyridiniumsalze mit n=11, 13, 15 oder 17 und $Z^-$ = Chlorid, Bromid oder Jodid offenbart.

Im CHEMICAL ABSTRACTS, Band 72, Nr. 5, 2. Feb. 1970, S. 251-252, Ref.No. 21101k; Columbus, Ohio, USA, wird N-Cetylpyridiniumbromid und N-Cetyl-3-carbamoylpyridiniumbromid offenbart.

In TETRAHEDRON LETTERS, Nr. 26, 1977, S. 2277-2280; Pergamon Press, Oxford, GB, wird 3-Hydroxycetylpyridiniumbromid offenbart.

Es ist eine Aufgabe der vorliegenden Erfindung, neue N-alkylierte quaternäre Pyridiniumverbindungen bereitzustellen. Diese Verbindungen weisen den Vorteil auf, daß aus ihnen micellare oder vesikuläre Strukturen herstellbar sind, in die pharmazeutisch aktive Verbindungen eingeschlossen werden können. Pharmazeutische Zubereitungen, die diese micellaren oder vesikulären Strukturen mit den darin eingeschlossenen pharmazeutischen Verbindungen enthalten, weisen überraschende und nicht vorhersehbare synergistische Wirkungen auf, die zu einer Erhöhung der Wirksamkeit der pharmazeutisch aktiven Verbindungen führen und die Verringerung ihrer Konzentration in der pharmazeutischen Zubereitung ermöglichen.

Diese Aufgabe wird erfindungsgemäß dadurch gelöst, daß N-alkylierte quaternäre Pyridiniumverbindungen gemäß Patentanspruch 1 bereitgestellt werden.

Vorzugsweise Ausführungsformen der Erfindung sind:

1. N-Alkyl-4-hydroxypyridinium der Formel

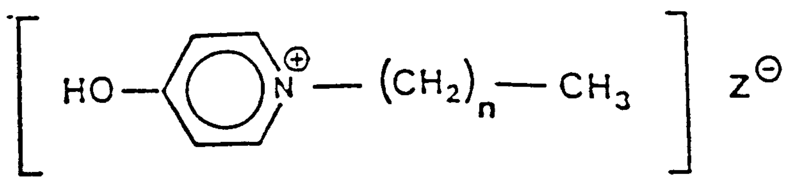

wobei $Z^-$ die vorstehenden 17 Anionen und n = 8 - 20 bedeuten.

2. Hexadecyl-4-hydroxypyridinium der Formel

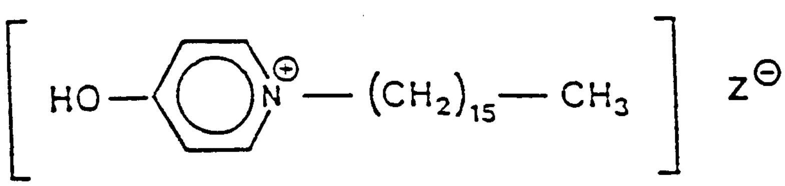

wobei $Z^-$ die vorstehenden 17 Anionen und n = 8 - 20 bedeuten.

3. 4-[1,1bis n-Alkyl (Niederalkyl)] N-Hexadecyl-pyridinium-Verbindungen der Formel

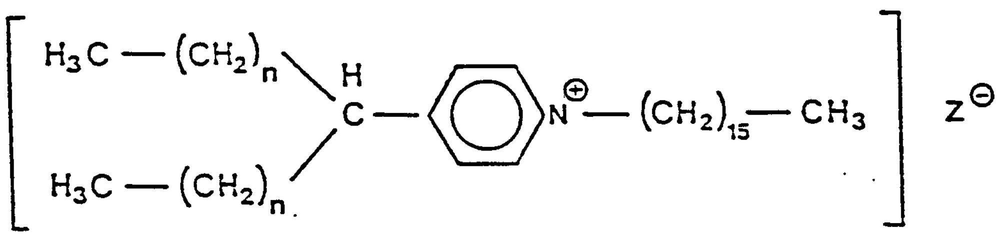

wobei Z⁻ die vorstehenden 17 Anionen und n = 8 - 20 bedeuten.

4. 3,5-bis [(n-Alkyloxy)carbonyl] N-Hexadecyl-pyridinium-Verbindungen der Formel

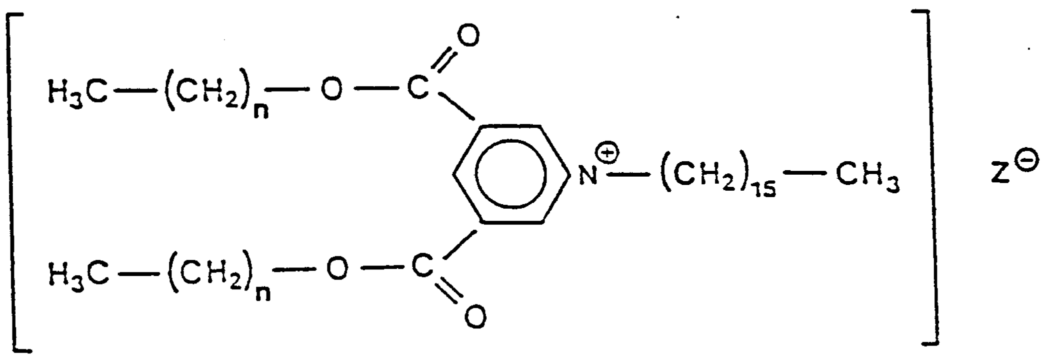

wobei $Z^-$ die vorstehenden 17 Anionen und n = 8 - 20 bedeuten.

Herstellung der erfindungsgemäßen n-alkylierten quaternären stickstoffhaltigen Heterozyklen:

a.) Allgemein zur Herstellung:

Diese kationischen Tenside sind dadurch charakterisiert, daß sie eine sehr kleine Micellbildungskonstante (KMK) von ungefähr $1{,}0 \cdot 10^{-7}$ - $1{,}5x10^{-7}$ Mol/Liter haben, sehr stark antimikrobiell und antifungal wirksam sind, keine Polydispersität in Gegenwart von anorganischen Anionen bzw. Potenzierungsgemischen zeigen, und z.T. selbst mikrobielle Stoffwechselprodukte (Antimetabolite) sind, die nicht toxisch für die Wirtzelle sind.

Die Ausbildung der salzartigen Struktur dieser Klasse von kationischen Tensiden ist u.a. in der Elektronendichte-Verteilung der heteroaromatischen Kerne bzw. in ihrer Basizität, einschließlich des Einflußes der Substituenten, begründet. Eine notwendige Bedingung, welche zur Ausbildung von quartären Salzen dieser sechsgliedrigen heteroaromatischen Klasse führt, besteht darin, daß die Elektronendichte an Stickstoff, welcher quartärniert wird, nach MO-SCF-Rechnungen einen Betrag von -0,08 (z.B. Pyrazin-$N_4$) bis -0,159 (z.B. Imidazol-$N_1$, Purin-$N_7$) haben muß. Diese Stabilität der einzelnen hier beschriebenen heterozyklischen kationischen Tenside wird außerdem noch durch ihre Symmetrie und Kettenlänge der Alkylkette am quartären Stickstoff bestimmt:

Die Länge der Alkylkette am quartären Stickstoffatom bestimmt nicht nur Schmelzpunkt und Hydrophobizität der später in wäßrigen Lösungen gebildeten kationischen Micellen, sondern auch die Ausbeuten nehmen mit zunehmender Kettenlänge ab, während die Reaktionszeiten z.B. in Nitrobenzol oder 2-Ethoxyethanol zunehmen.

Stabile und leicht kristallisierbare Verbindungen werden für $C_{12}$-$C_{18}$ erhalten, wobei das Gegenion $Z^-$ ausnahmslos Bromid und Chlorid ist. Die anderen Verbindungen können leicht aus Aceton oder Chloroform umkristallisiert werden. Die entsprechenden Jodverbindungen sind temperatur- und lichtempfindlich.

b.) Spezielle Herstellung:

1. Die Verbindungen des substituierten Pyridins als sechsgliedriger Heterozyklus lassen sich aus den entsprechenden substituierten Alkylbromiden oder Jodiden in Methanol bei 35°C und substituierten Pyridinen mit einer Ausbeute von 70 % herstellen. Die entsprechenden molaren Mengen des substituierten Alkylbromides, die fast alle im Handel erhältlich sind, aber durch Hochdruckflüssigkeitschromatographie (HPLC) präparativ nachgereinigt werden müssen, werden zunächst in Methanol (10-facher Volumenüberschuß gemessen am substituierten Pyridin)

gelöst, und unter Stickstoff die stöchiometrische Menge des jeweils substituierten Pyridins, das ebenfalls in Methanol gelöst ist, unter Rühren zugetropft. Es wird über 6 Stunden unter Rühren am Rückfluß bei 70°C erhitzt, so daß die Reaktionsausbeute fast quantitativ ist. So ist z.B. die Ausbeute von Hexadecyl-4-hydroxy-pyridiniumchlorid oder Bromid in Methanol als Lösungsmittel 95%, mit Ethanol 80 % und Ether/Ethanol nur 40 %. 3,5 Dihydroxy-dodecylpyridinium-bromid bildet sich quantitativ nach der vorhergehenden Vorschrift aus Dodecyl-bromid und 3,5 Dihydroxy-pyridin in siedendem Chloroform nach 4 Stunden (Schmelzpunkt 180°C) siehe Tabelle 1.

2. Reinigung der entsprechenden Pyridiniumverbindungen. Durch wiederholtes Umkristallisieren aus Gemischen von Methanol/Ether, beginnend mit $^{40}/60$ ($^{v}/v$); $^{50}/50$ ($^{v}/v$) und schließlich $^{90}/10$($^{v}/v$) erhält man die gewünschten Produkte mit konstantem Schmelzpunkt, einheitlich Molekulargewicht und spezifischer oberflächenaktiven Eigenschaften (gemessen durch die Konzentrationsabhängigkeit der Oberflächenspannung). Außerdem zeigen diese Verbindungen die vorne geschilderten typischen $^{1}$H-NMR-Signale. Die zahlreichen $CH_2$-Gruppen und die $CH_3$-Gruppe erzeugen eine deutlich sichtbare Absorptionsschwingung im IR-Spektrum bei 2930 $cm^{-1}$ und 2850 $cm^{-1}$ (Methylengruppe) eine mittelschwache Bande bei 2960 $cm^{-1}$ und eine schwache bei 2870 $cm^{-1}$, welche der Methylgruppe zugeordnet werden kann.

Eine schnelle und quantitative Trennung der n-Alkyl-pyridiniumhalogenide von nicht umgesetzten n-Alkyl-bromiden und Pyridin wird durch präparative Hochdruckflüssigkeitschromatographie auf einer RP18-Säule mit Hilfe des Elutionsgemisches bestehend aus 60 % ($^{v}/v$) Methanol (Ethanol) und Acetonitril 40 % ($^{v}/v$) bei einem isokratischen Säulendruck von 0,53 atm und einer UV-Detektion als Monitor bei 260 nm erreicht.

Herstellung von 3,5 - [(n-Alkoxy)-carbonyl] Pyridinium N-Alkylen:

Die entsprechenden 3,5 bis [(n-Alkoxy)-carbonyl] N-Alkyle des Pyridins können mit großen Ausbeuten aus den entsprechenden Reaktionen der Pyridin - (3,5)-di-karbonsäurechloride mit den entsprechenden N-Alkylalkoholen in Azeton oder Di-isopropyl-keton zu den jeweiligen 3,5 bis [(n-Alkoxy)-carbonyl]-Pyridin-verbindungen mit entsprechender weiterer Reaktion mit den n-Alkyl-Halogeniden, insbesondere von-Alkyljodiden, in Gegenwart von Silberchlorid in 20 % ($^{v}/_{v}$) Ethanol-Wasser hergestellt werden. Das präzipitierte Silberjodid wird abfiltriert und das Lösungsmittel wird unter Wasserstrahlvakuum abgezogen.

Die als Zwischenprodukt entstandenen 3,5-bis [(n-Alkoxy)-carbonyl]-Pyridine sind kristalline Verbindungen und sind u.a. in Ethanol, Propanol und Dimethylsulfoxid beim Erwärmen gut löslich und können daraus umkristallisiert werden. Die farblosen Kristalle kristallisieren entweder mit einem Molekül Wasser, Ethanol oder Dimethylsulfoxid.

Im $^{1}$H-NMR-Spektrum ($CDCl_3/Me_4Si$) beobachtet man in Abhängigkeit von der Wasserkonzentration bei $\approx\delta$ 4,4 ppm ein charakteristisches $H_2O$-Signal.

Die 3,5 bis [(n-Alkoxy) carbonyl] N-Alkyl-Pyridinium Salze werden alle für (n = 8 - 20) aus Aceton/Wasser 40/60 ($^{v/}_{v}$) umkristallisiert (Tabelle 1).

Charaktieristische Signale im $^{1}$H-NMR-Spektrum dieser erfindungsgemäßen Verbindungen in $CDCl_3/Me_4Si$: $\delta$ 0,85 (6H, t, J$\approx$5Hz); $\delta\approx$1,19 - 1,30 (28 H - 72 H, m, für $n_8$ bis $n_{20}$); 4,40 (4 H, t, J < 7 Hz); 5,03 (3H, s) 9,20 (1H, t, J$\approx$1,-8Hz) und 10,00 (2H, d, J, < 2 Hz).

Die Tabelle 1 zeigt die physikalisch-chemischen Eigenschaften dieser Verbindungen in Abhängigkeit vom Anion und die entsprechenden durch inelastische Lichtstreuung gemessenen hydrodynamischen Radien. (Tabelle 2)

Die entsprechenden 4-[1,1bis n-alkyl]-pyridinium-Verbindungen können u.a. fast quantitativ durch Umsatz von 1,1-bis n-Alkyl-bromiden und Pyridin in Gegenwart von $Hg\ Br_2$ und Bromoform im Autoklaven bei 100°C und 20 atm fast quantitativ erhalten werden.

Die erhaltenen 4-[1,1 bis n-alkyl-]-N-Alkyl-pyridinium Salze werden vorzugsweise aus Chloroform umkristallisiert und ergeben farblose Kristalle. Die Kristalle, die nochmals umkristallisiert werden können aus wäßrigen Aceton-Lösungen, enthalten ein Molekül Wasser.

Die charakteristischen $^{1}$H-NMR-Signale dieser Verbindungen in $CDCl_3/Me_4Si$ liegen in den folgenden Ordnungen: $\delta$ 0,94 (6 H, t, J$\approx$4 Hz), 1,20 - 1,29 (14 H - 70 H, für $C_8$ - $C_{20}$, H, m); 2,80 (1H, q, J < 2 Hz, nicht aufgelöst, aber sehr charakteristisch), und 7,7 - 9,5 (4 H,m), sowie die charakteristischen ($CH_2$)-Signale durch die Alkylkette am quaternären Stickstoff.

Des weiteren ergeben sich im Kernresonanzspektrum scharfe Signale mit z.T. schwacher Linienbreite, die einen Hinweis auf die Bildung von Micellen oder Vesikeln mit einem Durchmesser kleiner als 600Å liefern. Die scharfen Signale bei $\delta$ ca. 0,89 ppm ($-CH_3$), $\delta$ ca. 1,28 ppm, ($-CH_2-$) und $\delta$ ca. 3,23 ppm ($N-(CH)_2-$) sind für die Micellen dieser N-Tenside charakteristisch.

Tabelle 1 zeigt einige charakteristische Verbindungen aus der Klasse der erfindungssgemäßen Heterozyklen.

Tabelle 2 zeigt die hydrodynamischen Radien von charakteristischen erfindungsgemäßen Verbindungen in Abhängigkeit von den Anionen $Z^-$.

Tabelle 1

Charakteristische Eigenschaften der N-alkylierten quaternären Pyridinium-Verbindungen

| Nr. | Verbindung | $Z^-$ | Fp°C | Analyse (%) gefunden C | H | N | Z | KMK X $10^6$M |
|---|---|---|---|---|---|---|---|---|
| 1 | N-Hexadecyl-4-hydroxy-pyridinium | Br 1/2 $H_2O$ | 85 | 59,86 | 10,94 | 4,37 | 24,83 | 0,95 |
| 5. | N-Hexadecyl-5-carboxamid-pyridinium | Br | 155 | 52,28 | 11,20 | 7,87 | 21,63 | 0,70 |
| 7. | 3,5-bis[(Hexydecyloxy) carbonyl]N-Hexadecyl-Pyridinium | Cl | 123 | 75,33 | 11,72 | 1,59 | 4,04 | 0,81 |

Tabelle 2

| N-Tensid | $Z^-$ | ⟨$R_H$⟩ | (Å) |
|---|---|---|---|
| N-Hexadecyl-5-Carboxamid pyridinium | $Br^-$ | 120,0 | +/- 15,0 |
| | $Cl^-$ | 55,0 | +/- 10,0 |
| | $Fumarat^-$ | 70,0 | +/- 10,0 |
| | $Maleat^-$ | 120,0 | +/- 11,0 |
| 3,5-Dihydroxy-N-Hexadecyl pyridinium | $Cl^-$ | 1000,0 | +/- 20,0 |
| | $Br^-$ | 1500,0 | +/- 25,0 |
| | $Salizylat^-$ | 250,0 | +/- 20,0 |
| | $NO_3^-$ | 290,0 | +/- 20,0 |
| N-Hexadecyl-5-Carboxyamid-pyridinium | $Br^-$ | 120,0 | +/- 10,0 |
| | $Cl^-$ | 55,0 | +/- 10,0 |
| | $Fumarat^-$ | 70,0 | +/- 5,0 |
| | $Maleat^-$ | 120,0 | +/- 10,0 |
| 3,5-bis [(n-hexadecyloxy) carbonyl] -N-Hexadecyl-pyridinium | $Cl^-$ | 350,0 | +/- 20,0 |
| | $Br^-$ | 400,0 | +/- 20,0 |
| | $Fumarat^-$ | 2500,0 | +/- 100 |
| | $Salizylat^-$ | 1000,0 | +/- 100 |
| N-Octyl-5-Carboxamid-pyridinium | $Br^-$ | 100,0 | +/- 10 |
| | $Cl^-$ | 150,0 | +/- 10 |

Diese erfindungsgemäß bereitgestellten Verbindungen bilden micellare und vesikuläre Strukturen, in die pharmazeutisch aktive Verbindungen eingeschlossen werden können. Pharmazeutische Zubereitungen, die derartige micellare und vesikuläre Strukturen, in die pharmazeutisch aktive Verbindungen eingeschlossen sind, enthalten, weisen überraschende und für den Fachmann nicht vorhersehbare Eigenschaften auf. Insbesondere sind synergistische Effekte erkennbar: die Wirksamkeit der Wirkstoffe, die in den erfindungsgemäßen micellaren und vesikulären Strukturen eingeschlossen sind, wird unerwarteterweise erhöht, wodurch ihre Dosis in der pharmazeutischen Zubereitung bei gleicher Wirksamkeit verringert werden kann. Dies ist insbesondere bei hochtoxischen Wirkstoffen, beispielsweise bei anorganischen Wirkstoffen wie $Hg(Cn)_2$, bedeutsam.

Die folgende Aufstellung gibt eine nicht erschöpfende Zusammenstellung der auf den nachfolgenden Beschreibungsseiten näher ausgeführten Vorteile der Erfindung.

a) Senkung der Hemmkonzentrationen antiviraler Wirkstoffe von Cytarabin, Idoxuridin und Trifluorthymidin um ca. 30% im Gegensatz zur Anwendung pharmazeutischer Zubereitungen, in denen die Wirkstoffe nicht micellar oder vesikulär in die erfindungsgemäßen Pyridiniumverbindungen eingeschlossen sind.

b) Verstärkung der antifugalen Wirkung von beispielsweise Econazol, Clotrimazol, Miconazol um ca. 35 %;

c) Verstärkung der antifugalen Wirkung von Amphotericin B um ca. das Zehnfache,

d) Verstärkung der fungistatischen Wirkung von ZnEDTA, ZnO und $Hg(CN)_2$;

e) Hemmung der Vermehrung von Herpes simplex-Viren in der Zellkultur durch micellaren Einschluß von $Hg(CN)_2$, Zn, $ZnSO_4$ oder ZnEDTA;

f) Hemmung der Thymidilat-Synthetase durch micellar eingeschlossenes $Hg(CN)_2$ und hierdurch bedingte cytostatische Wirkung auf Leukämiezellen;

g) Induktion einer Interferonproduktion in Zellkulturen in Gegenwart von micellar oder vesikulär eingeschlossenem $Hg(CN)_2$.

Die erfindungsgemäß bereitgestellten Pyridiniumverbindungen wirken weiterhin überraschend auf hochreaktive Sauerstoffmoleküle, die in den Zellen entstehen; hierdurch wird ein positiver Einfluß auf entzündliche Prozesse ausgeübt (antiphlogistische Wirkung). Diese therapeutische Wirksamkeit ist eine Eigenschaft der erfindungsgemäßen Pyridiniumverbindungen an sich und ist unabhängig von der Zugabe weiterer pharmazeutischer Wirkstoffe und unabhängig vom Vorliegen der erfindungsgemäßen Verbindungen als micellare oder vesikuläre Strukturen.

Anwendungen:

Diese hier neu beschriebenen Verbindungen, haben überraschenderweise eine sehr kleine KMK im Bereich von $10^{-5}$ - $10^{-7}$ Mol/Liter, die weitgehend unabhängig ist von pH und Ionenstärke (≦ 0,1 M). Außerdem besitzen sie, da sie z.T. selbst Antimetabolite sind, biochemische bzw. pharmakodynamische Wirkung. Sie sind in der Lage die Zellmembranen von neoplastischen Geweben aufgrund ihrer "einwertigen" kationischen Natur zu penetrieren und sodann durch Mechanismen nach Bildung der entsprechenden Nukleoside und Phosphorylierung in die Transkription als auch Translation inhibitorisch einzuwirken. Dies ist insbesondere von Bedeutung für infektiöse Prozesse bakterieller (Prophagen), oder vor allem viraler Natur.

Beachtenswert ist für eine spätere Anwendung dieser $N^+$-Tenside die Steuerung der kolloidchemischen "Aggregate" (Micellen oder Vesikel) durch die Gegenionen $Y^-$ sowohl anorganischer als auch organischer Natur. Diese $N^+$-Tenside können im Gegensatz zu vielen anderen Amphiphilen, welche nicht wasserlöslich sind, aufgrund ihres hydrophoben Effektes "sheet-like" Doppelmembranstrukturen in Wasser oder wäßrigen Lösungen ausbilden. Meistens haben sie zylindrische Form, die allerdings sehr abhängig ist vom Gegenion: bei Anwesenheit von primärem Phosphat ($H_2PO_4^-$) als auch in Gegenwart von Glukonat oder Galaktoronat entstehen hoch orientierte micellare Faserstrukturen, die durch diese Anionen stabilisiert werden. So haben z.B. gelartige Präparationen, helikale Strukturen auf der Basis von micellaren Zylindern.

Wie vorstehend ausgeführt wurde und auf den folgenden Beschreibungsseiten im einzelnen näher ausgeführt werden wird, zeigen die erfindungsgemäßen N-alkylierten quaternären Pyridiniumverbindungen in pharmazeutischen Zubereitungen, in denen sie als micellare oder vesikuläre Strukturen, in die pharmazeutische Wirkstoffe eingeschlossen sind, vorliegen, überraschenderweise synergistische Effekte auf. Diese synergistische Wirkung wurde überraschenderweise auch bei an sich aus dem Stand der Technik bekannten N-alkylierten quaternären Pyridiniumverbindungen beobachtet, die strukturell eng mit den erfindungsgemäß bereitgestellten Pyridiniumverbindungen verwandt sind und die die folgende allgemeine Formel (II) aufweisen:

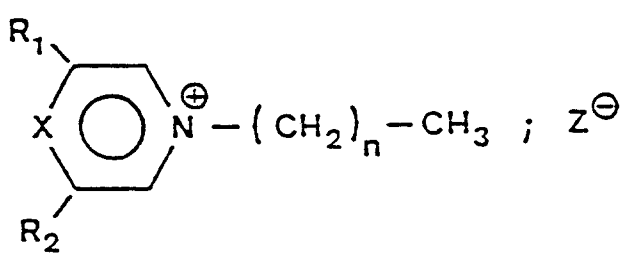

X = CH ; C-OH

X = C - $CH[(CH_2)_n-CH_3]_2$

$R_1$ =

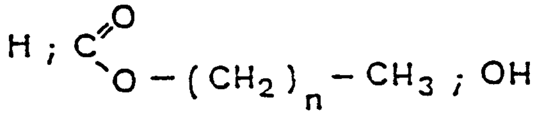

$R_2$ =

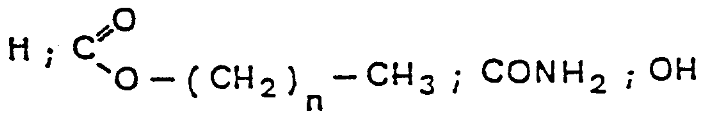

wobei

n = 8 bis 20, insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

bedeuten.

Erfindungsgemäß werden demnach pharmazeutische Zubereitungen bereitgestellt, die N-alkylierte quaternäre Pydridiniumverbindungen der allgemeinen Formel (II) als micellare oder vesikuläre Strukturen enthalten, in die pharmazeutische Wirkstoffe eingeschlossen sind; diese sind in einem Lösungsmittel dispergiert, dessen pH-Wert $\leqq$ 7 ist, wobei die kritische Mizellbildungskonzentration (KMK) im Bereich von 1,0 x $10^{-7}$ bis 1,5 x $10^{-4}$ Mol/l liegt.

Aus der US 2 643 967 sind konzentrierte wäßrige, Antibiotika enthaltende Lösungen bekannt, die Tyrothricin und damit verwandte therapeutisch aktive Verbindungen umfassen und als Lösungsmittel ein wasserlösliches Salz einer quaternären Pyridiniumverbindung enthalten. Beispiel 7 der US 2 643 967 beschreibt die Herstellung einer wäßrigen Lösung von N-(Colamino formyl-methyl)pyridiniumchlorid, die Einstellung eines pH-Wertes auf etwa 7 und die Zugabe einer alkoholischen Lösung von 4 % von Tyrothricin. Nach diesem Verfahren sollen physikalisch und bakteriell stabile, klare, wäßrige Lösungen von Tyrothricin, welches ein wasserunlösliches Antibiotikum ist, hergestellt werden, wobei eine wäßrige oder alkoholische Lösung eines Lösungsvermittlers, nämlich einer quaternären Pyridiniumverbindung, mit der Tyrothricinlösung gemischt wird.

Diese Druckschrift offenbart nicht die Bildung von Mizellen oder von Vesikeln, in welche das Tyrothricin eingeschlossen wird. Die in der US '967 beschriebene Vorgehensweise führt auch nicht automatisch und zwangsläufig zur Bildung von Mizellen oder Vesikeln, in die der Wirkstoff eingeschlossen wird. Hierzu ist, wie erfindungsgemäß im folgenden näher beschrieben wird, beispielsweise eine Ultraschallbehandlung und/oder eine säulenchromatographische Trennung notwendig. Ohne Einhaltung der kritischen Mizellbildungskonzentration im Bereich von 1,0 x $10^{-7}$ bis 1,5 x $10^{-4}$ Mol/l sind keine definierten Mizellen, die pharmazeutisch nutzbar sind, herstellbar. Die US '967 gibt somit dem Fachmann keine Anregung, pharmazeutische Zubereitungen mit micellaren oder vesikulären Strukturen herzustellen, in welche das Antibiotikum eingeschlossen werden kann. Dies ist auch nicht verwunderlich, weil die erst erfindungsgemäß überraschend gefundenen Vorteile, d.h. der durch den Einschluß der pharmazeutisch aktiven Verbindungen in die erfindungsgemäßen micellaren oder vesikulären Strukturen erreichte synergistische Effekt, weder vorbeschrieben noch nahegelegt worden waren.

Auch die übrigen Druckschriften, die auf Seite 2 ff. dieser Beschreibung abgehandelt wurden, beschreiben die erfindungsgemäße pharmazeutische Zubereitung nicht und legen sie auch nicht nahe.

Erfindungsgemäß wurde weiterhin überraschend gefunden, daß die Verbindungen der oben angegebenen allgemeinen Formel (II) eine antiphlogistische Wirksamkeit aufweisen. Hierdurch war es möglich, pharmazeutische Zubereitungen bereitzustellen, die antiphlogistische Wirkungen aufweisen und als Wirkstoff eine oder mehrere Verbindungen der oben angegebenen Formel (II) enthalten. Der Mechanismus, der dieser Wirksamkeit zugrunde liegt, wird im folgenden im einzelnen beschrieben.

Micellen in wässriger Lösung, sowohl nichtionische, kationische und anionische, sind in der Literatur in zahlreichen Veröffentlichungen beschrieben worden. (Mittal, K.L. (1977) Micellization, Solubilization and microemulsions, Plenum Press, New York. - Mittal, K.L. (1979), Solution Chemistry of Surfactants, Plenum Press, New York. - Menger, F.M.,(1977). In Bioorganic Chemistry III. Macro- and Multicomponent Systems (E.E.Van Tanelen, Ed.), Academic Press, New York. - Menger, F.M. (1979a) Acc. Chem. Res. 12, 111-117. On the Structures of Micelles. - J.H.Fendler, E.J. Fendler (1975) Catalysis in micellar and macromolecular Systems, Academic Press.). Ihr Aufbau und ihre galenische, medizinische und technische Verwendung ist Gegenstand zahlreicher Untersuchungen. So ist die antiseptische Wirkung von Cetylpyridiniumchlorid, Benzethoniumchlorid und Benzalkoniumchlorid oder deren Bromide bekannt. Auch, daß sie in geringen Konzentrationen bakterizide Wirkung in vitro gegenüber einer großen Anzahl von grampositiven und gramnegativen Bakterien zeigen, wobei die gramnegativen wesentlich empfindlicher als die grampositiven reagieren. Auch sind bestimmte gramnegative Bakterien resistent gegenüber diesen quartären Ammoniumbasen, z.B. Pseud. cepalia, Mycobakt. tuberculosis.

Normalerweise haben kationische Micellen in wäßriger Phase zusätzlich in ihrem hydrophoben Kern, der weitgehend durch die aliphatische Kette und ihre Länge bestimmt wird, eine hydrophobe-hydrophile Grenzschicht (Sternlayer), die hydratisiert ist und z.T. die Gegenionen beherbergt. Die Größe dieser Grenzschicht liegt im allgemeinen zwischen 7-10 Å. Außerdem sind sie noch mit der Guy-Chapman-Layer von 10-20 Å umgeben, die nicht elektrostatisch gebundene Gegenionen, z.B. $Cl^-$, $Br^-$, $HSO_4^-$ und unstrukturiertes Wasser enthalten. Nur die Konzentrationen der Gegenionen als auch andere Ionen bewirken eine Senkung der kritischen Micellenbildungs-Konzentration (KMK) bei konstanter Temperatur, Druck und chemischen Potentials, wobei die Natur der Gegenionen die Form und Größe der Micellen in wässriger Phase bestimmen können. Dies bewirken allerdings nur die Fraktion von Gegenionen, welche im Stern-layer in der Nähe des quartären Stickstoffs sich befinden.

Die reinen, bislang bekannten kationischen quartären Ammoniumbasen - offiziell auch als Invertseifen bezeichnet - haben nur eine begrenzte und nicht spezifische antimikrobielle Wirkung (siehe z.B. W. Forth, D. Henschler, W. Rummel, Allgemeine und spezielle Pharmakologie und Toxikologie, 4. Auflage. B.I. Wissenschaftsverlag, 1983, S. 616). Daher sind ihre Einsetzbarkeit z.B. als Konservierungsmittel oder als Desinfiziens in den operativen Fächern der Medizin oder auf Infektionsstationen (Antiseptika) begrenzt, trotz ihrer geringen Toxizität. Domagk erkannte 1935 (siehe Wallhäußer, K.H.: Sterilisation, Desinfektion, Konservierung, Keimidentifizierung, Betriebshygiene. 2. Aufl., Thieme, Stuttgart, 1978), daß die quartären Ammoniumbasen nur dann bakterizid wirksam sind, wenn mindestens einer der Substituenten am Stickstoff aus einer linearen Alkylkette mit 8-18 Kohlenstoffatomen besteht, wobei die optimale Kettenlänge bei $C_{12}$-$C_{16}$ liegt. Die bekanntesten Vertreter dieser Stoffklasse sind die Benzalkonium-Salze (Chloride und Bromide). Darüber hinaus sind Hexadecylpyridinium-chlorid und Benzethonium-chlorid bekannt und haben medizinische und pharmazeutische Bedeutung erlangt. Die Wirkung dieser Invertseifen ist bekanntlich stark milieuabhängig. Durch Seifen z.B. wird die Wirkung weitgehend aufgehoben, wie auch im sauren pH-Bereich. Auch Blut, Eiter, Stuhl sowie Schmutz führen gleichfalls zur Inaktivierung. Außerdem haben sie eine Eiweiß fällende Wirkung, die schon bei geringen Konzentrationen der $N^+$-Tenside einsetzt, d.h. im Bereich von 1-2 Gew.% von wäßrigen Lösungen. Bei Konzentration dieser bekannten Tenside, welche nur das 2-3-fache der kritischen KMK betragen, erfolgt zwar keine eiweißfällende Wirkung (Denaturierung), doch eine reversible Inaktivierung von Enzymsystemen und Stützproteinen durch Entfaltung der aktiven drei-dimensionalen Struktur. ("loss of activity through unfolding")

Bekannt ist auch die antibakterielle und unspezifische Wirkung von quartären Ammoniumverbindungen und ihre oberflächenaktive Wirkung, von Dequalinium-acetat, Cetyldimethyl-ammonium-bromid (CTAB) und Hexadecyl-pyridiniumchlorid (CPCl), (siehe z.B. Goodman and Gilman's, The Pharmacological Basis of Therapeutics, EDS. A.G. Goodman, L.S. Goodman, Th.W. Rall, F. Murad, 1985, 7th Edition, Collier, MacMillan Publishing Company, N. Y., S. 971,; Merck Index, 1985). Die micellaren Eigenschaften dieser Verbindungen sind mit ihrer Oberflächenaktivität und antimikrobiellen Eigenschaften in Beziehung gesetzt worden (siehe Attwood, D, and Florence, A.T., Surfactant Systems, Chapman and Hall, London u. New York, 1983). Jedoch kann die unspezifische Oberflächenaktivität dieser quartären aliphatischen und aromatischen Ammoniumbasen nicht a priori als Voraussetzung für die antibakterielle, antifungale uund keratolytische Wirkung angesehen werden, da nichtionische Detergentien, z.B. Brij, Triton X 100, Lubrol etc. nicht reaktiv werden.

Organische quartäre Ammoniumbasen des Typs ($R_n$, $R_1$, $R_2$, $R_m$, $N^+$ )$Y^-$, ($HET{\equiv}N^+$-$(CH_2)_x$-$CH_3$)$Y^-$ und $[H_3C)_3$.C-$CH_2$-$C(CH_3)_2$-$X_1$-$[O$-$(CH_2)_2]_2$-$N^+(CH_3)_2$-$CH_2$-$X_2]Y^-$ sind nur zum Teil bekannt, z.B. Hexadecyltrimethylammoniumchlorid und Bromid (Cetyltrimethylammonium-), Hexadecylpyridiniumchlorid bzw. Bromid (Cetylpyridiniumchlorid) und N,N'-DimethylN- 2 2- 4-(1,1,3,3-tetrymethylbutyl)phenoxyethylphenylmethaniumchlorid (Benzethoniumchlorid, Methylbenzethoniumchlorid) und die Benzalkoniumchloride mit Alkylresten von $C_8H_{17}$ bis $C_{18}H_{37}$. Diese genannten $N^+$-Tenside haben alle eine kleine kritische Micellbildungskonstante (KMK) im Bereiche von $10^{-4}$-$10^{-5}$ Mol, je nach Milieubedingungen, wie z.b. Ionenstärke, Temperatur, Druck und Zusatz von organischen Lösungsmitteln bestimmter Dielektrizitätskonstanten. Der Einfluß eines Anions, $Y^-$, wie fraktionierte Bindungen, Zahl der Anionen an der Micelloberfläche (Sternlayer) und ihr Einfluß auf die geometrische Form der gesamten kationischen Micelle der oben genannten quartären organischen Ammoniumbasen ist bisher wenig untersucht. So auch die Form dieser o.g. Tensde in Gegenwart von Potenzierungsgemischen, wie Glyzerol, Dimethylsulfoxid, Ethanol, Propanol und ihre Stabilität gegnüber Temperatur und Aufnahmefähigkeit von hydrophoben (lipophilen) pharmazeutischen Wirkstoffen. Hier liegen keine quantifizierbare Untersuchungen auch der o.g. $N^+$-Tenside vor.

Der relativ breite und undifferenzierte Wirkungsmechanismus der bereits bekannten quartären organischen Ammoniumbasen und das damit bedingte Anwendungsgebiet als Antiseptika und ihre toxische Wirkung bei höheren therapeutischen Dosen, hat die Anwendung dieser organischen quartären Ammoniumbasen pharmazeutisch beschränkt. Auch ist für 1 Gew.%-ige oder höher wäßrige Lösungen, Cremen und Salben hypersensitive, allergische und topische Irritationen beobachtet worden, so daß ein gezielter therapeutischer Einsatz nur bedingt möglich ist.

Bekannt ist die bakterizide Wirkung von Chlorhexidin bei grampositiven und gramnegativen Bakterien, jedoch resistent gegen gramnegative Bazillen.

Pharmazeutische Präparationen, welche eine gezieltere Therapie mit micellar eingeschlossenen pharmazeutischen Wirkstoffen, z.B. antiviraler, antifungaler, antineoplastischer Natur in therapeutisch wirksamen Dosen und einer geeigneten pharmazeutischen Zubereitung (Galenik) liegen nicht vor.

Ein großer Nachteil der bisher bekannten pharmazeutischen Zubereitungen von quartären organischen Ammoniumbasen, auch in Gegenwart von Potenzierungsgemischen, liegt in der Polydispersität der kolloidalen micellaren Lösungen. Je nach pharmazeutischer Zubereitungsform, pH-Wert, Ionenstärke, Gegenanion Y und Temperatur liegen in einer pharmazeutischen Zubereitung bislang Micellen verschiedener Form und Größe sowie Stabilität und Aufnahmekapazität von pharmazeutischen Wirkstoffen vor.

Im weitesten Sinne versteht man unter Micellen durch Assoziation gebildete Aggregate von gelösten Molekülen. Im engeren, heute hauptsächlich verwendeten Sinn bezeichnet man als Micellen diejenigen Aggregate, die sich aus Tensid-Molekülen in wäßrigen Lösungen oberhalb einer bestimmten Temperatur (Krafft-Punkt) bzw. einer charakteristischen Konzentration bilden. Diese Konzentration nennt man kritische Micellbildungskonzentration (KMK; englisch: critical micellization concentration, cmc). Beim Überschreiten der KMK bleibt die Monomerenkonzentration praktisch konstant und die überschüssigen Tensid-Moleküle bilden Micellen. Diese können in verschiedener Gestalt (Kugeln, Stäbchen, Scheibchen) auftreten, abhängig von der chemischen Konstitution des Tensids sowohl von Temperatur, Konzentration oder Ionenstärke der Lösung. Die Micellen haben charakteristische Aggregationszahlen mit einer meist nur geringen Verteilungsbreite. Das Erreichen der KMK gibt sich durch sprunghafte Änderungen der Oberflächenspannung, (was man zur Messung der KMK ausnützt) des osmotischen Drucks, der elektrischen Leitfähigkeit und Viskosität zu erkennen.

Bei den Micellen handelt es sich um thermodynamische stabile Assoziationskolloide grenzflächenaktiver Stoffe, bei denen die hydrophoben Reste der Monomeren im Inneren der Aggregate liegen und durch hydrophobe Wechselwirkung (van-der-Waals-Kräfte) zusammengehalten werden; die hydrophilen Gruppen sind dem Wasser zugewandt und vermitteln durch Solvatation die Löslichkeit des Kolloids.

Weitere Informationen über Micellen finden sich in Römpps Chemielexikon, 8. Auflage, Franckh'sche Verlagsbuchhandlung Stuttgart, 1985, Seite 2600ff.

Durch die vorliegende Erfindung wird eine pharmazeutische Zubereitung geschaffen, die den Wirkstoff in möglichst stabiler Form enthält und bei welcher der Wirkstoff am Ort des pathologischen Geschehens möglichst schnell und vollständig freigesetzt wird.

Vorzugsweise ist diese pharmazeutische Zubereitung aufgebaut aus einer Micelle, bestehend aus einem kationischen Tensid mit einem einwertigen Anion in einer Menge von 0,01 bis 0,1 Gew.% bezogen auf die gesamte pharmazeutische Zubereitung, und einem hydrophoben pharmazeutischen Wirkstoff in einer Menge von 0,001 bis 0,5 Gew.% bezogen auf die gesamte pharmazeutische Zubereitung, dispergiert in einem Lösungsmittel, dessen pH-Wert $\leqq$ 7,0 ist, in einer Menge von 99,40 bis 99,989 Gew.% bezogen auf die gesamte pharmazeutische Zubereitung, wobei die kritische Micellbildungskonzentration (KMK) im Bereich von 1,0 . $10^{-7}$ bis 1,5 . $10^{-4}$ Mol/Liter liegt.

Diese hier beschriebenen Micellen in wäßriger Phase haben bei einer hydrophoben Kettenlänge von 15-($CH_2$)-Gruppen einschließlich ihres quartären Stickstoffs im aromatischen Gebilde einen Durchmesser von ¯50-100 Å, je nach Natur der Gegenionen.

Weitere vorzugsweise Ausführungsformen der Erfindung:

Während der Gesamtbereich der kritischen Micellbildungskonzentration (KMK) im Bereich von 1,0 . $10^{-7}$ bis 1,5 . $10^{-4}$ Mol/Liter liegt, liegt die KMK vorzugsweise im Bereich von 1,0 bis 8,5 . $10^{-7}$/Liter.

Vorzugsweise ist das kationische Tensid mit dem einwertigen Anion in einer Menge von 0,05 bis 0,1 Gew.%, bezogen auf die gesamte pharmazeutische Zubereitung, enthalten.

Besonders gute Ergebnisse werden erzielt, wenn das kationische Tensid mit dem einwertigen Anion in einer Menge von 0,08 - 0,1 Gew.%, bezogen auf die gesamte pharmazeutische Zubereitung, enthalten ist.

Vorzugsweise ist der hydrophobe pharmazeutische Wirkstoff in einer Menge von 0,06 - 0,05 Gew.%, bezogen auf die gesamte pharmazeutische Zubereitung, enthalten.

Besonders gute Ergebnisse werden erzielt, wenn der hydrophobe pharmazeutische Wirkstoff in einer Menge von 0,001 - 0,005 Gew.%, bezogen auf die gesamte pharmazeutische Zubereitung, enthalten ist.

Vorzugsweise Lösungsmittel sind Wasser oder Wasser + Glycerin oder Wasser + Glycerin + Ethanol.

Vorzugsweise ist das einwertige Anion ein monobasischer oder dibasischer Fettsäurerest.

Vorzugsweise ist das einwertige Anion Acetat, Propionat, Fumarat, Maleat, Succinat, Aspartat oder Glutamat.

Vorzugsweise ist das einwertige Anion ein Zuckerrest.

Vorzugsweise ist das einwertige Anion Glukonat, Galacturonat oder Alginat.

Vorzugsweise ist das einwertige Anion Chlorid, Bromid, Jodid oder Hydrogensulfat.

Vorzugsweise ist der hydrophobe pharmazeutische Wirkstoff ein antimikrobieller Wirkstoff oder ein antifungaler Wirkstoff oder ein antiproliferativer Wirkstoff oder ein antiviraler Wirkstoff.

Vorzugsweise ist der hydrophobe pharmazeutische Wirkstoff eine anorganische Verbindung der Elemente Zink oder Quecksilber oder Wolfram und/oder Antimon. Vorzugsweise ist dabei die anorganische Verbindung $Z_nSO_4$ oder $Z_nO$ oder $Hg(CN)_2$ oder $(NH_4)_{18}$ $(NaW_{21}$ $Sb_9O_{86})_{17}$ oder auch ein Alkali- oder Erdalkalisalz der Phosphonsäure $ROP(O)Me_2$ oder auch ein N-Phosphonoacetyl-1-aspartat.

Vorzugsweise ist der hydrophobe pharmazeutische Werkstoff ein Antibiotikum oder ein antiviraler Wirkstoff oder ein antifungaler Wirkstoff oder ein antineoplastischer Wirkstoff.

Vorzugsweise ist das Lösungsmittel Wasser und/oder Ethanol und/oder Glycerin. Vorzugsweise ist das Lösungsmittel Wasser und/oder Ethanol und/oder Dimethylsulfoxid.

Während der pH-Wert des Lösungsmittels jedenfalls $\leqq 7$ sein muß, ist der vorzugsweise pH-Wert des Lösungsmittels = 5 bzw. in der Nähe von 5.

Die pharmazeutische Zubereitung kann erfindungsgemäß im wesentlichen dadurch hergestellt werden, daß zunächst in einem Reaktionsgefäß das Lösungsmittel vorgelegt wird, dann das kationische Tensid unter Rühren bei Zimmertemperatur zugegeben wird, dann zur entstandenen isotropen micellaren Lösung der hydrophobe pharmazeutische Wirkstoff unter Rühren bei Zimmertemperatur zugegeben wird und zu dessen vollständiger Lösung weitergerührt wird.

Besonders günstige Ergebniss werden mit den kationischen Tensiden der allgemeinen Formel II erzielt, wenn x = 14 ist, d.h., wenn die Alkylkette 15 C-Atome aufweist.

Diese geradkettigen $C_{15}$-Abkömmlinge der N-Tenside zeichnen sich insbesondere aus durch eine einfache chemische Herstellung. Außerdem haben sie überraschenderweise die niedrigste KMK (diese liegt ca. bei $2{,}5 \cdot 10^{-7}$ Mol/Liter). Sie sind weiterhin durch $Z^-$ sehr leicht zu steuern (Form, Molekulargewichtsverteilung, Polydispersität). Außerdem sind sie variabel aufgrund ihrer Größe der Alkylkette und daher bezüglich Aufnahme der pharmazeutischen Wirkstoffe. Schließlich zeichnen sie sich durch leichte Kristallisierbarkeit aus.

Wie bereits erwähnt, ist der Rest Hexadecylpyridinium an sich (als reine chemische Verbindung) bekannt. Nicht bekannt ist der erfindungsgemäße Einfluß des zugehörigen Anions ($Z^-$) auf die Micellengröße und die Form der Micelle.

<u>Herstellungsverfahren für die pharmazeutische Zubereitung:</u>

<u>Generelles zur Herstellung der wässrigen Phase:</u>

Um eine monodisperse, homogene und isotrope wäßrige Lösung der $N^+$-Tenside sowohl in Hinsicht auf Form (sphärisch, oval, langgestreckt) und Größe als auch auf Molekulargewichtsverteilung zu erreichen, müssen die angeführten Lösungen einschließlich ihrer eingeschlossenen hydrophoben pharmazeutischen Wirkstoffe

a. ultraschallbehandelt werden, z.B. bei 100 Watt, eine Minute, eventuell anschließend dann durch b.
b. durch Säulenchromatographie, z.B. auf einer Agarose A 0,5 m, Sepharose 2 B, Sephadex G 200, DEAE-Sepharose C1-6B bei pH 6,0 oder auf einem Ultragel AcA44 (pH 6.0 - 6.5) oder BiO-Gel 1,5 m bei pH $\leqq 7{,}0$ nachgereinigt; oder
c. auf einem linearen Dichtegradienten, z.B. von 1 - 30 Gew.% Subrose, in einer präparativen Ultrazentrifuge in einem SW-27-Rotor bei 25.000 UpM für 12 Stunden zentrifugiert werden. Bei Anwendung einer Zonal-Zentrifugation bei gleichem Gradienten (20°C) können bei 10,000 UpM große Mengen von homogenen Populationen von Micellen und Vesikeln zentrifugiert werden.
d. DEAE-Zellulose-Säulenchromatographie bei pH 5,0 - 6,5 (pH$\leqq$7), z.B. durch einen Phosphatgradienten (linear von 0,01M $KH_2PO_4$/0,01M $K_2HPO_4$, pH 6,5 bis zu 0,05M $KH_2PO_4$/0,05M $K_2HPO_4$ im totalen Elutions-Volumen von 1000 ml) gereinigt werden, bis die entsprechende, gewünschte Population von Micellen bzw. Vesikeln erhalten worden ist.

So ist es möglich, die gewünschten homogenen Populationen von Micellen oder Visikeln einschließlich ihrer eingeschlossenen pharmazeutischen Wirkstoffe, in Form von wiederholbaren konstanten Molekulargewichten und geometrischen Formen zu erhalten. Dies ermöglicht, Monomere der Tenside von den Micellen als auch von nicht eingeschlossenen pharmazeutischen Wirkstoffen quantitativ zu trennen.

<u>Herstellung der homogenen, micellaren Lösung in wäßriger Phase:</u>

Die wäßrige Phase kann reines Wasser sein. In der Regel wird jedoch eine wäßrige Lösung eines Elektrolyten gewählt. Z.B. kann eine wäßrige Lösung aus NaCl oder $CaCl_2$ ($MgCl_2$) verwendet werden. Zusätzlich können aktive pharmazeutische Wirkstoffe von genannter Art eingeführt werden, die dann micellar gelöst werden auch eventuell unter Beschallung.

Die meisten Verfahren sind auf eine Einkapselung hydrophiler Wirkstoffe beschränkt. Mit der vorliegenden Erfindung ist es möglich, hydrophobe z.B. lipophile anorganische ($Hg(CN)_2$) und organische Wirkstoffe (Amphotericin B) micellar einzuschließen. Auch können hydrophile Anionen, die pharmazeutische Bedeutung haben, z.B. Salizylat, je nach Natur des N-Tensides an der externen Oberfläche der Micelle eingeschlossen werden.

Die Erfindung kann dazu verwendet werden, um entweder hydrophile oder lipophile Substanzen oder auch beide Substanzen einzuschließen. Im Falle von hydrophoben Wirkstoffen werden diese dann zusammen mit dem N-Tensid

der Formel I und II in einem Glycerin/Ethanol-Gemisch, bestehend aus 15 Gew.% Glycerin, 15 Gew.% Ethanol und 70 Gew.% Wasser oder 50 Gew.% Ethanol und 50 Gew.% Wasser gelöst, eventuell geschüttelt bzw. ultraschall behandelt und anschließend auf die wäßrige Phase mit einem Gehalt von Glycerin/Ethanol von maximal 15 g Glycerin, 5 g Ethanol in 100 g Wasser verdünnt. Anschließende Gel-Permeationschromotographie oder präparative HPLC- können ungewünschtes Material entfernen und eine homogene, isotrope Lösung liefern. Während hydrophobe Substanzen vornehmlich über eine organische Phase (50 %) und anschließende Verdünnung (Wasser) hergestellt werden, werden hydrophile pharmazeutische Wirksubstanzen vorzugsweise in der wäßrigen Flüssigkeit eingesetzt, die zur Dispergierung der micellaren Lösung benutzt werden. Im Bedarfsfalle können jegliche nicht aufgenommene, aktive Wirkstoffe aus der Dispersion unter Anwendung bekannter Techniken, wie z.B. Dialysieren, Zentrifugieren, Gel-Permeationschromotographie entfernt werden.

Die Form und Größe, als auch der Hydratationsgrad der micellaren Lösungen der N-Tenside ist u.a. abhängig von $Z^-$, weniger von der Struktur des Heterozyklus, wohl aber von der hydrophoben Kettenlänge $(CH_2)_x$. So können z.B. in Gegenwart von $Br^-$ oder Salizylat$^-$ große stäbchenförmige Micellen von Hexadecylpyridinium erhalten werden von einer Größenordnung von L = 10.000 Å und einem Durchmesser von 100 - 500 Å, während man in Gegenwart von Chlorid Micellen der Größenordnung von 50 - 100 Å in wäßriger Lösung erhält. In diesem Falle gibt die Form und Größe der Micelle die Konzentration des zu verkapselnden (micellar) Wirkstoffes an und gestaltet sich somit umgekehrt wie bei Liposomen.

Der Vorteil der Erfindung gegenüber der Verkapselung bei Liposomen liegt

1. in der Dichtigkeit dieser N-Tenside, welche den micellar gebundenen pharmazeutischen Wirkstoff aufgrund der vorne aufgeführten Kräfte nicht freilassen kann und

2. der Steuerung der Form und Größe der Micellen durch $Z^-$, damit Steuerung der Aufnahmekapazität an hydrophoben bzw. hydrophilen Wirkstoffen, ohne großen, tiefgreifenden Einfluß des Heterozyklus auf die KMK.

Die erfolgte Bildung der kleinen und großen Micellen der N-Tenside in wäßriger Phase lassen sich anhand von physikalischen Meßmethoden nachweisen, z.B. mit gefriergetrockneten Proben ("freeze fracture") im Elektronenmikroskop oder durch Röntgenkleinwinkel-Streuung, durch dynamische Lichtstreuung, durch Kernresonanzspektroskopie ($^1H$, $^{13}C$ und $^{31}P$), als auch durch Transmissionselektronenmikroskopie. Abb. 2 zeigt z.B. elektronenmikroskopische Aufnahmen von micellar eingeschlossenem $Hg(CN)_2$ in Hexadecylpyridiniumchlorid.

Im Kernresonanzspektrum ergeben sich scharfe Signale mit schwacher Linienbreite, welche einen Hinweis auf die Bildung von Micellen mit einem Durchmesser kleiner als 600 Å liefert. Scharfe Signale bei δ ca. 0,89 ppm ($-CH_3$), δ ca. 1,28 ppm ($-CH_2-$) und δ ca. 3,23 ppm ($-N-(CH_3)_2$ sind z.B. für die Micellen der N-Tenside der allgemeinen Formel I und II charakteristisch. Für eingeschlossene Wirkstoffe in diesen Micellen der N-Tenside ist ein Methylsignal bei δ ca. 0,87 - 0,89 ppm charakteristisch, das jedoch in einem Triplett aufgespalten ist und eine wesentlich geringere Linienbreite hat als das Methylsignal, das als Singlett vorkommt bei δ = 0,89 ppm, welches allerdings nur von der Micelle herrührt.

Diese wäßrigen Phasen, welche die erfindungsgemäß erhaltenen Micellen mit eingeschlossenen Wirkstoffen enthalten, sind Verabreichungssysteme, die sich gegebenenfalls nach Konzentrierung, z.B. durch Ultrafiltration, Ultrazentrifugation oder Lyophilisieren mit anschließendem Auflösen in einer wäßrigen Phase, für die orale (p.o.) oder topische Verabreichung eignen.

Bei oraler Verabreichung können die micellar gebundenen pharmazeutischen Wirkstoffe der N-Tenside der wäßrigen Phase mit pharmazeutisch unbedenklichen Verdünnungsmitteln oder Trägern oder mit üblichen Zusätzen, z.B. Farbstoffen oder Geschmackstoffen, vermischt und als Sirup oder in Form von Kaseln verabreicht werden.

So besteht eine homogene isotrope micellare wäßrige Lösung aus einem N-Tensid der Formel II und I mit einem antiviralen Wirkstoff, insbesondere mit $Hg(CN)_2$, oder $ZnSO_4$, ZnEDTA, Idoxuridin, 5-Ethyl-2'-desoxyuridin oder Trifluorthymidin, Amantadin, Rimantadin (α-Methyladamantan) und Viderabin (9-β-Arabino ⟨1,4⟩adenin) und Ribavirin (1-β-D-Ribofuranosyl-1,2,4-triazole-3-carboxamid) als auch mit 2,6-Di-amini-kuban 1,1':3,3'-Bis-cyclobutan oder einfach substituierte 2,6-di-amino-Verbindungen ($CF_3$,Cl,$OCH_3$) in Gegenwart oder Abwesenheit von Glycerin/Ethanol dispergiert (20°C; Ionenstärke <0,2 M).

Eine homogene, isotrope micellare wäßrige Lösung besteht aus einem N-Tensid der Formel II und/oder Formel I vorzugsweise mit einem antifungalen Wirkstoff, vorzugsweise mt 5-Fluorcytosin, Clotrimazol, Econazol, Miconazol oder Oxyconazol (Z-Form) als auch mit Amphotericin B, Nystatin und ZnO.EDTA als anorganischer antifungaler Wirkstoff, sowie $Hg_2(CN)_4$ ($Hg(CN)_2$ liegt hier als Polymer vor, wobei das Dimere das zugrundeliegende Bauprinzip ist (in wäßriger Lösung) dispergiert.

Eine homogene, isotrope wäßrige Lösung besteht aus einem N-Tensid der Formel I und/oder der Formel II vorzugsweise mit einem antineoplastischen Wirkstoff, insbesondere 5-Fluorcyanid, $Hg(CN)_2$.4 (Ascorbat oder Acetylacetonat), Azauridin, Cytarabin, Azaribin, 6-Merkaptopurin, Desoxycoformycin, Azathioprin, Thioguanin, Vinblastin, Vincristin, Daunorubicin, Doxorubicin in Gegenwart oder Abwesenheit von Glycerin/Ethanol dispergiert.

Eine homogene, isotrope wäßrige Lösung besteht aus einem N-Tensid vornehmlich der Formel II oder der Formel

I vorzugsweise mit Aminoglykoside wie z.B. Canamycin, Gentamycin, Neomycin etc. oder Tetrazyklinen, Chloramphenicol oder Erythromycin als bakteriostatische (grampositive) oder Clindamycin (gegen nicht sporenbildende anaerobe Bakterien) oder Rifampicin als bakteriziden, als auch Bacitracin, Tyrotricin und Polymycine in Gegenwart oder Abwesenheit von Glycerol/Ethanol dispergiert.

Die homogene Mischung kann auch anschließend in Gelen auf der Basis von Alginat, Hydrogelstrukturen wie z.B. Sephadex Agarose, Propyl-zellulose, Propyl-hydroxy-zellulose, in Gegenwart von DMSO, Glycerol dispergiert werden, wobei die pharmazeutischen Wirkstoffe micellar bei den gewünschten Konzentrationen enthalten sind.

Man dispergiert z.B. durch Schütteln, Rühren der wäßrigen Phase oder durch Ultraschallbehandlung, welche die zuvor hergestellte homogene isotrope Mischung enthält. Die Bildung der micellaren Strukturen mit den eingeschlossenen Wirkstoffen, pH ≦ 7,0, 20°C, findet spontan, d.h. ohne große zusätzliche Energiezufuhr von außen und mit großer Geschwindigkeit statt. Die Konzentration an N-Tensid der Formel I und II und Einschlußverbindung kann erhöht werden, wenn die KMK um mindestens das Zehnfache überschritten wird in der wäßrigen Phase bei konstantem chemischen Potential und Temperatur.

Die KMK ist eine variable Größe für die Menge der Monomeren der N-Tenside, welche man in einem bestimmten Volumen Wasser unter Verwendung von pH-Schwankungen ≦ 7,0 lösen kann. Die KMK, die erfindungsgemäß nicht sehr abhängig ist von der Natur des Gegenions, welches nur die Form bestimmt, da weit oberhalb der KMK gearbeitet wird, kann durch elektrochemische Verfahrenn (Leitfähigkeit, Potentiometrie) durch Messung der Überführungszellen im Zusammenhang mit den Gegenionen, der Oberflächenspannung, Dampfdruckerniedrigung, Gefrierpunkterniedrigung und osmotischer Druck, Messung der Dichte, des Brechungsindex, der elastischen und unelastischen Lichtstreuung (Diffusionskoeffizienten, Stokes' Radius) und der Viskosität, sowie durch Gelfiltration und Röntgenkleinwinkel-Streuungsmessungen bestimmt werden. Nanosekunden-Fluoreszenz als auch die Messung der Fluoreszenspolarisation lassen zusätzlich Bestimmungen der Lage der eingeschlossenen pharmazeutischen Wirkstoffe durch die N-Tenside der Formel I und II zu, z.B. durch ZnEDTA oder $Hg(CN)_2$ als Quencher und Amphotericin B als Verstärker. Positronium-Vernichtungs-Messungen an diesen beschriebenen micellaren Lösungen mit den eingeschlossenen Wirkstoffen lassen ebenfalls Aussagen über die Menge (Konzentration) des eingeschlossenen pharmazeutischen Wirkstoffes in Abhängigkeit von der Natur und Konzentration von $y^-$ zu.

Wäßrige Phasen mit einem pH-Wert >7,0 werden nach der Dispersion zentrifugiert. Die Neutralisation auf pH ≦ 7,0 ist notwendig, um eine Zerstörung des Heterozyklus in Formel II als auch des Wirkstoffes und/oder der Micellen unter basischen Bedingungen zu verhindern. Physiologisch gängige und verträgliche Säuren sind z.B. verdünnte wäßrige Mineralsäuren und Salzsäure, Schwefelsäure oder Phosphatsäure oder organische Säure, z.B. Niederalkansäuren wie Essigsäure oder Propionsäure. Meistens reagieren die wäßrigen Phasen der kationischen N-Tenside der Formel I und II sauer bis neutral, können aber auch durch Sörensen-Puffer oder organische Inertpuffer, wie z.B. HEPES, MOPS oder MES auf pH-Werte zwischen 3 und 7,0 genau eingestellt werden.

<u>Herstellung der homogenen, micellaren Lösung in nichtwässrigen Phasen:</u>

Die Auswahl der betreffenden Lösungsmittel ist von der Löslichkeit des betreffenden pharmazeutischen Wirkstoffes darin abhängig. Geeignete Lösungsmittel sind z.B. Methylenchlorid, Chloroform, Alkohole, z.B. Methanol, Ethanol und Propanol, Niederalkancarbonsäureester, (Essigsäure-Ethylester), Ether oder Mischungen dieser Lösungsmittel. Nach Herstellen der micellaren Lösung und Zugabe des pharmazeutischen Wirkstoffes, gelöst im organischen Lösungsmittel, wird das organische Lösungsmittel entweder durch die vorne erwähnten Verfahren a. - d. oder durch Abblasen mit Inertgas, z.B. Helium oder Stickstoff, entfernt.

<u>Beispiel 1:</u>

Man löst 10 mg Hexadecylpyridinium-chlorid in 100 ml einer Wasser/Ethanol-Mischung (85:15; $^w/w$) bei 25°C unter Rühren und addiert 10 ml Glycerol. Der pH-Wert sollte um 6,5 sein, kann jedoch mit HCl auf diese oder einen anderen pH-Wert (=7,0) eingestellt werden. Diese Lösung wird dann auf 20±0,01°C abgekühlt, anschließend ultraschall-behandelt (Bronson-Sonifier, Mass.,U.S.A.) für zwei Minuten bei 10 Watt. Die Bildung der Micellen wird durch Messung des Diffusionskoeffizienten mittels inelastischer Lichtstreuung bestimmt und dann mach der Beziehung

$$D^{o}_{20,W} = \frac{k_B \cdot T}{6\pi\eta_o \cdot R_H} \quad (1)$$

$T = t° + 273$

$\eta_o$ = Viskosität des Lösungsmittels

$k_B$ = Boltzmann-Konstante

$D^{o}_{20,W}$ = Diffusionskonstante

der Stokes' Radius ($R_H$) berechnet. In Gegenwart von $Cl^{\ominus}$ als $Y^{\ominus}$ sollte er nicht größer als 50 Å, von $Br^{\ominus}$ nicht größer als 1000 Å sein. Zur Bildung von Mikroemulsionen von Micellen bestimmter Größe dispergiert man bei Raumtemperatur (20°C) einen filmartigen Rückstand, den man durch Eindampfen der oben genannten Lösung, im Rotationsverdampfer erhält, in 1/10 des ursprünglichen Volumens durch 10-minütiges Schütteln. Man erhält eine leicht opalisierende, wässrige Lösung. Zum Einschluß eines pharmzeutischen Wirkstoffes, z.B. 5-Fluorurazil, Cytarabin oder Idoxuridin können, diese in Wasser schwerlöslichen Substanzen direkt, d. h. in fester Form oder als wässrige Suspension eingegeben werden. So gibt man z. B. Trifluoridin, 1.0 - 3.0 mg, bei 20°C unter Umrühren entweder als Mikroemulsion (Suspension) oder zur wässrigen micellaren Lösung der quartären Ammoniumbase direkt zu. - Eine quantitative Dosierung dieser genannten Nukleosid-und Adeninnukleosid-Verbindungen kann auch durch Dialyse erreicht werden:

Die micellare Lösung der vorne genannten Konzentration (gepuffert, ungepuffert, pH ≅ 6,0, Ionenstärke variabel, T = 293°K) wird in ein Dialyseschlauch (Fa. Servant oder Pharmacia) gefüllt, verschlossen und unter stetem Rühren bei Raumtemperatur gegen eine eingestellte Lösung von pH ≦7,0 die das vorne genannte Pyridin- oder/und Adenin-Nukleosid bestimmter Konzentration enthält, für 2 Std.dialysiert. Die Abnahme der Extinktion bei 260 nm mit der Zeit der Dialyse erlaubt die Kontrolle des micellaren Einbaues der vorne genannten Wirkstoffe in den hydrophoben Kern des Hexadecylpyridinium-chlorides (Tab.1).

Tabelle 1

| (20°C, pH 5,5) | | | | | |
|---|---|---|---|---|---|
| Experiment | $R_H$ (Å) (±5,0Å) | Konzentration | | Ausbeute (%) | |
| | | Trifluoruridine mg/100 ml | Idoxuridin mg/100 ml | | |
| 1 | 45,0 | 5 | 7,5 | 95 | 95 |
| 2 | 45,0 | 7,5 | 10,5 | 95 | 98 |
| 3 | 50,5 | 10,0 | 12,5 | 94 | 98 |
| 4 | 60,0 | 12,0 | 15,0 | 96 | 98 |
| 5 | 60,0 | 15,0 | 17,0 | 96 | 97 |
| 6 | 65,0 | 17,0 | 20,0 | 96 | 96 |
| 7 | 71,5 | 20,0 | 21,5 | 100 | 98 |
| 8 | 75,0 | 25,0 | 23,0 | 100 | 100 |
| 9 | 75,0 | 30,0 | 24,0 | 100 | 100 |
| 10 | 78,0 | 50,0 | 30,0 | 100 | 100 |

Die erfolgte Bildung von kleinen micellaren Gebilden in der vorne genannten Lösung ist im NMR-Spektrum durch die Signale $\delta$ = 1,25 (Methylen), $\delta$ = 0,86 (Methyl) erkennbar. Durch Inkorporation der vorne genannten pharmazeutischen Wirkstoffe findet je nach Absättigung im hydrophoben Kern eine Verschiebung von $\delta$ = 1,25 (Methylen) statt, jedoch nicht von $\delta$ = 0,86 (Methyl).

Die Größe der Micellen kann durch inelastische Lichtstreuung nach Formel (1) leicht bestimmt werden (Tabelle 1). Die Größe, einschließlich der Form und zur Erhaltung einer homogenen und monodispersen Lösung kann auch durch HPLC-Chromatographie, Gelpermeation- und Agarose-Chromatographie erreicht werden.

(Abb. 7)

Eine Konzentration der so hergestellten Micellen kann durch Druckdialyse erreicht werden mittels Fiberglas-Patronen definierter Porengröße. So ist es möglich, nicht nur eine definierte Konzentration an pharmazeutischem Wirkstoff vorzunehmen, sonder auch die Micellengröße, Aggregationstrate, Hydratation (Solvatation) konstant zu halten, da keine Fusion der Micellen ("intermicellar growth") eintritt. D.h. die Zahl der Michellen pro Volumeneinheit mit ihrem eingeschlossenen pharmazeutischen Wirkstoff nimmt zu (Konzentration hydrodynamischer Partikeln mit gleichem Molekulargewicht), nicht die Aggregationsrate, noch die Zahl der eventuell vorliegenden Monomeren, die durch Ultrafiltration abgetrennt werden.

Beispiel 2:

Analog zu Beispiel 1 können die Gegenionen $Y^{\ominus}$ = $Cl^{\ominus}$, $Br^{\ominus}$ etc. nach verfahrensgemäßer Herstellung durch Ionenaustauscher Chromatographie an DEAE-Sephadex A 50 oder DEAE-Sepharose oder durch umdialysiefen gegen das betreffende bzw. gewünschte Gegenion $Y^{\ominus}$ ausgetauscht werden.

a) eine nach Beispiel 1 hergestellte wässrige micellare Lösung wird auf pH = 7,0 mit 0,01 N NaOH gebracht (20°C). Dies kann entweder durch Titration oder Dialyse gegen 0,01 N NaOH über 10 Std. geschehen. Anschließend erfolgt eine Dialyse gegen 1N Fumarat oder Maleat-Lösung, wobei hier die Na-Salze der Fumar- bzw. Maleinsaure eingesetzt werden können. Die Dialyse ist nach 12 Std. beendet. Ein Verlust an antiviralen Wirkstoffen, die vorne genannt sind, tritt nicht auf.

b) eine nach Beispiel 1 hergestellte wässrige micellare Lösung, pH 6,0, wird auf einer DEAE-Sephadex A 50 (1,0x100 cm)-Säule, die zuvor mit einer gepufferten (0,01 M $K_2HPO_4$-Puffer) 0,1 N Salizylatlösung beladen wurde, mit einer Fließgeschwindigkeit von 10 ml/30 min eluiert (20°C. Das überschüssige Salizylat wird durch Dialyse gegen einen großen Überschuß Wasser/Ethanol/Glycerol (90/5/5; $^{g}$/g) von dem Säuleneluat beseitigt. Die DEAE-Sephadex A 50 Chromatographie kann auch unter Druck im Gegenstromverfahren mit dem gleichen Lösungsmittelsystem durchgeführt werden. Es resultiert bei der Austauscher-Chromatographie (DEAE-Sephadex A 50, DEAE-Sepharose $^{2}$B, 5B, DEAE-Cellulose, hügelig) ein homogener Peak, der nach den Kriterien, die in Beispiel 1 aufgezeigt worden sind, analysiert werden können. DEAE-Sephadex und DEAE-Sepharose haben den Vorteil, daß erhebliche Mengen an micellaren quartären Ammoniumbasen sowohl gereinigt als auch auf mono-Dispersität geprüft werden können.

Beispiel 3:

Analog zu Beispiel 1 wird eine micellare Lösung von Hexadecylpyridinium-chlorid mit folgenden pharmazeutischen Wirkstoffen hergestellt:

| 100 g Lösung enthalten: | |
|---|---|
| Hexadecylpyridinium-chlorid | 0,10 g |
| Atropin-Hydrochlorid (±) | 0,002 g |
| Zink-II-chlorid | 0,004 g |
| Glycerol | 10,0 g |
| Ethanol | 4,894 g |
| Wasser | 85,0 g |
| pH | 6,2 |

Diese Präparation habt einen hydrodynamischen Radius von 35,0±5,0 Å und eine Aggregationsrate von N = 35, bei einem Molekulargewicht des Monomeren von Hexadecylpyridinium-chlorid von 393,0. Jede Micelle dieses Durchmessers enthält im Durchschnitt 100 µg Zink und/oder 50 µg Atropin (-).

Die Abbildung 9 zeigt die Varianz im hydrodynamischen Radius, $R_H$, dieser Präparation. Außerdem zeigt es die vorne beschriebene erfindungsgemäße Auftrennung des Racemats Atropin in die optische Antipoden z.B. Hyocyamin (-). Die micellare Größenverteilung wird durch ZnII-chlorid nicht verändert.

Die Abbildung 10 zeigt die Varianz im hydrodynamischen Radius, $R_H$, der N-Hexadecyl-4-methyl-pyridinium-chlorid

und N-Hexadecyl-4-methyl-pyridinium-chlorid + Atropin-HCL.

Beispiel 4:

Man löst 5 mg 4-(17-Tritriacontyl)-N-methyl-pyridinium-chlorid, 1-2,0 mg Amphotericin B in 10 ml einer Chloroform/Methanol-Mischung (2:1) unter Stickstoff bei 25°C auf und dampft diese Lösung im Rotationsverdampfer ein. Der filmartige Rückstand wird in 5 ml destilliertem Wasser fünf bis zehn Minuten geschüttelt. Diese Lösung wird anschließend für drei Minuten ultraschallbehandelt bis sie nicht mehr opaleszierend ist. Man kann diese, je nach Bedarf, anschließend durch Zugabe von 0,5 ml eines fünffachen Konzentrates von Phosphat-gepufferter, isotonische Kochsalzlösung auf den pH-Wert von 5,5-6,5, bringen.

Diese so hergestellte Lösung wird in eine gerührte Ultrafiltrationscelle (z.B. Amicon$^R$) eingefüllt, welche anstatt des Ultrafilters mit einem geradporigen Filter mit einem Porendurchmesser von 0,05 $\mu$m versehen ist, in Abwesenheit von $Me^{2+}$-Ionen ($Me^{2+}$=$Ca^{2+}$,$Mg^{2+}$,) so filtriert, daß das Volumen in der Zelle nicht unter 30 ml absinkt. Hierdurch werden Vesikel einheitlicher Größe von < 50.000 Å hergestellt.

Form, Größe und Molekulargewichtsverteilung können wie im Beispiel 1 und 2 ermittelt werden. Das Pyridinium-Amphiphile wird aus den entsprechenden Jodiden mit Silberchlorid in 10 % ($^v/v$) Ethanol-Wasser hergestellt. Die farblosen Kristalle haben einen $F_p$ = 64°C (aus Aceton umkristallisiert) und kristallisieren mit einem Molekül Wasser.

1 H-NMR ($CDCl_3/Me_4Si$): $\delta$ 0,93, (6H,t,J~4Hz), 1,28 (60 H,m), 2,8 (1H,q,J<2Hz, nicht aufgelöst), 4,75 (3H,s), 7,7-9,5 (4H,m). Charakteristisch ist ein $H_2O$-abhängiges Signal bei $\delta$ 4,4.

Anal: Calc. für $C_{39}H_{74}NCl \cdot H_2O$ (MW 610,50) C, 76,72; H, 12,55; Cl, 5,81; gefunden: C 76,53, H, 12, 42; Cl, 5,78.

Beispiel 5:

Analog zu Beispiel 5 werden 10 mg 3,5-bis [(n-hexadecyloxy) carbonyl] -N-methyl-pyridiniumchlorid ($F_p$=102-102,5°) mit 2,0 mg Amantidin oder Rimantadin in 10 ml einer Ethanol/Wasser-Mischung (1:2) unter Stickstoff bei 20°C gelöst. Nach Ultraschallbehandlung (5 min, 20°C, 10 Watt) können die gebildeten Vesikel mit ihren eingeschlossenen Wirkstoffen Amantidin oder Rimantadin auf einer Sepharose 2B nach Größe und Molekulargewicht getrennt werden, um eine homodisperse Lösung von Vesikeln mit geringer Molekular-Polydispersität zu erhalten. Im $^1$H-NMR-spektrum steht man die deutlichen Signale der Methylen ( $\delta$ =1,28) und Methyl-Protonen ( $\delta$ =0,86).

Diese in Beispiel 5 und 6 gebildeten unilamellaren Vesikeln können im Elektronenmikroskop sichtbar gemacht werden. Dazu wird die Vesikel-Dispersion zunächst der "freeze-fracture"-Methode unterzogen. Auch kann durch "negative staining" mittels der zwei Tropfen-Methode auf Formvar oder Kohlegrids durchgeführt werden. Durch diese beiden Techniken ist es zusätzlich möglich, eventuelle Populationen von Vesikeln sichtbar zu machen.

Auch die unter Beispiel 1 und 2 angewendete Methode der inelastischen Lichtstreuung gestattet es, Form und Größe dieser Vesikel und ihrer eingeschlossenen pharmazeutischen Wirkstoffe zu bestimmen (Abb. 11).

3,5-Bis [(n-hexadecyloxy)carbonyl] -N-methylpyridiniumchlorid, $F_p$=102,0-102,5° (Aceton). $^1$H-NMR ($CDCl_3/Me_4Si$): $\delta$ 0,85 (6H,t,J 5Hz), 1,30(56H,m), 4,40(4H,t,J<7Hz), 5,03(3H,s) 9,20(1H,t,J<2Hz), 10,00(2H,d,J<2Hz).

Analyt. Calc.: $C_{40}H_{72}NO_4Cl$(MW 666,47):C72,10, H10,88, C15,32; gef. C 71,44, H 10,84, Cl 5,23.

Beispiel 6:

Man löst 3 ml Gentamycin analog zu Beispiel 1 oder in eines in der Tabelle 1 genannten Tenside der quartären Ammoniumbasen in 1 ml einer Chloroform/Methanol-Mischung (3:1) auf und dampft diese Lösung bis zu einem dünnen Film ein. Dieser wird dann in 10 ml Wasser dispergiert. Anschließend kann man die Lösung auf den gewünschten pH >3 < 6,5 mit Puffer einstellen. Man erhält eine klare Lösung.

Diese klare Lösung enthält je nach Verwendung des Tensides gemäß Tabelle 1 eine monodisperse Verteilung von mit Gentamycin beladenen Micellen in der gewünschten Größenordnung und Ausbeute (Abb. 12).

Beispiel 7:

Eine micellare Lösung von Hexadecylpyridinium-chlorid (Cetylpyridinium) wird analog zu Beispiel 1 (20°C) bereitet und enthält die folgenden Wirkstoffe:

| 100 g Lösung enthalten: | |
|---|---|
| Cetylpyridiniumchlorid | 0,10 g |
| Atropin-Hydrochlorid (±) | 0,004 g |
| Quecksilber II-cyanid | 0,004 g |
| Glycerin | 10,892 g |
| Ethanol | 5,0 g |
| Wasser | 84,0 g |
| pH, T = 293°K | 5,7 |

Diese Präparation hat erfindungsgemäß einen hydrodynamischen Radius von 35,0±10,0 Å und eine Aggregationszahl, n, von 35 bei einem Molekulargewicht des Monomeren von Cetylpyridiniumchlorid von 393,0. Jede Micelle von diesem Durchmesser enthält im Durchschnitt 5 µg $Hg(CN)_2$ und/oder ~ 5,0 µg Atropin (-) (Abb. 14).

Diese Präparation ist eine homogene Lösung, die Micellen der Größenordnung von 30-50 Å ($R_H$) enthält. Sie inhibiert das Wachstum von Influenza A Virus wie die nachfolgende Tabelle 6 zeigt (Abb. 13).

Tabelle 6

| Inhibitor[a)] | Titration der Infektion[b)], Plaque forming units | Inhibition[c)] |
|---|---|---|
| 1-Adamantanamin-HCl | $2x10^6$ | -1,11 |
| wässrige $Hg(CN)_2$-Lösung | $1x10^6$ | -1,30 |
| Cetylpyridiniumchlorid | $1,5x10^8$ | -0,11 |
| Präparation nach Beispiel 7 | $2x10^5$ | -1,45 |
| Kontrolle | $2x10^8$ | - |

a) Inhibitor-Konzentrationen werden in den in vitro Zellkulturen von 100 µM zugegeben.
b) Plaque-Assay wurde nach K. Tobita, A. Suginire, C. Enamote und M. Fusiyama, Med. Microbiol. Immunol., 162, 9 (1975) an Nierenepithelien (Hund,MDCK) in Gegenwart von Trypsin durchgeführt.
c) Die Inhibition ist angegeben als der negative dekadische Logerithmus des Quotienten der "Plaque forming units" in Gegenwart des Inhibitors zu der ohne Inhibitor: $^{10}$log (pfu/ml des Inhibitors / (pfu/ml Kontrolle).

Die Abbildung 7 zeigt die Varianz im hydrodynamischen Radius, $R_H$, dieser Präpartion. Außerdem zeigt es die vorne erfindungsgemäß beschriebene Auftrennung des Atropins in seine optimale Antipoden in Gegenwart von $Hg(CN)_2$.

Beispiel 8:

Man löst 30 mg (0,020 mMol) 3,5-bis[(n-Hexadecyloxy)carbonyl]-N-methyl-pyridiniumchlorid und 1,0 mg (~0,005 mMol) Polyoxin A in 10 ml 0,01 M $KH_2PO_4$, pH 6,5 bei 20°C, das 1 ml einer Mischung von tert. Butanol/Methanol/Ethanol (2:1:1) enthält. Die Lösung wird ultraschallbehandelt (20 Watt, 5 min) im Eisbad bei 0°C und anschließend auf 20 ml mit Phosphatpuffer, pH 7,0, aufgefüllt. Die klare, nicht opaleszierende Lösung wird auf einer Sepharose 2B-Säule bei pH 7,0 in Gegenwart von Phosphat bei Raumtemperatur chromatographiert. Die mit dem pharmazeutischen Wirkstoff dotierten Vesikel werden in einer Ultrafiltrationszelle (Amicon$^R$) mit einem Porendurchmesser von 0,05 µm unten geringem Überdruck konzentriert. Nach Durchtritt von 0,3-0,5 ml Filtrat sind alle Vesikeln mit Durchmesser 350 Å abgetrennt und die überstehende Dispersion kann ampulliert und für Behandlungsversuche eingesetzt werden. Abb. 15 zeigt die Inhibierung der Chitinsynthetase bei Digitonin-behandelten Zeilen (Sacchamyces cerivisiae und Candida albicans) nach Zusatz dieser Präparation in Abhängigkeit von der Polyoxin A-Konzentration

Abb.16: Bild des "Freeze-Etch".

Tabelle 5

| Nr. | Tensid | $Hg(CN)_2$ $K_i$, µM | $Hg(CN)_2$ • Ascorbat $K_1$, µM | kompetitiv mit: |
|---|---|---|---|---|
| 1 | Hexadecyl-pyridinium-$Cl^{\ominus}$ | 7,9 | 15,3 | dGTP |
| 2 | Hexadecyl-pyridinium-$Cl^{\ominus}$ | | | dGTP |
| 5 | 8-Keto-hexadecyl-pyridinium-Cl | 0,4 | 0,05 | dGTP |
| 6 | 8-Keto-hexadecyl-pyridinium-Cl | 2,5 | 7,5 | dGTP |
| 7 | 3,5-bis (n-hexadecyloxycarbonyl-N-methyl-pyridinium-Cl | 2,0 | 9,2 | dGTP |
| 8 | 4-(17-tritriacontyl)-N-methyl-pyridinium-Cl | 4 | 10,1 | dGTP |
| 9 | nach Tabelle 3 Nr. 9 | 9 | 0,5 | dGTP |
| 10 | nach Tabelle 3 Nr. 10 | 0,1 | 7,9 | dATP |

Die Inhibitor-Konzentrationen sind in 50 % der vollständigen Inhibierung angegeben. Der Assay, der verwandt wurde ist der gemäß von Clements, J; D'Ambrosio,J; Brown, N.C.; J.Biol.Chem. (1975) 250, 522 und Wright, G.E.; Brown, N.C.; Biochem.Biophys. Acta (1976) 432, 37.

Pharmakodynamische Versuche :

Die Redeutung hochreaktiver Sauerstoffmoleküle (Superoxidradikale $O_2$, Peroxide $H_2O_2$, Hydroxilradikale $^{\bullet}OH$, Singulettsauerstoff $^1O_2$) im entzündlichen Prozess ist bekannt (s. z.B. McCord, J.M, K. Wong: Phagocytosis-produced free radicales: roles in cytotoxicity and inflammation. In: Oxygen Free Radicales and Tissue Damage, Excepter Medica, Amsterdam-Oxford-New York, 1979, 343-360; Allgemeine und spezielle Pharmakologie, Herg. W. Forth, D. Henschler, W. Rummel, Biowissenschaftlicher Verlag, 1983 ). Sie entstehen u.a. bei der Phagocytosen durch aktivierte Leukozyten (Monozyten, Makrophagen, polymorphkernige, neutrophile Granulozyten) und können zur Abtötung körperfremder Zellen, wie Bakterien, Bazillen u.a. auch bei bestimmten Viren, wenn das Immunsystem und die für IgG bzw. dem Komplementanteil $C_3$ spezifische Rezeptoren der Phagozyten funktionstüchtig sind, dienen. Die phagozytierenden Zellen selbst werden durch ein System, bestehend aus mehreren Enzymsystemen vor Schädigung durch diese besonders aktiven Formen des Sauerstoffs intrazellulär geschützt.

Es wurde nun gefunden, daß quartäre Ammoniumbasen der allgemeinen Formeln I und II, alle in der Lage sind, bei pH $\leqq$ 7,0 diese Sauerstoffradikale zu eliminieren:

In allen Reaktionen, die im entzündlichen Gebiet zwischen pH 5,0 und 6,0 ablaufen, verlangen, was durch die verfahrensmässige Herstellung dieser Erfindung gewährleistet ist,einen pH-Bereich $\leqq$ 7,0. Dadurch werden die gebildeten, agressiven Sauerstoffradikale gemäß der Reaktion 1-4 durch das N-Tensid,z.B. Cetylpyridiniumchlorid, als auch die entstehenden hydratisierten, kurzlebigen Elektronen, die durch Zusammenstoß $^{\bullet}O_2^-$ -Dadikale mit $H_2O$ entstehen können, abgefangen. Dadurch haben die N-Tenside in dem pH-Bereich $\leqq$ 7,0 erfindungsgemäß eine membranprotektive Wirkung, so daß es nicht zu Entzündungsreaktionen gemäß eines Prostaglandinmechanismus kommen kann. Die hohe Einfangrate $^{\bullet}O_2^-$ -Radikale bei diesen N-Tensiden von k $=5x10^{12}N^{-1}$ $sec^{-1}$ und ihrer Abhängigkeit von der Ionenstärke, die allerdings durch Zusatz von Ethanol/Glycerol konstant gehalten werden kann, wird durch die elektrostatische Doppelschichtstruktur der quartären Ammoniumbasen erklärbar.

So werden erfindungsgemäß fehlgeleitete lytische Reaktionen, an denen aggressive Sauerstoffradikale beteiligt sind, als pathogene Mechanisman der entzündlichen Erkrankungen, hervorgerufen durch Mikroorganismen und Viren, verhindert. So werden auch u.a. die cytotoxische Wirkung der Folgeprodukte dieser agressiven $.O_2^-$ -Radikale durch die erfindungsgemäßen N-Tenside, wie am Beispiel von Cetylpyridiniumhalogenid gezeigt, verhindert und u.a. Depolymerisationen von Hyaluronsäuren, Proteoglykanen, Collagenfibrillen, Cytoskeletons usw. und auch bei mukösen und membranösen Geweben (äussere Oberflächen) verhindert.

Weiterhin wurde gemäß der beschriebenen verfahrensmässigen Herstellung gefunden, daß Verbindungen der Struktur I und II die Infektion von menschlichen Zellen in vitro vermindert wird, so daß die erfindungsgemäß hergestellten micellaren Lösungen von I und II einen Schutz für die Zellen bzw. deren externer Oberfläche darstellen.

Es wurde weiter gefunden, daß dieser Schutz durch Inkorporation von $Hg(CN)_2$, ZnEDTA und/oder antiviralen, anti-fungalen und antibakteriellen Wirkstoffen verstärkt wird:

Es wurde gefunden, daß bei Inkubation von mit Influenzavirus, Untergruppe $A_2$, infizierte Monolayer-Zellkulturen von Vero-Zellen als auch bei Herpes simplex-Virus HSV I-III in vitro mehr als 60 % der Zellen vor der Infektion durch das betreffende Virus geschützt werden.

Es wurde weiter gefunden, daß der Effekt der Protektion durch die $N^+$-Tenside gemäß der allgemeinen Formel I und II für Mono-layer Zellfunktionen in vitro durch die antiviralen Wirkstoffe nicht verstärkt wird, wohl aber werden die Hemmkonzentrationen der antiviralen Wirkstoffe von Cytarabin, Idoxuridin, Trifluorthymidin als auch Herpes simplex Virus Typ 1 oder Influenza Virus Typus $A_2$ infizierte Monolayer-Zellen um 30 % gesenkt gegenüber Applikationen, die keine quartäre Ammmoniumbasen gemäß Formel I und II enthielten. Die Kombination von $N^+$-Tensid gemäß der allgemeinen Formel I und II erwies sich somit als das wirksame Virustatikum in Kombination mit micellar gebundenen antiviralen Wirkstoffen (Abb. 4).

Es wurde weiter gefunden, daß die $N^+$-Tenside gemäß der allgemeinen Formel I und II die antifungale Wirkung in Kombination mit antifungalen Wirkstoffen wie Econazol, Clotrimazol, Miconazol verstärkt (≈ 35 %), da die $N^+$-Base bei geeignetem Gegenion in der Lage ist, Cholesterol aus der externen Membran des Pilzes oder Hyphen unter Bildung von gemischten Micellen ("mixed micelles") zu extrahieren und dann die antifungalen Wirkstoffe, die wieder gebunden sind, in das Zellinnere des Fungus injizieren kann.

Es wurde weiter gefunden, daß die antifungale Wirkung durch Amphotericin B und eines N-Tensides der Formel II, vorzugsweise Hexadecylpyrianium-bromid, Decyl- und Hexadecyl-1-pyridinium-chlorid oder Hexadecyl-4-hydroxy-pyridinium-chlorid durch einen bislang nicht bekannten Mechanismus um das zehnfache verstärkt wird. Dies beinhaltet erfindungsgemäß, daß eine wesentlich geringere Wirkstoffkonzentration des pharmazeutischen Wirkstoffes ausreicht, um die gleichen therapeutischen Effekte zu erreichen.

Es wurde u.a. gefunden, daß die fungistatische Wirkung durch micellaren Einbau von $Z_nEDTA$ und ZnO, insbesondere auch durch $Hg(CN)_2$ in die N-Tenside der Formel I und II, insbesondere bei Hexadecyl-pyridiniumchlorid und Hexadecyl-4-hydroxy-pyridiniumbromid verstärkt wird, insbesondere bei Konzentrationen der anorganischen Wirkstoffe, wo sie selber noch nicht wirksam sind.

Es wurde gefunden, daß erfindungsgemäß die Micellen der N-Tenside in wässriger Phase bei pH $\leqq$ 7,0 therapeutische Mengen von Benzoylperoxid, des schlecht wasserlöslich und alkohollöslich ist, micellar binden können. So können z.B. 1 g Benzoylperoxid in 20 ml Bentethoniumchlorid oder in 25 ml Hexadecylpyridiniumchlorid, insbesondere aber in 3-Hexadecylbenzothiazonium- bromid gelöst werden. Bei örthlicher Anwendung löst die micellare Lösung ähnliche Schäleffekte an der Haut aus wie Tretinoin. Aufgrund seiner zusätzlichen sehr guten bakteriostatischen Eigenschaften, sowohl des Benzoylperoxides als auch des N-Tensides, ist erfindungsgemäß diese Kombination bei entzündlichen Akneformen besonders geeignet, z.B. bei Acne comedonica, Acne papulo-pustulosa und Acne conglobata.

Es wurde gefunden, daß die erfindungsgemäß hergestellten Micellen in wässriger Phase der N-Tenside, in denen $Hg(CN)_2$ oder ZnO, $ZnSO_4$, ZnEDTA micellar eingeschlossen ist, in der Zellkultur irreversibel und virusspezifisch die Vermehrung von Herpes simplex Viren infolge Hemmung der viralen DNS-Polymerase, hemmen. Die nichtinfizierten Zellen bleiben weitgehend unbeeinflußt, so daß die in dieser Erfindung beschriebenen Verfahren, z.B. für Hexadecylpyridiniumchloid, 3-Hexadecylbenzothiazolium-bromid (Sulfat) einschließlich der vorne genannten anorganischen Wirkstoffe, zu einem risikolosen Therapeutikum führt. Die adstringierenden Eigenschaften von $Hg(CN)_2$, ZnO, $ZnSO_4$, ZnEDTA, spielen keine Rolle, da im hydrophoben Core der Micellen keine freien Ionen vorliegen, a) da z.B. $Hg(CN)_2$ (richtiger $Hg_2(CN)_4$) undissoziiert ist, b) die anorganischen Wirksubstanzen durch ihre Lipophilie eingeschlossen sind und c) fast kein Wasser im hydrophoben Kern vorliegt.

Der kombinierte Effekt, die Bildung von gemischten Micellen ("mixed micelles") der N-Tenside gemäß der allgemeinen Formel I und II mit der durch den Virus befallenen Membran <u>und</u> der Phospholipid-Doppelmembran des Virus selber und die anschließende antivirale Wirkung auf die Virus DNS-Polymerase durch die vorne genannten anorganischen und organischen Wirkstoffe wie der Nukleosid analoge, 5-Ethyl-2'-desoxy-uridin und Viderabin, konnte gemäß Abb. 4a, b nachgewiesen werden.

Dieser Mechanismus konnte auch bei Rhino- und Influenza-Viren nachgewiesen werden. Ähnliche Wirkungen, jedoch bei geringeren Wirkstofftonzentrationen wurden für 1,1':3,3'-Biscyclobutan und für 1,1':3,3'Amin substituierte Kubane gefunden.

Es wurde gefunden, daß die verstärkte antivirale Wirkung bei Phospholipid-Viren, Adeno-Viren und Herpes simplex I durch das N-Tensid und die micollar eingeschlossenen Wirkstoffe gemäß folgenden biochemischen Mechanismen ihre Wirkung synergistisch entfalten:

a) Bindung an DNS, RNS-bildende Enzymsysteme, Entfaltung der Polypeptidkette durch das N-Tensid (Denaturierung) verstärkte.

b) "template"-Bindung, z.B. Daunomycin, Adriamycin

c) Bindung an Nukleosidanaloga, z.B. das vorne erwähnte ara-CTP-C5'-triphosphat des Cytosin-arabinosids, Azathioprin;

d) Bindung von anorganischen Wirkstoffen, z.B. $ZnSO_4$, ZnO, $Hg(CN)_2$, Wolframsäure-antimonate, z.B. $(NH_4)_{18}$ $(NaW_{21}Sb_9O_{86})_{17}$ und $K_{18}(KW_{21}Sb_9O_{86})_{17}$, sowie Hg-substituierte Kubane des vorne genannten Typus. Im Zusammenhang mit der antiviralen Wirkung der micellar eingeschlossenen antiviralen Wirkstoffe gemäß der verfahrensgemäßen Bearbeitung ist eine Reduktion der $ED_{50}$ um 20-25 % in vitro gegenüber dem reinen Wirkstorf zu verzeichnen, so daß die gleiche molekularbiologische Wirkung bei einer ca. 20 %igen Dosis durch den micellaren Effekt zu erzielen ist. Dies gilt insbesondere für micellar eingeschlossenes Rubaricin in Hexadecyl-pyridinium-Bromid, Hexadecyl-benzothiazolium-chlorid und Benzethonium-chlorid. DNA-Viren, Herpes-Viren sind bei diesen Beispielen am sensitivsten im Gegensatz zu Rimantadin + N-Tenside der Formel I und II, welche primär in vitro gegen RNA-Viren wirksam sind.

Es wurde weiter gefunden, daß die Antitumoraktivität von Adenosin-Desaminase Inhibitoren, die micellar gemäß Formel I und II verfahrensgemäß gelöst sind, z.B. Erythro-9-(2-hydroxy-3-nonyl)-Adenin, Deoxycoformycin, um das Zehnfache verstärkt wird. Das gleiche konnte auch für Aspartat-Transcarbamylase Inhibitoren festgestellt werden; so wurde durch micellar eingeschlossenes N-(phospono-acetyl)-aspartat die Biosynthese von Pyrimidin durch Blockierung der Karbamylierung von Aspartat um das 20-fache verstärkt.

Außerdem wurde gefunden, daß sowohl micellar eingeschlossenes $Hg(CN)_2$, $ZnSO_4$, ZnO oder ZnEDTA, wie auch 5-trifluor-methyl-2'-Desoxyuridin, welches aus Trifluor-methyl-Urazil in vitro entsteht, die Thymidin-Synthetase - ein Schlüsselenzym der DNS-Synthese - irreversibel hemmt.

Die Pyrimidin-Biosynthese wird durch Pyrazofurin, einem natürlich vorkommenden Antibiotikum, welches micellar eingeschlossen ist, um 20 % irreversibel gehemmt, bei gleichzeitiger Reduzierung der Zelltoxizität.

Es wurde weiterhin gefunden, daß die Diffusionsbarriere für Antibiotika, z.B. Tetrazyklinen, Aminoglykosiden auch für β-Lactamantibiotika (Penicillin) nach einer gewissen Zeit bei E. coli Bakterien für die micellar eingeschlossenen Wirkstoffe herabgesetzt werden. Diese Diffusionsbarriere ist konzentrationsabhängig für die vorne genannten Wirkstoffe, jedoch nicht für die verfahrensgemäß hergestellten N-Tenside. Hier handelt es sich um Faltungsprozesse an der äusseren Membran, primär um die Anderung der Struktur der Porine innerhalb der äusseren Membran von E. coli, so daß z.B. die anorganischen Wirkstoffe $Hg(CN)_2$, $ZnSO_4$, ZnEDTA in das Periplasma der äusseren Zellmembranen von gramnegativen Bakterien hinein diffundieren können.

Die Porine sind membranöse, wassergefüllte Poren, durch die hydrophile pharmazeutische Wirkstoffe in das Inrere der Zelle diffundieren können. Hydrophobe pharmazeutische Wirkstoffe können diese Porine nicht passieren. Die $N^+$-Tenside, der Erfindung können diese wassergefüllten Poren passieren. Somit können micellar eingeschlossene pharmazeutische, hydrophobe (lipophile) Wirkstoffe, insbesondere anorganischer Natur, durch den hydrophilen, äußere Form der $N^+$-Tenside durch passive Diffusion ins Zellinnere gelangen.

Hier reagieren sie dann außerdem mit den Zellwand-synthetisierenden Enzymen, insbesondere mit $Hg(CN)_2$ bei Konsentrationen von 10 μg/ml, ZnEDTA bei c= 5 μg/ml.

Die Geschwindigkeit der Diffusion von micellar eingeschlossenen Wirkstoffen steigt mit steigendem hydrophoben Charakter, normalerweise ist dies genau umgekehrt, z.B. sinkt die Diffusionsgeschwindigkeit bei gramnegativen Bakterien mit steigendem hydrophoben Charakter. Weiterhin begünstigt eine positive Ladung die Diffusion und die Ausbildung von "mixed micelles" von diesen erfindungsgemäß herzustellenden N-Tensiden.

Die Gültigkeit dieser Befunde konnte durch Untersuchungen der Diffusions- und Auflösungsgeschwindigkeiten verschiedener radioaktiv ($C^{14}$) markierter N-Tenside an der Membran (Periplasma) konzentrationsabhängig nachgewiesen werden.

Es wurde außerdem in vitro gefunden, daß die Thymidilat-Synthetase (TS) (EC2.I.I.45) sowohl durch $Hg(CN)_2$ in wässriger Lösung als auch in micellarer Lösung eines N-Tensides der Formel I und II, in dem $Hg(CN)_2$ hydrophob gelöst ist, gehemmt wird. TS katalysiert die Umwandlung von dUMP und $CH_2$-$H_4$-Folat zu dTMP und $H_2$-Folat. Da dieses Enzym für die Synthese von dTMP essentiell ist, also für die DNS-Synthese schlechthin, stellt es somit auch ein Target für pharmazeutische Wirkstoffe gegen neoplastische Zellen dar.

Es wurde nun gefunden, daß z.B. eine erfindungsgemäß hergestellte Lösung von Hexadecylpyridinium-chlorid, das $Hg(CN)_2$ hydropnob gebunden hält, gemäß der Tabelle 1 aufgeführten zytostatischen Aktivitäten gegenüber Leukämiezellen(L1210-Zellen) hat. So konnte u.a. gefunden werden, daß TS, dUMP und $Hg(CN)_2$ als anorganische pharmazeutischer Wirkstoff einen ternären Komplex gemäß (A,B)

A

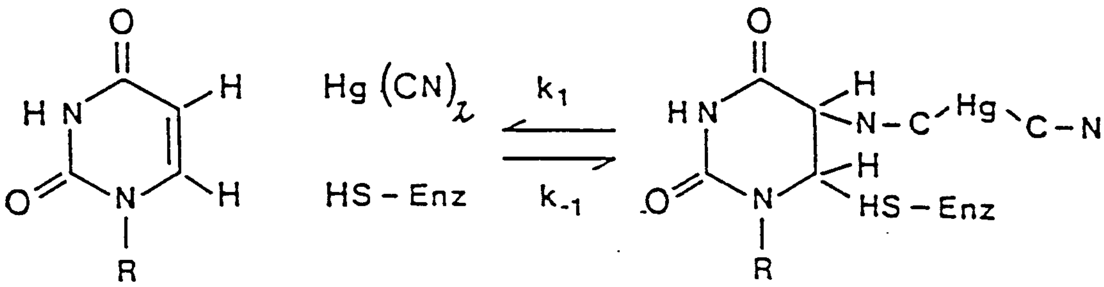

(cl UMP)

R = DESOXy RiBOSE

B

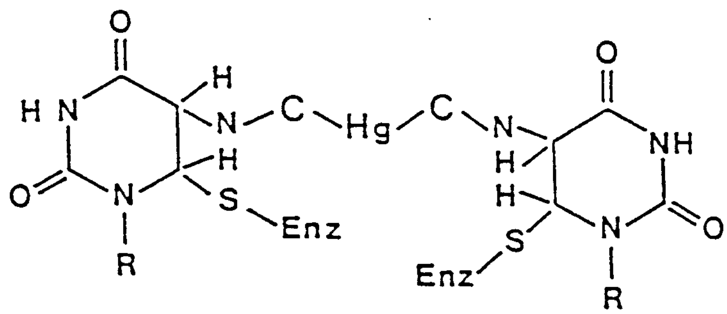

bilden, der durch Säulenchromatographie auf Sephadex. G-25 und BiO-Gel P10 isoliert werden kann. Die Bildung des Komplexes gemäß Gleichung A hat eine Bildungskonstante von $k_1$ = 0,51 $h^{-1}$, im Falle von Hexadecylpyridinium-chlorid, und $k_1$ = 0,79 $h^{-1}$ bei Benzethoniumchlord und micellar eingeschlossenem $Hg(CN)_2$. Die Dissoziationskonstanten belaufen sich auf $k_{-1}$ = 0,015 $h^{-1}$ (CPCl) und $k_{-1}$ = 0,02 $h^{-1}$, also beide sehr langsam, sowohl die Bildung als auch die Dissoziation des Komplexes. Dagegen ist die Bildung des Dimeren nach B wesentlich schneller: $k_1$ = 0,02 $h^{-1}$ und $k_{-1}$ = 0,015 $h^{-1}$ bei CPCl und $k_1$ = 0,01 $h^{-1}$, 0,03 $h^{-1}$ für Benzethoniumchlorid. D.h., daß micellare Lösungen von quartären Ammoniumbasen gemäß Formel I und II bei pH $\leqq$ 7,0, welche $Hg(CN)_2$ hydrophob gebunden halten, sind daher therapeutisch bei langsam wachsenden Tumoren, wo andere Inhibitoren bei TS unwirksam sind und die beobachtete Cytotoxizität gegenüber den normalen Zellen von anderen Antimetaboliten bei schnell wachsenden, proliferierenden Zellen kann daher verlangsamt werden.

Ribavirin, welches ein synthetisches 1,2,4-Triazolnukleosid ist, besitzt ein breites antivirales Spektrum für DNA- und RNA-Viren. Micellar eingeschlossenes Ribavirin durch kationische Tenside der Form ($Het{\equiv}N^+$-$(CH_2)_x$-$CH_3$)$Y^{\ominus}$ sehr schnell die Membranbarriere passiert, schneller als der pharmazeutische Wirkstoff selber. Auch die Umwandlung von Ribavirin zu Monophosphaten, Diphosphaten und Triphospaten durch die spezifischen zellulären Enzyme wird gesteigert, so daß die Hemmung der virusinduzierten Enzyme, welche notwiendig sind für die virale Nukleinsäurebiosynthese, beschleunigt wird. Während Ribavirin alleine nur einen moderaten Effekt auf die zelluläre DNA-Synthese hat und zytotoxisch im Bereiche von 200-1000 µg/ml bei normalen Zellinien ist, sinkt die Zytotoxizität in Gegenwart von kationischen Micellen, wenn micellar eingeschlossen, auf 50 µg/ml (in vitro-Teste), gemessen gegen Herpes simplex (DNA-Virus) infizierten Zellen.

Amantadin (1-Adamantanamin-HCl) besitzt besondere pharmakodynamische Wirkung gegen Influenzs-Viren (Klasse A). Die Replikation der meisten Influenza-A-Stämme werden in vitro zwischen 0,2 - 0,6 µg/ml gehemmt. Micellar eingeschlossenes Amantadin in kationische Micellen, insbesondere der Form ($Het{\equiv}N$-$(CH_2)_x$-$CH_3$)$Y^{\ominus}$ bewirken eine Reduzierung der Konzentration an pharmazeutischen Wirkstoff auf 0,05 µg/ml Amantadin gemessen nach Hayden et. al., (Plaque inhibition assay for drug susceptibility resting of influenca viruses. Antimicrob. Ag. Chermother. 1980, 17: 865-70; Grunert et al.; Sensitivity of influenza A/New Jersey 18/76/(Hswl-N1)-virus to amantadine HCl, J. Inf. Dis. 1977; 136, 297-300). Während Amantadin gegen Influenza-Virus Typ B fast keine Aktivität besitzt, besteht eine Hemmung von micellar eingeschlossenem Amantadin in den kationischen Tensiden der Formel

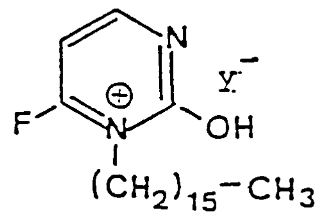

$Y^-$ = Fumarsäure-rest

(2-Hydroxy-5-Fluor-Hexadecylpyrimidinium-fumarat)

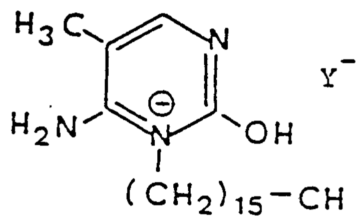

$Y^-$ = Fumarsäure-rest

(2-Hydroxy,5-methyl-6-amino-hexadecylpyrimidinium-fumarat)

Bei einer Konzentration von 0,01 Gew.% von Amantadin bei Influenza-Virus Typ B entsprechend einer Konzentration von 0,5 μg/ml pharmazeutischen Wirkstoff in vitro.

Ein überraschender Effekt von micellar eingeschlossenem Amantadin in den beiden kationischen Tensiden der obigen Formeln wurde gefunden, das Influenza-Virus Typ A gegen Amantadin in vitro nicht resistent wird, während es bei Amantadin allein der Fall ist.

Rimantadin-HCl (α-Methyl-1-adamantanmethylamin-hydrochlorid) hat die gleichen pharmakodynamischen Wirkungen in vitro wie Amantadin, jedoch iststärker wirksam bei gleicher Dosis. Auch hier wurde überraschenderweise gefunden, daß micellar eingeschlossenes Rimantadin in ein kationische Tensid, insbesondere der beiden obigen Formeln, der gleichein vitro-Effekt gefunden wurde, wie beim reinen pharmazeutischen Wirkstoff, allerdings bei einer wesentlich geringeren Dosis von 0,05 μg/ml.

Unter den anorganischen pharmazeutischen Wirkstoffen enfaltet $Hg_2(CN)_4$ und micellar gebundenes $Hg(CN)_2$ in kationischen Micellen ein überraschendes antivirales, interferoninduziertes Spektrum. In Zellkulturen von Lymphozyten und Fibroblasten konnte eine Induktion von Interferon $\alpha_1$ und Interferon β nach Inkubation mit micellar eingeschlossenen $Hg(CN)_2$ in einem kationischen Tensid der Formel

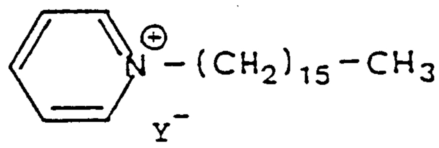

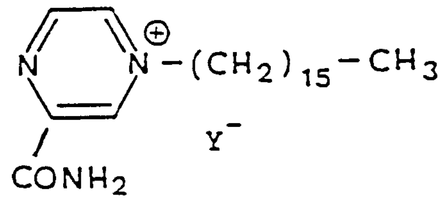

$Y^-$ = Chlorid

$Y^-$ = Salizylat

Hexadecylpyridiniumchlorid

2-Carboxamid-hexadecyl-pyrazinium-salizylat

bei einer Konzentration von $Hg(CN)_2$ von 5 µg/ml bis zu 15 µg/ml von einer 0,1 % ($^g/g$) kationischen Tensides fiestgestellt werden. Es wurde im Falle von Interferon $\alpha_1$ Konzentrationen von 20-50 Units/ml ermittelt, im Falle von Interferon β 10-20 Units/ml. Durch den micellaren Einbau des Quecksilbercyanids ist die Freisetzung des Interferon $\alpha_1$ insbesondere aber durch das Interferon β bei Fibroblastenkulturen um das 10 bis 100-fache gegenüber einfachen wässrigen, gepufferten Lösungen von Quecksilber II-canid gesteigert.

<u>Sekundäreffekte</u>

Die beobachteten Nebenwirkungen von Interferon $\alpha_1$, wie z.B. Kopfschmerzen, Lymphozytopenie, leichtes bis mittelschwerts Krankheitsbefinden liegen bei den hier beschriebenen pharmazeutischen Zubereitungen, insbesondere gegen Influenza- und Rhinoviren, nicht vor bzw. wurden nicht beobachtet. Dies liegt vornehmlich daran, daß wesentlich geringere Einheiten/ml des Interferon α, induziert durch den anorganischen pharmazeutischen Wirkstoff, therapeutisch zur Anwendung kommen als bei einer Therapie mit Interferon α alleine. So wurden auch keine toxischen Effekte, z.B. gastrointestinale Störungen, Gewichtsverlust, Alopezia, periphere Krämpfe, Paraesthesien und Knochenmarksdepressionen beobachtet, welche allerdings reversibel sind.

Die hämatologische Toxizität, welche im Falle von Interferon $\alpha_1$ dosisabhängig ist (Thershold-dosis $3x10^6$ Units/ml), liegt bei diesen pharmazeutischen Zubereitungen für Quecksilbercyanid, Rimantadin, Amantadin und Ribavirin nicht vor.

b) Die pharmazeutische Zubereitung, bestehend z.B. aus Hexadecylpyridiniumchlorid in Gegenwart von micellar eingeschlossenem Quecksilbercyanid bewirken in der Zellkultur eine Interferonproduktion. Es handelt sich hier um eine Interferoninduktion durch freigesetztes Quecksilbercyanid das örtlich in hohen Konzentration vorkommt und mit einem relative hohem Molekulargewicht von 4500 durch Ausbildung von polymeren Strukturen. (Paradies, oligomere Struktur von Quecksilbercyanid in Lösung, Vortrag Deutsch-Österreichische Chemische Gesellschaft, Innsbruck, Mai 1986.) Somit gehört diese pharmazeutische Zubereitung zu den Stoffen definiertor Interferoninduktoren von hohem Molekulargewicht wie z.B. synthetische doppelsträngige RNA:PolyI:PolyC als auch niedrigem Molekulargewicht wie z.B. 10-Carboxymethy-9-acridanon. Trotz dieser Heterogenität der Interferonwirkung ist es antivral wirksam. Diese Wirkung diente zum biologischen Nachweis dieser pharmazeutischen Zubereitung. Es kann gesagt werden, daß die Interferonbehandlung von Zellen in Zellkultur derartig verändert werden, daß eine nachfolgende Virusreplikation in diesen Zellen gehemmt wird. Dadurch unterscheiden sich Interferone in ihrem Wirkungsmechanismus grundsätzlich von Virustatika, die den Stoffwechsel der viren selbst hemmen. Da Interferone auf die Zellen wirken, ist es nicht verwunderlich, daß sie auch andere Wirkungen auf die Zellen ausüben können. Dies gilt nicht nur für Zellkulturen sondern hat auch Auswirkungen für den Gesamtorganismus. Es ist bekannt aus vielfältigen Untersuchungen, daß Interferon eine Vielzahl von Viren in ihrer Vermehrung hemmt. Das Ausmaß der Hemmung ist abhängig vom jeweiligen Virussystem. Somit scheint die Vermehrung fast aller Viren durch Interferon-Behandlung der Zelle hemmbar zu sein. Es gibt offensichtlich verschiedene Mechanismen, mittels deren Interferone wirksam sein können. So wird z.B. die Vermehrung von Retroviren auf die Bildung des "budding" d.h. des Ausschleusens neu gebildeter Virionen beeinflußt. Ein ähnlicher Mechanismus scheint auch für die pharmazeutische Zubereitung mit micellar eingeschlossenem $Hg(CN)_2$ zuzutreffen. So wurde im Rahmen der Erfindung beim Herpes simplex-Virus (HSV 1-3) durch die pharmazeutische Zubereitung bestehend aus Cetylpyridiniumchlorid und Quecksilbercyanid (s. Beispiel) induziertes Interferon auf die Synthese früh viral-kodierter Proteine des HSV die sogenannten β-Proteine nachgewiesen. Beim SV40-Virus scheint Interferon ebenfalls auf einen sehr frühen Schritt der Vermehrung zu wirken, der noch vor der primären Transkription liegt.

Die erfindungsgemäße pharmazeutische Zubereitung, speziell mit micellar eingeschlossenes Quecksilbercyanid, ließen die Interferon bedingte Hemmung bei verschiedenen lytischen RNA-Viren beobachten. Die Inhibition erfolgt auf der Ebene der Regulation der viralen Proteine. Sie wird hervorgerufen durch Induktion bestimmter zellulärer Enzyme. Bei näherer Untersuchung wurde gefunden, daß die Enzyme der 2',5'-Oligo-adenylat-Synthetase, der 2,5-A-abhängigen Endoribonuklease und die dsRNA-abhängige Proteinkinase hemmen. Für die beiden ersteren konnte durch Korrelationsstudien und durch Charakterisierung von Zellmutanten eine Rolle in der antiviralen Aktivität gegen lytische RNS-Viren durch micellar gebundenes $Hg(CN)_2$ nachgewiesen werden.

In diesen experimentell nachgewiesenen Effekten konnte auch ein antiproliferativer Effekt dieser pharmazeutischen Zubereitung auf die Interferone in vielfältiger Weise auf die Membranen und das Zytoskelett von Zellen nachgewiesen werden. So beeinflussen sie weiterhin die Expressionen einer Reihe wichtiger Gene, z.B. die der Haupthistokompatibilitätslokus, die sogenannten Transplantationsantigene. Somit zeichnen sich auch immunregulatorische Wirkungen ab (Interleukin-1-Synthese). Dadurch ergeben sich therapeutisch und pharmakodynamisch folgende Aspekte: Die Induzierung der Interferone durch diese pharmazeutische Zubereitung führt zu verstärkter Expression der Zelloberflächenproteine, die die wichtigste Rolle bei der Immunantwort spielen. Es sind dies die sogenannten Transplantationsantigene. Weiter ist anzuführen, daß mindestens zwei wichtige zelluläre Komponenten der körpereigenen Abwehr aktiviert werden, nämlich die Makrophagen und die natürlichen Killerzellen. Außerdem, was vor allem das Interferon $\gamma$ betrifft, scheinen auch die Funktionen vom B-Lymphozyten maßgeblich durch diese pharmazeutische Zubereitung beeinflußt zu werden.

Somit ergibt sich für die erfindungsgemäße pharmazeutische Zubereitung, insbesondere bestehend bei Hexadecylpyridiniumchlorid und micellar eingeschlossenem Quecksilbercyanid, eine Induktion, wenn auch keine spezifische, von Interferon. Es kann kein Zweifel daran bestehen, daß Interferone nicht nur in vitro sondern auch in vivo nicht nur antiviral sondern auch immunregulatorisch wirksam sind. Obwohl der direkte antivirale Effekt auch in vivo bedeutsam sein kann, spielen im Organismus bei der Interferon-Wirkung, wie sie oben aufgezeichnet worden sind, weitere indirekte Mechanismen eine Rolle, z.B. die Aktivierung von Makrophagen speziell bei Influenza-Virus A und B. Die Tatsache, daß Interferon $\gamma$ Makrophagen aktivieren kann, scheint auch im Hinblick auf bakterielle und parasitäre Infektionen wichtig zu sein, da bei deren Abwehr Makrophagen eine entscheidende Rolle spielen.

Posologie und therapeutische Indikationen:

Die therapeutischen Indikationen sowie die Dosis ergeben sich durch die micellar eingeschlossenen Konzentrationen der pharmazeutischen Wirkstoffe:

- So bestehen Indikationen für aufkommende und bestehende Erkältungskrankheiten, die vornehmlich durch Influenza- und Rhinoviren verursacht werden;
- katarrhalische Entzündungen viruider Genese;
- Hautinfaktionen und infektiöse Dermatosen;
- hartnäckige, antiseptische Wundbehandlung;
- Dermatomykosen;
- Mykosen;
- Prophylaxe und Therapie baktariell bedingtar Hautläsionen wie z.B. Pyodermie, Otitis media;
- mikrobielle und sekundär infizierte Ekzeme;
- Überempfindlichkeit gegen Makrolid-Antibiotika;
- Akne, insbesondere entzündliche Formen mit Papeln und Pusteln;
- baktarielle Infektionen und Sekundärinfektionen der Haut, sofern sie durch grampositive und/oder gramnegative meklocyclinsensible Keime hervorgerufen werden;
- Akne vulgaris;
- Verhütung von Nabelinfektionen;
- chirurgische und traumatische Wunden;
- lokaler Schutz vor Infektionen und mit Antibiotika empfindlichen Keimen infizierte Wunden;
- Furunkel, Karbunkel, Abszess;
- Dermatomykosen, verursacht durch Dermatophyten, Hefen, Schimmelpilze und anderen Pilzen, Pityriasis versicolor,
- Erythrasma durch Cornebakterien;
- Candidosen der Haut und Schleimhäute
- Herpes simples I-III, Herpes-Keratitis
- solare und senile Keratosen, prämaligne Veränderungen und oberflächliche Basaliome, auch in strahlengeschädigter Haut;
- Plattenepitelkarzinome der Haut und Mukosa im Kopf- und Halsbereich; dermatologische Malignome;

Je nach Indikationsstellung und pharmazeutischem Wirkstoff richtet sich die spezielle Dosis. Da die Erhaltungs- und Anfangsdosis der hier beschriebenen pharmazeutischen Wirkstoffe bekannt sind, wird je nach Applikationsart und galenischer Zubereitung z. B. Cremen, Zäpfchen, Tropfen, Tabletten, Kapseln und liposomartige Verkapselungen nur 50 % der normalen therapeutischen Gesamtdosis benötigt, je nach Konzentration des micellar eingeschlossenen pharmazeutischen Wirkstoffes.

Schilderung der Dosisreduzierung aufgrund des potenzierenden (synergistischen) Effektes:

Micellen in wässriger Lösung, auch mit angeschlossenen lipophilen Wirkstoffen, stehen im dynamischen Gleichgewicht mit ihren monomeren Tensiden, d.h. die Micellen ändern Form, Größe und Hydratation. U.a. verlassen monomere kationische Tenside eine einzelne Michelle, um sich an einer anderen Micelle in wässriger Lösung wieder einzugliedern, so daß - auch wenn die Konzentration des Tensides weit über der KMK liegt - immer eine gewisse Konzentration von fluktuierenden Monomeren besteht. Durch Zugabe des Potenzierungsgemisches wird diese Dynamik dahingehend gestört, daß

1.) bei konstanter Temperatur und chemischen Potentials die Form, Größe und monodisperse Homogenität der isotropen Lösung erhalten bleibt, somit tritt auch kein Verlust an micellar eingeschlossenem lipophilen (hydrophoben) pharmazeutischen Wirkstoff ein.
2.) Die Konzentration an Monomeren, welche destabilisierend auf die geometrische Form der Micelle wirkt, wird zugunsten eines Einbaues in die"vollständigan" Micelle in isotroper Lösung eingeschränkt. Dies bewirkt, daß das System einschließlich der micellar eingaschlossenen hydrophoben pharmazeutischen Wirkstoffen "leckt". Dies wird vornehmlich dadurch verhindert, daß das Potenzierungsgemisch, insbesondere Glycerin und Dimethylsulfoxid, die Wassarstruktur an der externen Oberfläche der Micelle so einfrieren ("Tridymit-Struktur"), daß sie eisartige Strukturen annehmen und die Wassermoleküle sehr unbeweglich werden.
3.) Die pharmazeutische Zubereitung wirkt aufgrund das potenzierenden Effektes durch Glycerin, wie z.B. in vitro gezeigt werden konnte, weniger zytotoxisch, d.h. es schädigt vornehmlich die beschädigte (infizierte) Zelle, weniger die gesunde Zelle im Zellverband.

Die Erfindung weist insbesondere folgende Vorteile auf:
Die in dieser Erfindung hergestellten N-Tenside, einschließlich ihrer verfahrensgemäßen Einschließung der Wirkstoffe, bedingt eine erhebliche, z.T. bis zu 80%ige Reduzierung der Toxität der anorganischen Wirkstoffe, z.B. bei $Hg(CN)_2$, (auch $HgCl_2$, $Hg_2Cl_2$, $HG(NH_2)Cl$ [Präzipitat], ZnEDTA) und Zn Salzen im allgemeinen, als auch bei nephrotoxischen, ototoxischen Antibiotika, insbesondere bei Polymixinen, Erythromycin, Gentamycin, Tetrazyclin, von ca. 30 % da

1.) die Micellen, als auch ihre eingeschlossenen Wirkstoffe, nicht - wegen ihrer Größe - resorbiert werden,

2.) die micellar eingeschlossenen Wirkstoffe sich nur am Ort - meistens topisch - entfalten, so daß geringe Konzentrationen an Wirkstoff ausreichen, da zusätzlich noch der synergistische Effekt des N-Tensides besteht.

So wurde u.a. gefunden, daß der hemmende Effekt der Speichelsekretion von Atropin durch Hexadecylpyridiniumchlorid durch micellare Katalyse um das Zehnfache verstärkt wird, bei verfahrensgemäßer Herstellung der N-Tenside, pH $\leqq$ 7,0. Die verstärkte Wirkung auf die Peripherie ist u.a. durch die micellare Auftrennung des Racemates in L(-) Hyocyamin verantwortlich (siehe Abbildung 1).

Die Erfindung erstreckt sich auch auf die antiphlogistischen Eigenschaften der hier dere lipophile (hydrophobe) pharmazeutische Wirkstoffe einzukapseln.

Die meisten bisher bekannten Verfahren leiden entweder an einer zu geringen Einkapselwirkung oder an einer Begrenzung der Materialtypen, welche eingeschlossen werden sollen, oder auch an beiden. So sind bekanntermaßen die meisten dieser Prozesse auf den Einschluß hydrophiler Materialien und pharmazeutischer Wirkstoffe beschränkt und können nicht wirkungsvoll den Einschluß von lipophilen pharmazeutischen Wirkstoffen vornehmen. Im Gegensatz dazu sind alle derzeit verfügbaren Verfahren mit Ausnahme das von Banghan et al. (Biochim.Biophys.Acta 443:629-634, 1976) nur für die Einkapselung biologisch aktiver Substanzen in oligo-multilamellaren oder unilamellaren Liposomen geeignet.

Ein besonderer Vorteil dieser pharmazeutischen Zubereitung auf der Basis vesikulärer Strukturen von $N^+$-Tensiden ist die hydrophobe Einkapselung von pharmazeutischen Wirkstoffen. Eine besonders vorteilhafte Folge der durch Ultraschallbehandlung und Gegenionen hergestellten großkalibrigen Vesikel ist darin zu sehen, daß die Gefahr des Austritts des pharmazeutischen Wirkstoffes aus der Bläschenhaut des Ansatzes reduziert oder eliminiert wird. Deshalb kann diese Form des quartären $N^+$-Tenside auf der Basis von sechsgliedrigen Heterozyklen, insbesondere für das Einkapseln hydrophober pharmazeutischer Wirkstoffe herangezogen werden, die dazu verwendet werden können, um lokale

z.B. örtlich begrenzte statt systemische Wirkungen zu erzielen.

Während die meisten der bekannten Verfahren auf die Einkapselung hydrophiler Wirkstoffe beschränkt sind, kann mit dieser Erfindung die Einkapselung von hydrophoben pharmazeutischen Wirkstoffen vorgenommen werden. Versuche haben gezeigt, daß sogar anorganische lipophile pharmabeschriebenen quartären organischen Ammoniumbasen. Das Gegenion $Z^-$ nimmt verfahrensbedingt Einfluß auf die Größe, Form und micellare Stabilität, kann aber auch selber ein pharmazeutischer Wirkstoff sein, so daß eine Arzneimittelwirkung pharmakodynamisch verstärkt werden kann. Die hier beschriebenen Tenside haben den großen Vorteil, daß sie neben intrinsischen Pharmakodynamischen Eigenschaften milieuabhängig und darüber hinaus im sauren pH-Bereich stabil sind. Die verfahrensmäßigen erhältlichen Micellen als pharmazeutische Zubereitung mit eingeschlossenen lipophilen (hydrophoben) pharmazeutischen Wirkstoffen wirken als Träger für antimikrobielle, antivirale, keratolytische, antifungale und antineoplastische Wirkstoffe, können aber u.a. selber antimikrobielle, antifungal und antiviral und antiphlogistisch (topisch) wirksam sein.

Insbesondere beschreibt die vorliegende Erfindung eine pharmazeutische Zubereitung zur Herstellung von micellaren gelösten hydrophoben anorganischen Wirkstoffen wie Quecksilber-II-Cyanid, Zink, Wolfram und Antimon-Verbindungen sowie Salze der Phosphonsäure. Es wurde gefunden, daß diese anorganischen Verbindungen sowohl antivirale als auch antineoplastische Wirkungen haben.

Auch die Bildung von vesikulären Strukturen der quartären Ammoniumbasen von 4- und 3,5-substituierten Hexadecylpyridinium-$Z^-$ erfolgt spontan bei konstanter Temperatur, Druck, Ionenstärke einschließlich in Gegenwart von eingesetzten stöchiometrisch pharmazeutischen Wirkstoffen, welche sowohl vesikulär (Hohlraum) als auch micellar (doppelmembranartig) gebunden werden können.

Insbesondere bezieht sich die Erfindung auf ein verbessertes Herstellungsverfahren zur Erzeugung von multilamellaren Lipidbläschen auf der Basis von kationischen Tensiden, die dazu benutzt werden können, insbesonzeutische Wirkstoffe wie z.B. Quecksilber-II-Cyanid mit hoher Wirksamkeit eingeschlossen werden können und ihre pharmakodynamische Wirkung durch das Potenzierungsgemisch noch verstärkt werden kann.

Dieser Nachteil wird durch die neuen N-Tenside der allgemeinen Formel I wie auch die Vesikel auf der Basis von $N^+$-Tensiden entweder durch micellaren Einschluß der pharmazeutischen Wirkstoffe oder durch kovalente Verknüpfung der Wirkstoffe mit dem $N^+$-Tensid unter Beibehaltung der äußeren morphologischen Form der gesamten Micelle aufgehoben.

Bekannt ist die bakterizide Wirkung von Chlorhexidin bei grampositiven und gramnegativen Bakterien, jedoch resistent gegen gramnegative Bazillen. Gefunden wurde, daß micellare Lösungen von quartären Ammoniumbasen gemäß der allgemeinen Formel I und insbesondere II die 2-4 Gew.% Chlorhexidin hydrophob im micellaren Kern gebunden halten, die Resistenz gegen gramnegative Bazillen aufgehoben und ihre therapeutische Wirksamkeit im Verhältnis zum Chlorhexidin alleine verstärken. Die beobachteten Nebenwirkungen von Chlorhexidin wie Kontaktdermatiden, Hauteffekte topischer Art, Photosensibilität der Haut, finden bei verfahrensgemäßer Herstellung der micellaren Lösungen der N-Tenside der Formel I und II nicht statt.

Es ist Gegenstand der vorliegenden Erfindung, die eingangs erwähnte Heterogenität von Form und Größe der Micellen auch in Gegenwart von Potenzierungsgemischen abzustellen. Es wird somit gewährleistet, daß eine monodisperse Form von kationischen organischen Ammoniumbasen auch in Gegenwart von pharmazeutischen Wirkstoffen und Potenzierungsgemischen bei der Herstellung erreicht wird.

Diese erfindungsgemäßen quartären organischen Ammoniumbasen beseitigen die oben geschilderten Nachteile der bislang herkömmlichen Invertseifen. So besteht außerdem großes Interesse sowohl an der therapeutischen Verwendung von quartären Ammoniumbasen, die sowohl als pharmazeutischer Wirkstoff fungieren <u>und</u> als Träger von Wirkstoffen unterschiedlichster Art, z.B. antimikrobieller, antiviraler, antifungaler oder antineoplastischer Natur, micellar aufnehmen können. Sie sollen daher die eingangs genannten, vor allem milieu-bedingten Nachteile nicht besitzen.

Die erfindungsgemäßen, mit pharmazeutisch kovalent verbundenen Wirkstoffe, auf der Basis von quartären Ammoniumbasen, haben den Vorteil

1. daß diese maskierten Antimetabolite aus der Pyrimidin- bzw. Purin-Reihe, keine intramoleculare Wechselwirkungen anionischer bzw. kationischer Natur eingehen. Sie sind neutral geladen (z.B.kein Nukleotid-dianion durch das Phosphat) und können daher ungehindert in die pro- bzw. eukarvontische Zelle diffundieren, so daß hohe intrazelluläre Antimetabolit- (z.B. 5'-Nukleotid) Konzentrationen erreicht werden;

2. daß die pharmazeutischen Wirkstoffe durch N-C-Hydrolyse mittels der vorhandenen Enzymsysteme der germinalen bzw. eukarvontischen Zellen, erst am Target oder auch topisch freigesetzt werden;

3. durch die Erhöhung der Hydrophobizität der Alkyl- bzw. Aryl-Kette bzw. Restes am $N^+$-Tensid wird die Membran-Permeabilität erhöht, so daß die pharmazeutischen Wirkstoffe quantitativ passiv in das Zytosol übertreten können. Im Gegensatz zu Di-Anionen oder Kationen, welche die Membran unter physiologischen pH-Bedingungen und Ionenstärken schwer passieren können, ist dies bei den verfahrensgemäßen $N^+$-Tensiden ungehindert;

4. die hohe Hydrophobizität bedingt auch einen hohen Verteilungskoeffizient im System $CHCl_3$-$H_2O$ bei pH 8,0;

5. durch die konzentrierte Aufnahme von hydrophoben bzw. hydrophilen pharmazeutischen Wirkstoffen wird zusätzlich zu den kovalent verankerten und die Wirkstoffkonzentration nach Penetration durch die germinale Membran, fungale Zellwand (Hemmung der Chitinsynthetase) oder virale Phospholipid-Doppelmembran durch einen Konzentrationsgradienten (extrazellulär - intrazellulär) erhöht. Dadurch resultiert eine geringe Anflutungszeit.

Im Gegensatz zu Liposomen als Träger von pharmazeutischen Wirkstoffen, wie sie z.B. in der US-Patentschrift 3,993,754 oder in der europäischen Patentschrift 0,102,324 genannt sind, haben diese hier erfindungsgemäß beschriebenen Micellen von quartären Ammoniumbasen die Vorteile,

1. daß sie wasserunlösliche Wirkstoffe micellar im sogenannten "liquid core" aufnehmen können, und dadurch sowohl topisch als auch enteral durch Öffnen der Micelle diese wasserunlöslichen Wirkstoffe kontrolliert freisetzen können, z.B. Rimantadin, Amantadin, Tromantadin, die bei Influenza-Viren bzw. Herpes Simplex-Viren sowohl der Haut als auch des Auges wirksam sind.

2. wasserlösliche Wirkstoffe können sowohl im Sternlayer als auch micellar gelöst werden, wenn sie selber hydrophobe Bereiche haben, wie z.B. Polyen-Verbindungen, Tetrazykline, Aminoglykoside und aromatische Antimetabolite, Z.B. Trifluorthymidin, Viderabin, Cytarabin, 5-Jod und 5-Fluor-desoxyuridin, 5-Ethyl-2'-desoxyuridin, Erythromycin und Nalidixinsäure.

3. Die hier erfindungsgemäß beschriebenen kationischen N-Tenside haben zwei spezifische Bindungsstellen mit hohen Bindungskonstanten ($K_B$ = 10-15μM) und großer Bindungskapazität (Kapazität 100 μg/Micelle): die eine ist bedingt durch die hydrophoben Wechselwirkungen zwischen dem "liquid core" der Micelle und dem hydrophoben Bereich des Wirkstoffs ( G=15-20kcal/Mol) als auch die π-π-Wechselwirkungen der hier beschriebenen Wirkstoffe zwischen den N-Heterozyklen der N-Tenside und den pharmazeutischen Wirkstoffen, die zweite Bindungsstelle ist unspezifischer Art und ist an der Grenzfläche zwischen Stern-layer und hydrophobem Kern lokalisiert. Die Bindungskonstante liegt im Bereich von $K_B$ = 20 mM, die Bindungskapazität 100-200 μg/Micelle. Die unspezifische Bindungsstellen liegen fast ausschließlich im Guy-Chapman-Layer. Im Gegensatz zu Liposomen, wo die Zahl der unspezifischen Bindungsstellen wesentlich höher ist, kann die Zahl der unspezifischen Bindungsstellen durch Zugabe von Ethanol/Glycerol ausgeschaltet werden, da die Kräfte, welche in dem Guy-Chapman-Layer wirken, durch Konzentrationen von Ethanol, Glycerin bis zu 30-35 Gew.% ausgeschaltet werden, ohne die Bindungskapazität, -stärke der hydrophoben Kräfte bzw. im Stern-layer zu beeinflussen (nur Polarität und Konfiguration).

4. Die hier beschriebene Erfindung hat den Vorteil, daß die micellar eingeschlossenen pharmazeutischen Wirkstoffe nicht wieder den micellaren Verband verlassen, wie z.B. bei Liposomen, die nach den bisher bekannten Verfahren "lecken". Die "Dichtigkeit" ("sealing") der vorliegenden Erfindung von micellar eingeschlossenen Wirkstoffen ist z.B. am Beispiel von micellar gebundenen radioaktiv markiertem Trifluorthymidin, Cytarabin und Idoxuridin nachzuweisen. So wurde u.a. gefunden, daß Idoxuridin erst nach 200 Tagen 5 Gew.% seiner ursprünglichen micellar eingeschlossenen Konzentration (2000μg) im Falle von Hexadecyl-pyridinium- oder Benzethonium-chlorid Micellen verliert. Die entsprechenden Werte für radioaktiv markiertes Trifluorthymidin und Cytarabin liegen bei 210 und 300 Tagen (20°).

5. Nach dem Verfahren der vorliegenden Erfindung lassen sich diese Micellen mit den eingeschlossenen anorganischen und organischen Wirkstoffen bei pH = 7,0 auf einfache Weise ohne großen apparativen Aufwand in wässriger Phase herstellen, welche kleine, einfache Micellen mit einem Durchmesser von ca. 50-100 Å und große Micellen mit einem Durchmesser von 600-10.000 Å - je nach Gegenion - enthalten. Außerdem werden durch ein Gemisch von Glycerol/Ethanol im Verhältnis von 2 Gew.% : 15 Gew.% gegenüber Wasser beide Micellen verschiedener Größenordnung sowohl in ihrer Form (Kugel, Hemizylinder, Stab, Diskus) als auch in ihrer Kompaktheit durch Senkung der KMK, wie auch durch Reduktion der Freien Energie der gesamten Micelle in der wäßrigen Phase infolge Verdünnung der Elektronendichte an der externen Oberfläche, stabilisiert. Mittels geeigneter Trennmethoden, z.B. HPLC, Ultrafiltration, Gelfiltration und/oder präparativer Zentrifugation kann man kleine von großen Micellen präparativ trennen.

6. Die Stabilität, Haltbarkeit und Lagerfähigkeit dieser so hergestellten Micellen aus quartären organischen Ammoniumbasen, pH = 7,0, gegenüber Temperatur, Dichtigkeit ("sealing and leaking") und Lagerfähigkeit ist durch die Einlagerung der pharmazeutischen Wirkstoffe in hydrophoben Kern der Micellen im Verhältnis zu den Micellen ohne Wirkstoffe bei gleichen Y erhöht. Im Gegensatz zu den Liposomen tritt hier kein Schmelzen bei höherer Temperatur (>40°C) ein, sondern bei verfahrensgemäßer Herstellung ändern sich die hydrodynamischen Verhältnisse

erst ab >60°C. Da bei zunehmender Temperatur diese so hergestellten Micellen von quartären Ammoniumbasen eher eine Reduzierung im hydrodynamischen Radius eingehen, daher kompakter werden, sind diese Art Micellen thermodynamisch stabiler als künstliche Liposomen oder Liposomen + quartäre Ammoniumbasen. Diese Vorgänge sind leicht im Routine-Verfahren durch inelastische Lichtstreuung bei der Herstellung zu prüfen.

7. Die Hydrophobizität bzw. die Penetration der $H_2O$-Moleküle in diese so hergestellten Micellen und deren Beeinflussung durch anorganische pharmazeutische Wirkstoffe, z.B. $Hg(CN)_2$, ZnEDTA, $ZnSO_4$, ZnO, Wolframsäure-antimonate, $K_{18}(KW_{21}Sb_9O_{86})_{17}$, als auch der organischen Substanzen ließ sich durch NMR-Spektroskopie nachweisen:

Am Beispiel des 8-Ketohexadecylpyridinium-chlorid (8-KHPCl) kann man die erfindungsgemäße Anwendung der Aufnahme von pharmazeutischen Wirkstoffen demonstrieren. Folgerichtig wurde nunmehr gefunden, daß eine chemische Verschiebung von 146,6 ppm für eine 0,1 molare micellare Lösung in Wasser auftritt, die jedoch durch z.B. $Hg(CN)_2$ nach 147,2 ppm verschoben wird. Micellen in wäßriger Lösung, die 0,05 molar an 8-KHPCl und 0,2 molar an CPCl (Cetylpyridiniumchlorid) sind, ergaben allerdings eine chemische Verschiebung von 147,2 ppm für das $^{13}C$-Carbonyl-Signal von 8-KHPCl. Vergleicht man diese beiden Zahlen mit den Verschiebungen von 8-KHPCl in Methanol (145,7 ppm) und Acetonitril (144,0 ppm) wird deutlich, daß die CO-Gruppe in dieser Micelle eine weitgehend wäßrige Umgebung einnimmt. $Hg(CN)_2$ spielt hierbei eine doppelte Rolle, die damit auch die therapeutische Breite in vitro bestimmt: der hydrophobe Charakter des $Hg(CN)_2$ bewirkt eine hohe Löslichkeit im hydrophoben Kern von z.B. Hexadecylpyridinium-chlorid als Monmer und bedingt eine chemische Verschiebung von $\delta_{c8}$ 27,5 ppm $^{13}C$ der $CH_2$-Kette auf 32,5 ppm, während in 8-KHPCl-Micellen Quecksilber II-cyanid in der Nähe der Ketogruppe ($C_9$) als $Hg_2(CN)_4$ in $H_2O$ (siehe oben) gelöst ist und durch diese $H_2O$-Löslichkeit ist die Konzentration von $H_2(CN)_4$ limitiert.

Abbildung 5 zeigt die Abhängigkeit der Extinktion der micellar eingeschlossenen anorganischen Wirkstoffe und des N-Phosphono-acetyl-L-aspartatsin Hexadecylpyridiniumchlorid.

<u>Legenden zu den Abbildungen, soweit sie noch nicht in der Beschreibung enthalten sind.</u>

2) Abhängigkeit des Stokes' Radius von N-Hexadecyl-5-carboxamid-chlorid mit mizellar eingeschlossenen 5-Fluorcytosin von der Temperatur
3) Abhängigkeit des Stokes' Radius von 2,-4-Dihydroxy-5-methyl-hexadecyl-pyridinium-chlorid mit mizellar eingeschlossenem Gentamycin von der Temperatur

Abb. 7:

1) Abhängigkeit des Stokes's Radius von 8-Ketohexadecyl-pyridinium-chlorid mit mizellar eingeschlossenem Z-Miconazol von der Temperatur
2) Abhängigkeit des Stokes' Radius von 8-Ketohexadecyl-pyridinium-chlorid mit mizellar eingeschlossenem Z-Miconazol + $Hg(CN)_2$ von der Temperatur

Abb. 11:

1) Abhängigkeit des Stokes' Radius von 3,5-bis [(n-hexadecyloxy) carbonyl] -N-methyl-pyridiniumchlorid mit lamellar eingeschlossenem Amantidin von der Temperatur; nicht ultraschallbekandelt
2) Abhängigkeit des Stokes' Radius von 3,5-bis [(n-hexadecyloxy) carbonyl]-N-methyl-pyridiniumchlorid mit lamellar eingeschlossenem Amantidin von der Temperatur; ultraschallbehandelt
3) Abhängigkeit des Stokes' Radius von 3,5-bis (n-hexadecyloxy) carbonyl -N-methyl-pyridiniumchlorid mit lamellar eingeschlossenem Rimantadin von der Temperatur; ultraschallbehandelt

Abb. 13:

1) Abhängigkeit des Stokes' Radius von N-Hexadecyl-pyridiniumchlorid und mizellar eingeschlossenem $Hg(CN)_2$ von der Temperatur; nicht ultraschallbehahdelt
2) Abhängigkeit des Stokes' Radius von N-Hexadecyl-pyridiniumchlorid und mizellar eingeschlossenem $Hg(CN)_2$ von der Temperatur; ultraschallbehandelt

**Patentansprüche**

**1.** N-alkylierte quaternäre Pyridiniumverbindungen der allgemeinen Formel (IA)

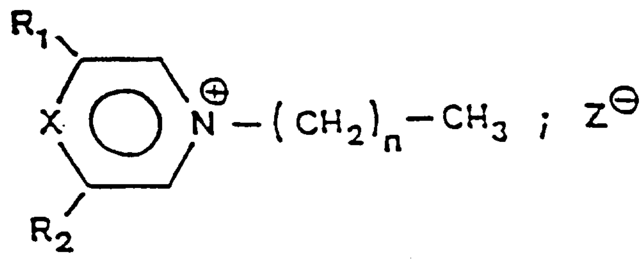

X = C-OH

X = C - $CH[(CH_2)_n\text{-}CH_3]_2$

$R_1$ =

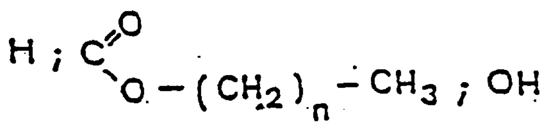

$R_2$ =

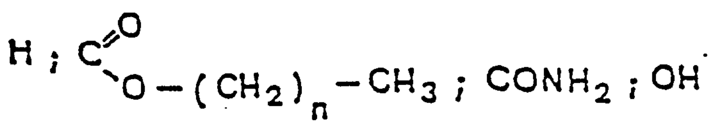

oder Formel (IB)

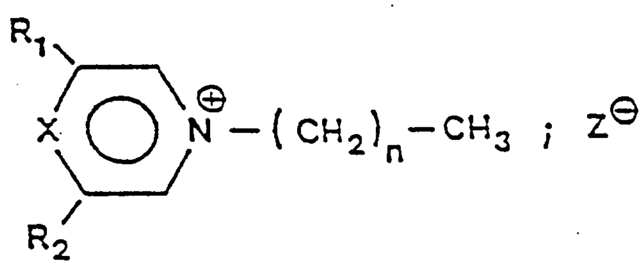

X = CH; C-OH

X = C - $CH[(CH_2)_n\text{-}CH_3]_2$

$R_1$ =

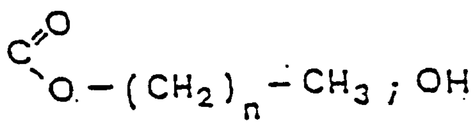

$R_2$ =

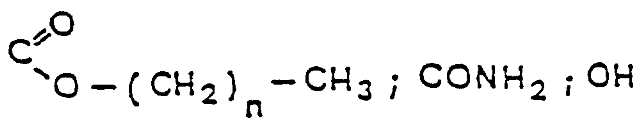

wobei

n = 8 bis 20, insbesondere 15 und
Z = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

bedeuten.

**2.** N-Alkyl-4-hydroxypyridinium nach Anspruch 1 der Formel

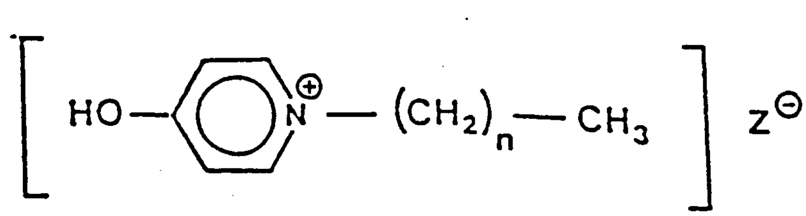

wobei $Z^-$ die Anionen gemäß dem Patentanspruch 1 und n = 8 - 20 bedeuten.

**3.** Hexadecyl-4-hydroxypyridinium nach Anspruch 1 oder 2 der Formel

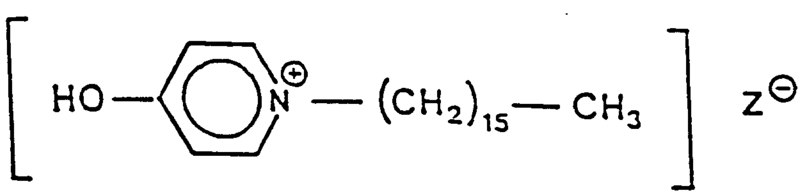

wobei $Z^-$ die Anionen gemäß dem Patentanspruch 1 bedeuten.

**4.** 4-[1,1bis n-Alkyl (Niederalkyl)] N-Hexadecyl-pyridinium-Verbindungen nach Anspruch 1 der Formel

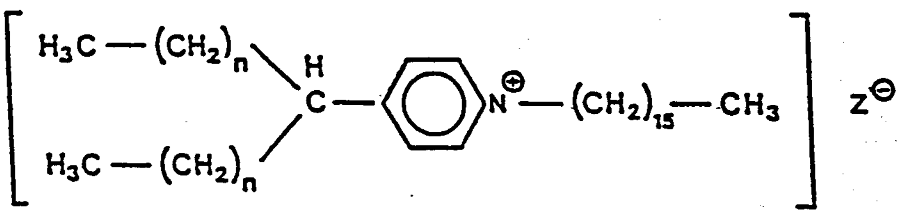

wobei Z⁻ die Anionen gemäß dem Patentanspruch 1 bedeuten.

5. 3,5-bis [(n-Alkyloxy)carbonyl] N-Hexadecyl-pyridinium-Verbindungen nach Anspruch 1 der Formel

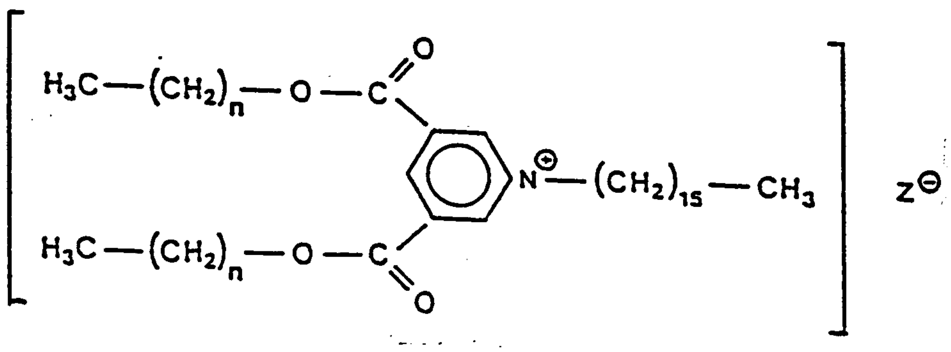

wobei Z⁻ die Anionen gemaß dem Patentanspruch 1 bedeuten.

6. Verfahren zur Herstellung der N-alkylierten quartären stickstoffhaltigen Heterozyklen nach einem oder mehreren der Ansprüche 1 - 5,
**dadurch gekennzeichnet,**
daß der zu alkylierende Heterozyklus in einem geeigneten Lösungsmittel gelöst wird, anschließend unter stetem Rohren die stöchiometrische Menge n-Alkyl-halogenid zugegeben wird, anschließend unter stetem Rühren am Rückfluß über eine geraume Zeit erhitzt wird, wonach das Endprodukt nach dem Abkühlen ausfällt.

7. Verfahren nach Anspruch 6,
dadurch gekennzeichnet,
daß der zu alkylierende, unsubstituierte Heterozyklus in einem geeigneten Lösungsmittel gelöst wird, die stöchiometrische Menge n-Alkyl-halogenid zugegeben wird, anschließend unter Druck bei 100°C für eine Reaktionsdauer von 8 Stunden die Reaktion vollendet wird, wonach das Endprodukt nach dem Abkühlen ausfällt.

8. Verwendung der Pyridiniumverbindungen nach einem oder mehreren der Ansprüche 1 - 5 zur Herstellung von micellaren oder vesikulären Strukturen in polaren oder unpolaren Lösungsmitteln, insbesondere mit extrem niedriger KMK.

9. Verwendung der Pyridiniumverbindungen nach einem oder mehreren der Ansprüche 1 - 5 zur Aufnahme von hydrophoben pharmazeutischen Wirkstoffen bei pH 6 - 8 in polaren Lösungsmitteln oder unpolaren Lösungsmitteln.

10. Verwendung der Pyridiniumverbindungen nach einem oder mehreren der Ansprüche 1 - 5 als eigenständige pharmazeutische Wirkstoffe bei pH 6 - 8 in polaren Lösungsmitteln.

11. Verwendung der Pyridiniumverbindungen nach einem oder mehreren der Ansprüche 1 - 5 als spektroskopische Marker (Reporter-Gruppen) in immunologischen und klinisch-biochemischen Analyseverfahren sowie zu kolloidchemischen Meßverfahren zur Bestimmung der KMK.

12. Pharmazeutische Zubereitung,
**dadurch gekennzeichnet,**

daß sie N-alkylierte quaternäre Pyridiniumverbindungen der allgemeinen Formel (II) als micellare oder vesikuläre Strukturen enthält, in die pharmazeutische Wirkstoffe eingeschlossen sind, dispergiert in einem Lösungsmittel, wobei der pH-Wert der Zubereitung ≦ 7 ist, und die kritische Mizellbildungskonzentration (KMK) im Bereich von 1,0 x $10^{-7}$ bis 1,5 x $10^{-4}$ Mol/l liegt und die Formel (II) ist:

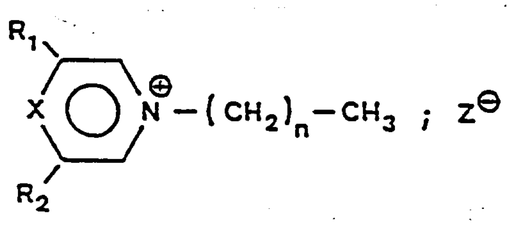

X = CH; C-ON

X = C - $CH[(CH_2)_n-CH_3]_2$

$R_1$ =

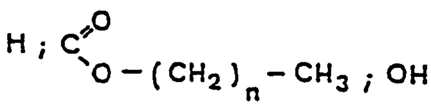

$R_2$ =

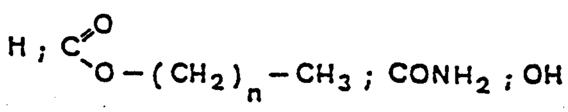

wobei

n = 8 bis 20, insbesondere 15 und

$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

bedeuten.

**13.** Pharmazeutische Zubereitung nach Anspruch 12,
dadurch gekennzeichnet,
daß die Wirkstoffe Antibiotika, Fungizide, antiviral wirkende Verbindungen, antineoplastisch wirkende Verbindungen und/oder antiphlogistisch wirkende Verbindungen sind.

**14.** Pharmazeutische Zubereitung nach Anspruch 12 oder 13,
dadurch gekennzeichnet,
daß die Wirkstoffe eine oder mehrere der Verbindungen der Gruppe von $ZnS0_4$, ZnO, ZnEDTA, $Hg(CN)_2$ und Wolframsäureantimonaten sind.

**15.** Pharmazeutische Zubereitung nach Anspruch 12 oder 13,
dadurch gekennzeichnet,
daß die Wirkstoffe eine oder mehrere der Verbindungen der Gruppe von Amphotericin B, Econazol, Clotrimazol,

Miconazol, Cytarabin, Idoxuridin und Trifluorthymidin sind.

16. Pharmazeutische Zubereitung nach Anspruch 12 oder 13,
dadurch gekennzeichnet,
daß die Wirkstoffe eine oder mehrere Verbindungen der Gruppe von Ribavirin, Amantadin, 1,1' : 3,3'-Biscyclobutan und
1,1' : 3,3'-Amino- oder Hg-substituierte Kubane sind.

17. Verfahren zur Herstellung einer pharmazeutischen Zubereitung nach Anspruch 12,
**dadurch gekennzeichnet**,
daß eine Verbindung der allgemeinen Formel (II) in einem geeigneten Lösungsmittel gelöst und diese Lösung in an sich bekannter Weise weiterbehandelt wird, um eine micellare oder vesikuläre Strukturen enthaltende Lösung zu erhalten, in die die pharmazeutischen Wirkstoffe eingeschlossen werden.

18. Verfahren nach Anspruch 17,
dadurch gekennzeichnet,
daß zum Erreichen micellarer oder vesikulärer Strukturen eine Ultraschallbehandlung durchgeführt wird.

19. Pharmazeutische Zubereitung mit antiphlogistischer Wirkung,
**dadurch gekennzeichnet**,
daß sie als Wirkstoff eine Verbindung der allgemeinen Formel (II) des Anspruchs 12 enthält.

**Claims**

1. N-alkylated quaternary pyridinium compounds of general formula (IA)

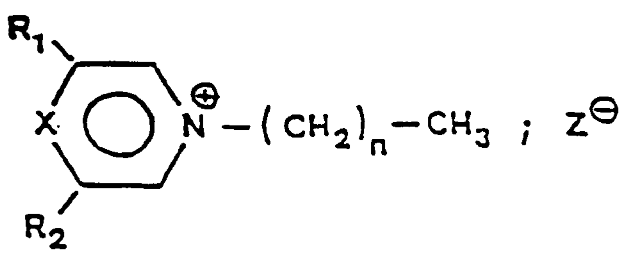

X = C-OH

X = C - $CH[(CH_2)_n-CH_3]_2$

$R_1$ =

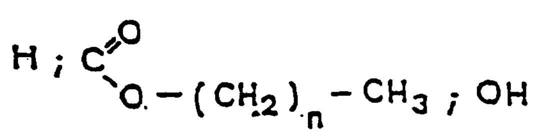

$R_2$ =

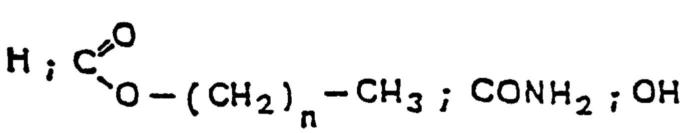

or formula (IB)

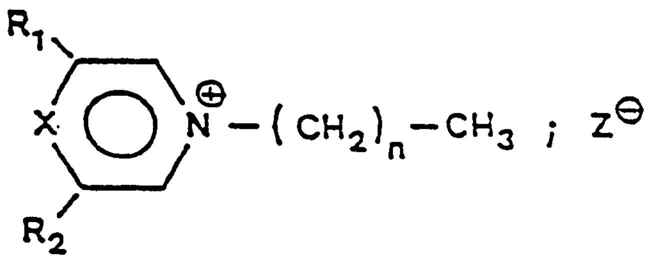

X = CH; C-OH

X = C - $CH[(CH_2)_n-CH_3]_2$

$R_1$ =

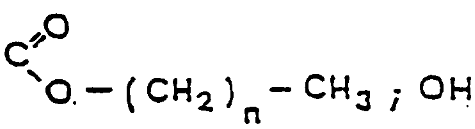

$R_2$ =

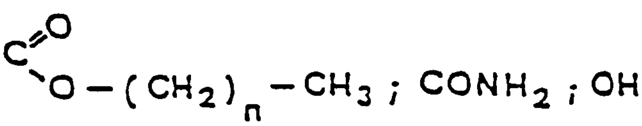

where

n = 8 to 20, particularly 15, and

Z represents chloride, bromide, iodide, maleate, formate, acetate, propionate, hydrogen sulphate, malate, fumarate, salicylate, alginate, gluconate, glucoronate, galactoronate, ethyl sulphate or hydrogen phosphate $H_2PO_4^-$.

**2.** An N-alkyl-4-hydroxypyridinium according to claim 1, of formula

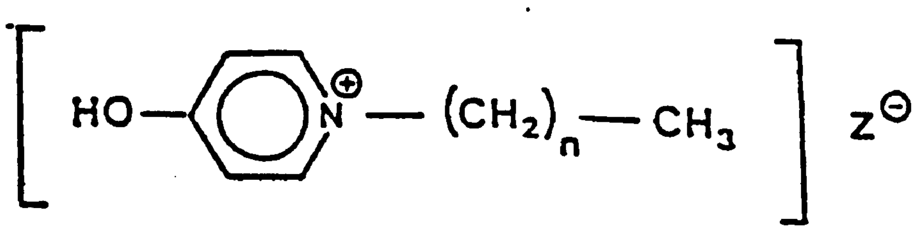

where $Z^-$ represents the anions according to claim 1 and n = 8 - 20.

3. A hexadecyl-4-hydroxypyridinium according to claim 1 or 2, of formula

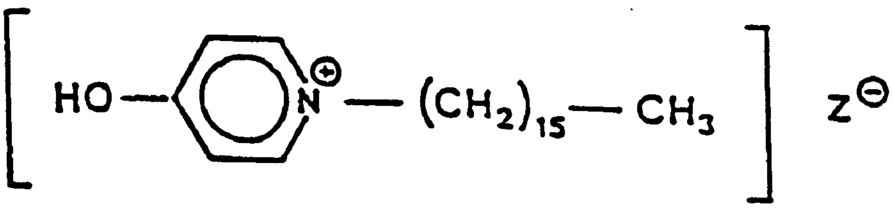

where $Z^-$ represents the anions according to claim 1.

4. 4-[1,1-bis-n-alkyl (lower alkyl)] N-hexadecyl-pyridinium compounds according to claim 1, of formula

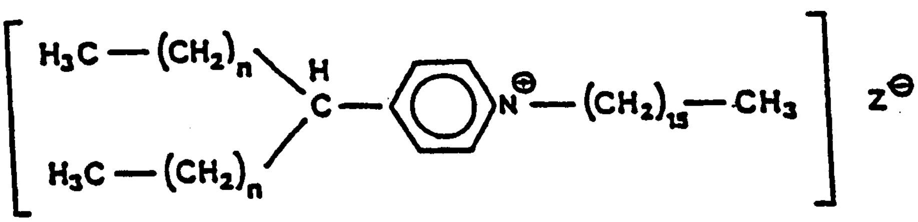

where $Z^-$ represents the anions according to claim 1.

5. 3,5-bis-[(n-alkoxy)carbonyl] N-hexadecyl-pyridinium compounds according to claim 1, of formula

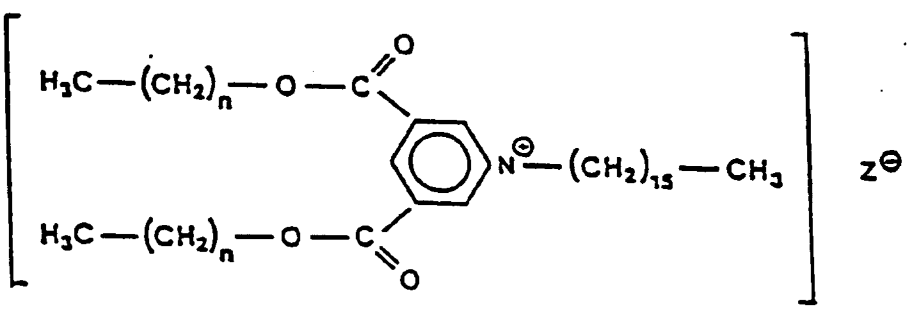

where $Z^-$ represents the anions according to claim 1.

6. A method of preparing the N-alkylated quaternary nitrogen-containing heterocycles according to one or more of claims 1 - 5,
**characterised in that**
the heterocycle to be alkylated is dissolved in a suitable solvent, the stoichiometric amount of n-alkyl halide is subsequently added with constant stirring, heating is subsequently effected with continuous stirring under reflux for some time, after which the final product precipitates after cooling.

7. A method according to claim 6,
characterised in that
the unsubstituted heterocycle to be alkylated is dissolved in a suitable solvent, the stoichiometric amount of n-alkyl halide is added, the reaction is subsequently completed under pressure at 100°C for a reaction time of 8 hours, after which the final product precipitates after cooling.

8. The use of the pyridinium compounds according to one or more of claims 1 - 5 for the production of micellar or vesicular structures in polar or nonpolar solvents, particularly with an extremely low CMC.

9. The use of the pyridinium compounds according to one or more of claims 1 - 5 for the absorption of hydrophobic pharmaceutical active ingredients at pH 6 - 8 in polar solvents or nonpolar solvents.

10. The use of the pyridinium compounds according to one or more of claims 1 - 5 as independent pharmaceutical active ingredients at pH 6 - 8 in polar solvents.

11. The use of the pyridinium compounds according to one or more of claims 1 - 5 as spectroscopic markers (reporter groups) in immunological and clinico-chemical methods of analysis and in colloid chemical methods of measurement for determining the CMC.

12. A pharmaceutical preparation,
**characterised in that**
it contains N-alkylated quaternary pyridinium compounds of general formula (II) as micellar or vesicular structures, in which pharmaceutical active ingredients are included, dispersed in a solvent, wherein the pH of the preparation is 7 and the critical micelle concentration (CMC) is within the range from 1.0 x $10^{-7}$ to 1.5 x $10^{-4}$ mole/l, and formula (II) is:

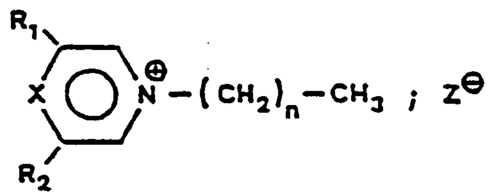

X = CH; C-OH

X = C - $CH[(CH_2)_n-CH_3]_2$

$R_1$ =

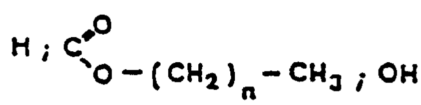

$R_2$ =

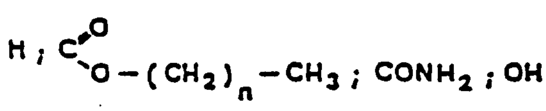

where

n = 8 to 20, particularly 15, and
$Z^-$ represents chloride, bromide, iodide, maleate, formate, acetate, propionate, hydrogen sulphate, malate, fumarate, salicylate, alginate, gluconate, glucoronate, galactoronate, ethyl sulphate or hydrogen phosphate $H_2PO_4^-$.

13. A pharmaceutical preparation according to claim 12,
characterised in that
the active ingredients are compounds with an antibiotic, fungicidal or antiviral action, compounds with an anti-neoplastic action, and/or compounds with an anti-phlogistic action.

**14.** A pharmaceutical preparation according to claim 12 or 13,
characterised in that
the active ingredients are one or more compounds of the group comprising $ZnSO_4$, ZnO, ZnEDTA, $Hg(CN)_2$ and tungstic acid antimonates.

**15.** A pharmaceutical preparation according to claim 12 or 13,
characterised in that
the active ingredients are one or more compounds from the group comprising amphotericin B, econazole, clotrimazole, miconazole, cytarabine, idoxuridine and trifluorothymidine.

**16.** A pharmaceutical preparation according to claim 12 or 13,
characterised in that
the active ingredients are one or more compounds from the group comprising ribavirin, amantadine, 1,1': 3,3'-bicyclobutane and 1,1': 3,3'-amino- or Hg-substituted cubanes.

**17.** A method of producing a pharmaceutical preparation according to claim 12,
characterised in that
a compound of general formula (II) is dissolved in a suitable solvent and this solution is further treated in a manner known in the art in order to obtain a solution containing micellar or vesicular structures in which the pharmaceutical active ingredients are included.

**18.** A method according to claim 17,
characterised in that
an ultrasonic treatment is effected in order to obtain micellar or vesicular structures.

**19.** A pharmaceutical preparation having an anti-phlogistic action,
characterised in that
it contains a compound of general formula (II) of claim 12 as an active ingredient.

**Revendications**

**1.** Composés pyrimidium N-alkylés quaternaires, de formule générale (IA)

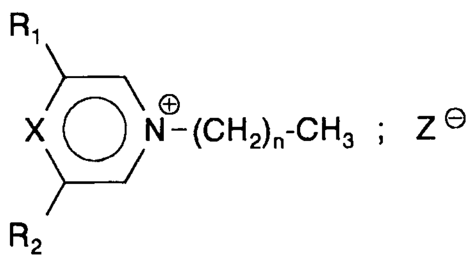

X = C - OH

X = C - $CH[(CH_2)_n - CH_3]_2$

$R_1$ =

H ; 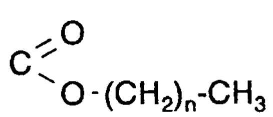 ; OH

$R_2$ =

H ; 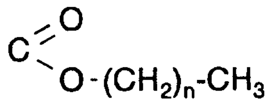 ; $CONH_2$ ; OH

ou de formule générale (IB)

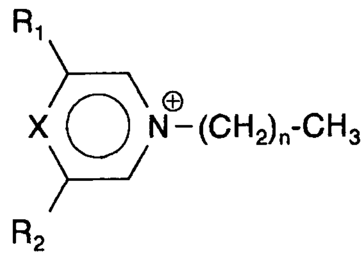 ; $Z^{\ominus}$

X = CH ; C - OH

X = C - $CH[(CH_2)_n - CH_3]_2$

$R_1$ =

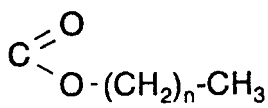 ; OH

$R_2$ =

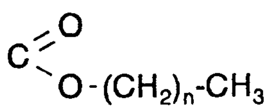 ; $CONH_2$ ; OH

dans lesquelles

n = 8 à 20, en particulier 15 et

Z = chlorure, bromure, iodure, maléate, formiate, acétate, propionate, hydrogénosulfate, malate, fumarate, salicylate, alginate, gluconate, glucuronate, galcturonate, éthylsulfte ou hydrogénophosphate $H_2PO_4^-$.

**2.** N-alkyl-4-hydroxypyridinium selon la revendication 1, de formule

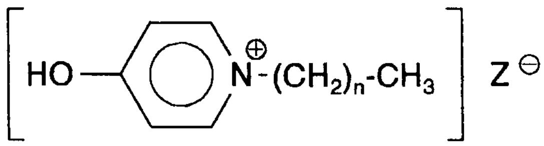

dans laquelle $Z^-$ représente les anions selon la revendication 1 et n = 8 - 20.

3. Hexadécyl-4-hydroxypyridinium selon la revendication 1 ou 2, de formule

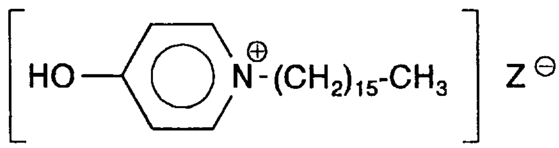

dans laquelle $Z^-$ représente les anions selon la revendication 1.

4. Composés 4-[1,1 -bis-*n*-alkyl (alkyle inférieur)] N-hexadécyl-pyridinium selon la revendication 1, de formule

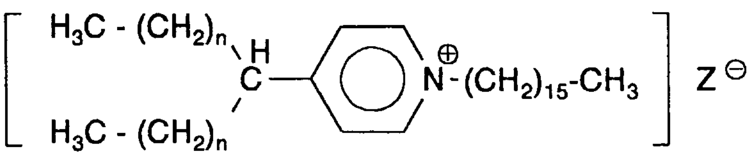

dans laquelle $Z^-$ représente les anions selon la revendication 1.

5. Composés 3,5-bis[(*n*-alkyloxy)carbonyl] N-hexadécyl-pyridinium selon la revendication 1, de formule

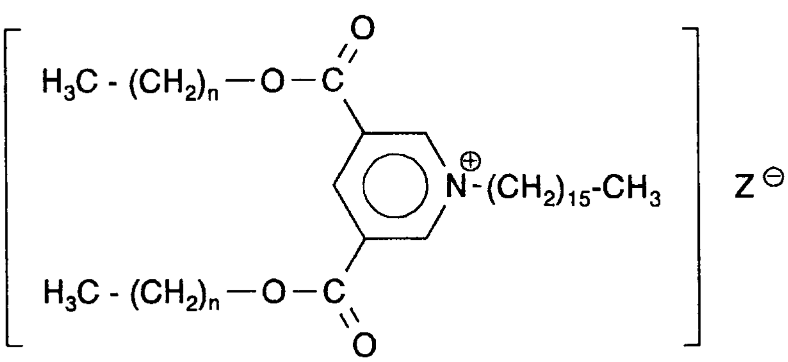

dans laquelle $Z^-$ représente les anions selon la revendication 1.

6. Procédé pour la préparation des hétérocycles azotés N-alkylés quaternaires selon une ou plusieurs des revendications 1 à 5, caractérisé en ce que l'hétérocycle à alkyler est dissous dans un solvant approprié, puis, sous agitation constante, on lui ajoute une quantité stoechiométrique d'halogénure de *n*-alkyle, puis il est chauffé pendant longtemps, sous reflux, sous agitation constante, le produit final précipitant après refroidissement.

7. Procédé selon la revendication 6, caractérisé en ce que l'hétérocycle non substitué à alkyler est dissous dans un solvant approprié, on lui ajoute la quantité stoechiométrique d'halogénure de *n*-alkyle, puis la réaction est achevée sous pression, à 100 °C, pendant une durée de 8 heures, le produit final précipitant après refroidissement.

**8.** Utilisation des composés pyridinium selon une ou plusieurs des revendications 1 à 5 pour la préparation de structures micellaires ou vésiculaires dans des solvants polaires ou apolaires, en particulier de CMC extrêmement faible.

**9.** Utilisation des composés pyridinium selon une ou plusieurs des revendications 1 à 5 pour le captage de principes actifs pharmaceutiques hydrophobes dans des solvants polaires de pH 6 - 8 ou dans des solvants apolaires.

**10.** Utilisation des composés pyridinium selon une ou plusieurs des revendications 1 à 5 comme principe actif original dans des solvants polaires à pH 6 - 8.

**11.** Utilisation des composés pyridinium selon une ou plusieurs des revendications 1 à 5 comme marqueur spectroscopique (groupes *reporter*) dans des procédés analytiques d'immunologie et de biochimie clinique, ainsi que pour des procédés de mesures en chimie des colloïdes pour la détermination de la CMC.

**12.** Préparation pharmaceutique, caractérisée en ce qu'elle contient des composés pyrimidium N-alkylés quaternaires de formule générale (II) sous forme de structures micellaires ou vésiculaires dans lesquelles sont enfermés des principes actifs pharmaceutiques, dispersées dans un solvant, le pH de la préparation étant ≦ 7, la concentration critique de formation de micelles (CMC) étant de $1,0 \times 10^{-7}$ à $1,5 \times 10^{-4}$ mol/l et la formule (II) étant :

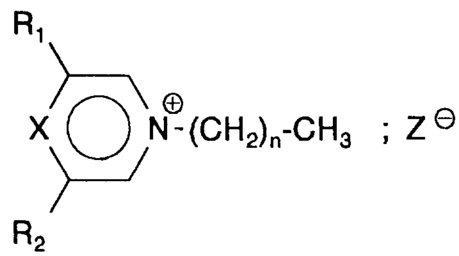

X = CH ; C - OH

X = C - $CH[(CH_2)_n - CH_3]_2$

$R_1$ =

H ; 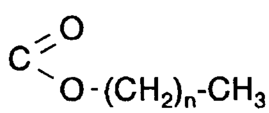 ; OH

$R_2$ =

H ; 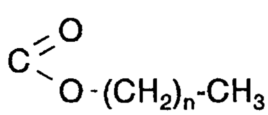 ; $CONH_2$ ; OH

dans laquelle

n = 8 à 20, en particulier 15 et
Z = chlorure, bromure, iodure, maléate, formiate, acétate, propionate, hydrogénosulfate, malate, fumarate, salicylate, alginate, gluconate, glucuronate, galacturonate, éthylsulfate ou hydrogénophosphate $H_2PO_4$-.

**13.** Préparation pharmaceutique selon la revendication 12, caractérisée en ce que les principes actifs sont des antibiotiques, des fongicides, des composés à action antivirale, des composés à action antinéoplasique et/ou des composés à action antiphlogistique.

**14.** Préparation pharmaceutique selon la revendication 12 ou 13, caractérisée en ce que les principes actifs sont un ou plusieurs des composé du groupe $ZnSO_4$, ZnO, ZnEDTA, $Hg(CN)_2$ et des antimonates d'acide tungstique.

**15.** Préparation pharmaceutique selon la revendication 12 ou 13, caractérisée en ce que les principes actifs sont un ou plusieurs des composés du groupe constitué des amphotéricine B,éconazol,clotrimazol,miconazol,cytarabine, idoxuridine et trifluorothymidine.

**16.** Préparation pharmaceutique selon la revendication 12 ou 13, caractérisée en ce que les principes actifs sont un ou plusieurs des composés du groupe ribavirine, amantadine, 1,1':3,3'-bis-cyclobutane et cubanes substitués par le groupe 1,1':3,3'-amino ou Hg.

**17.** Procédé pour la fabrication d'une préparation pharmaceutique selon la revendication 12, caractérisé en ce qu'un composé de formule (II) est dissous dans un solvant approprié et cette solution est traitée selon des procédés connus en soi pour obtenir une solution contenant des structures micellaires ou vésiculaires dans lesquelles les principes actifs sont incorporés.

**18.** Procédé selon la revendication 17, caractérisé en ce que, pour obtenir des structures micellaires ou vésiculaires , on passe par une étape de traitement aux ultra-sons.

**19.** Préparation pharmaceutique à activité antiphlogistique, caractérisée en ce qu'elle contient un composé de formule générale (II) de la revendication 12 comme principe actif.

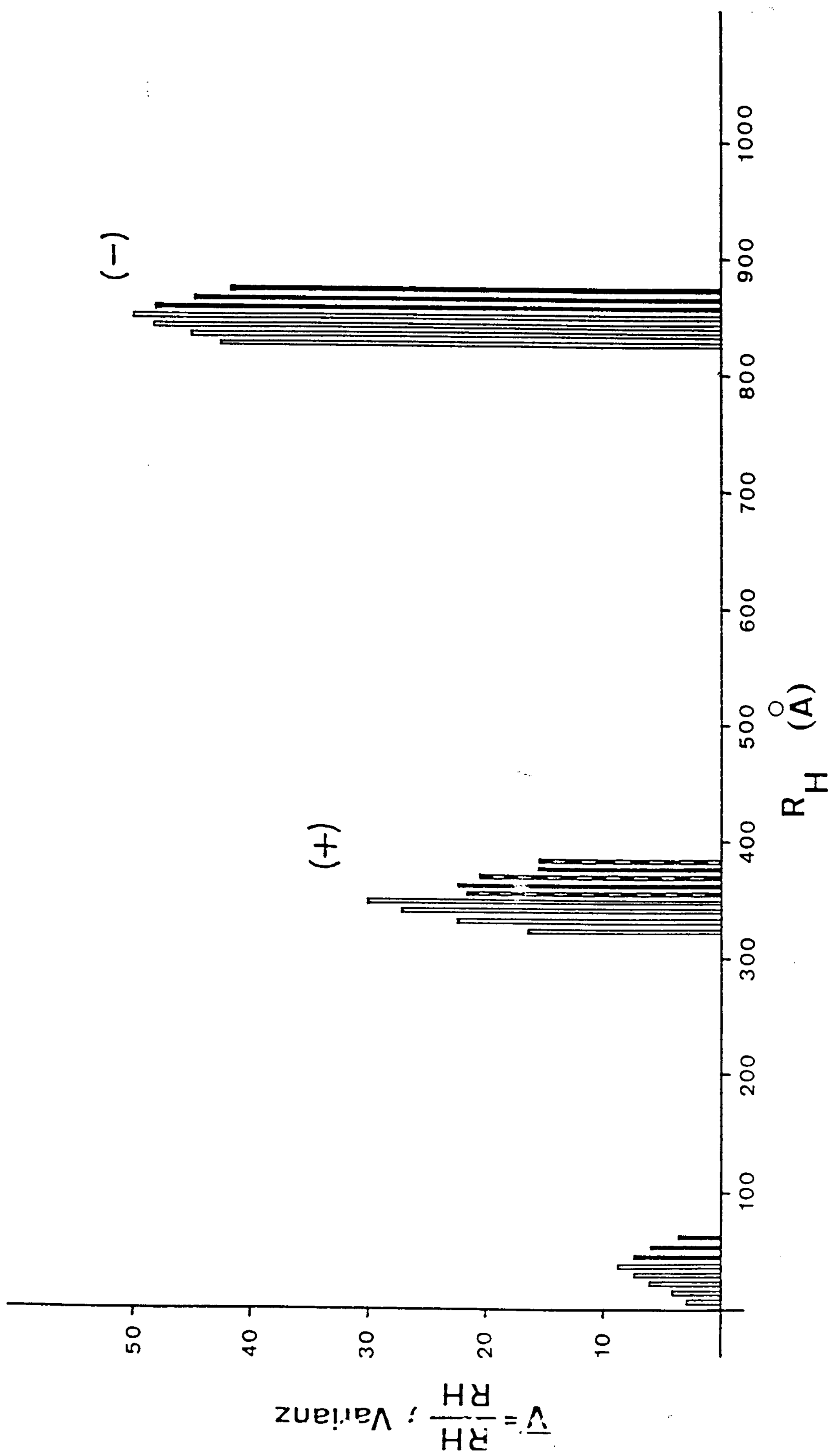
(−)
(+)
100
200
300
400
500
600
700
800
900
1000
$R_H$ (Å)
10
20
30
40
50
Varianz

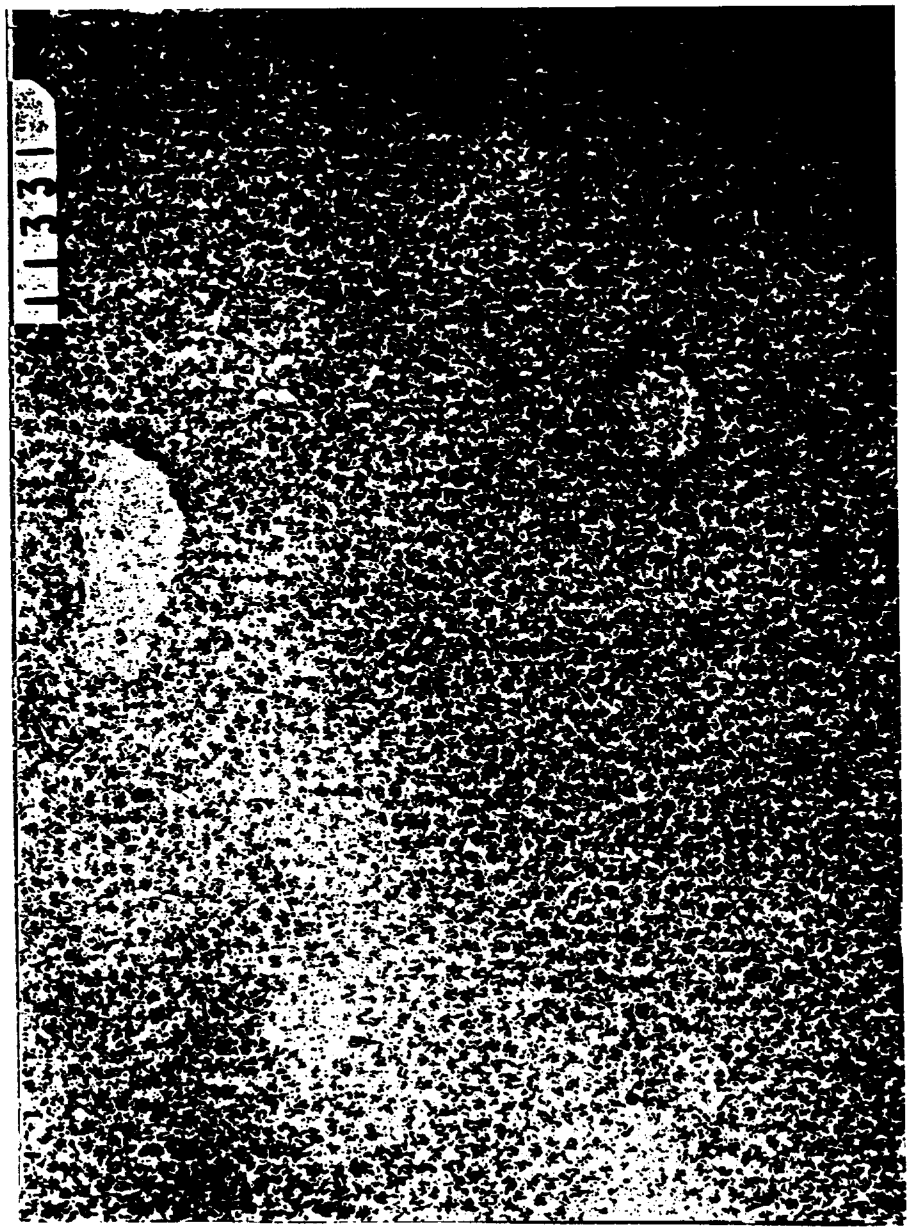

Abb. 2

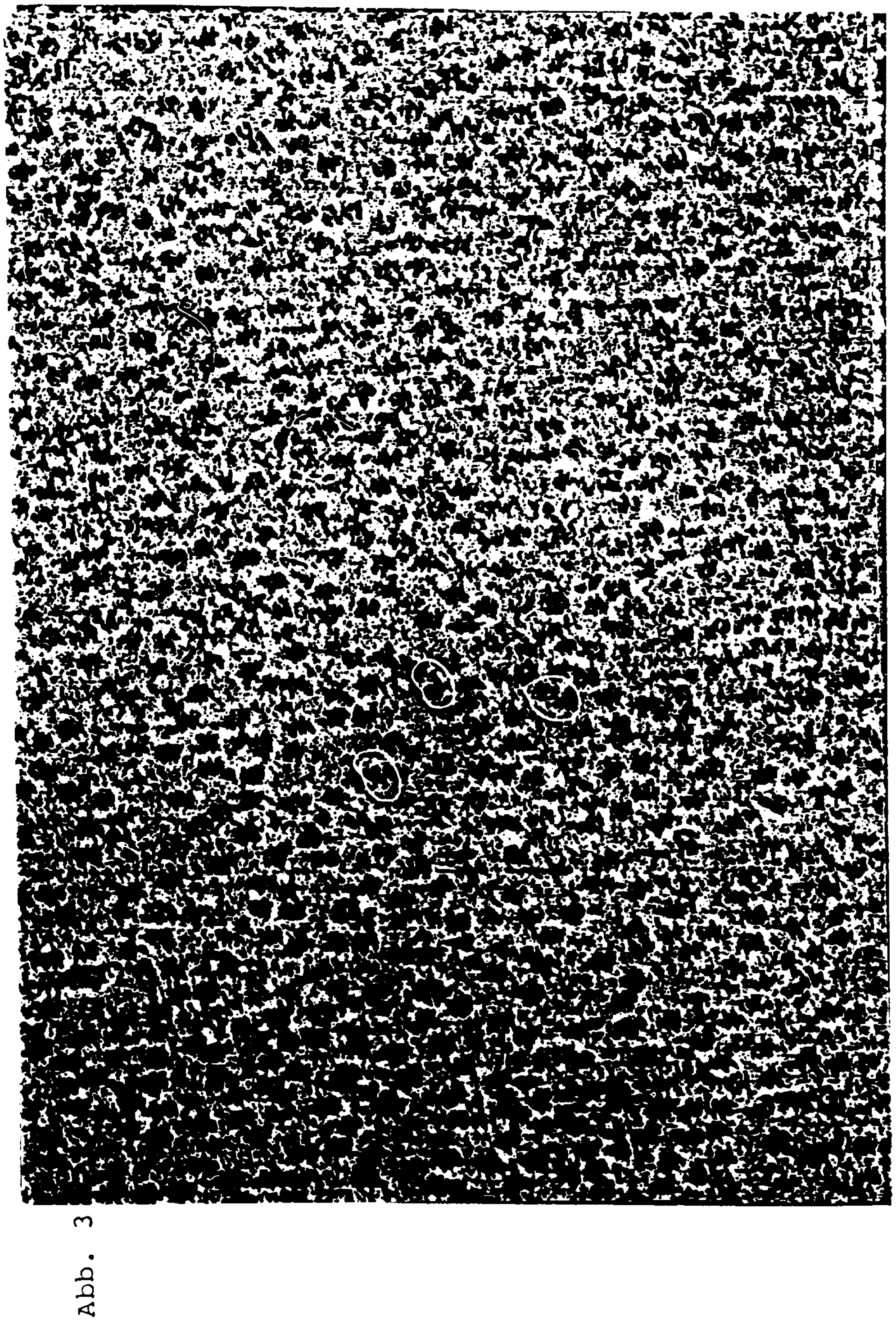

Abb. 3

Abb. 4

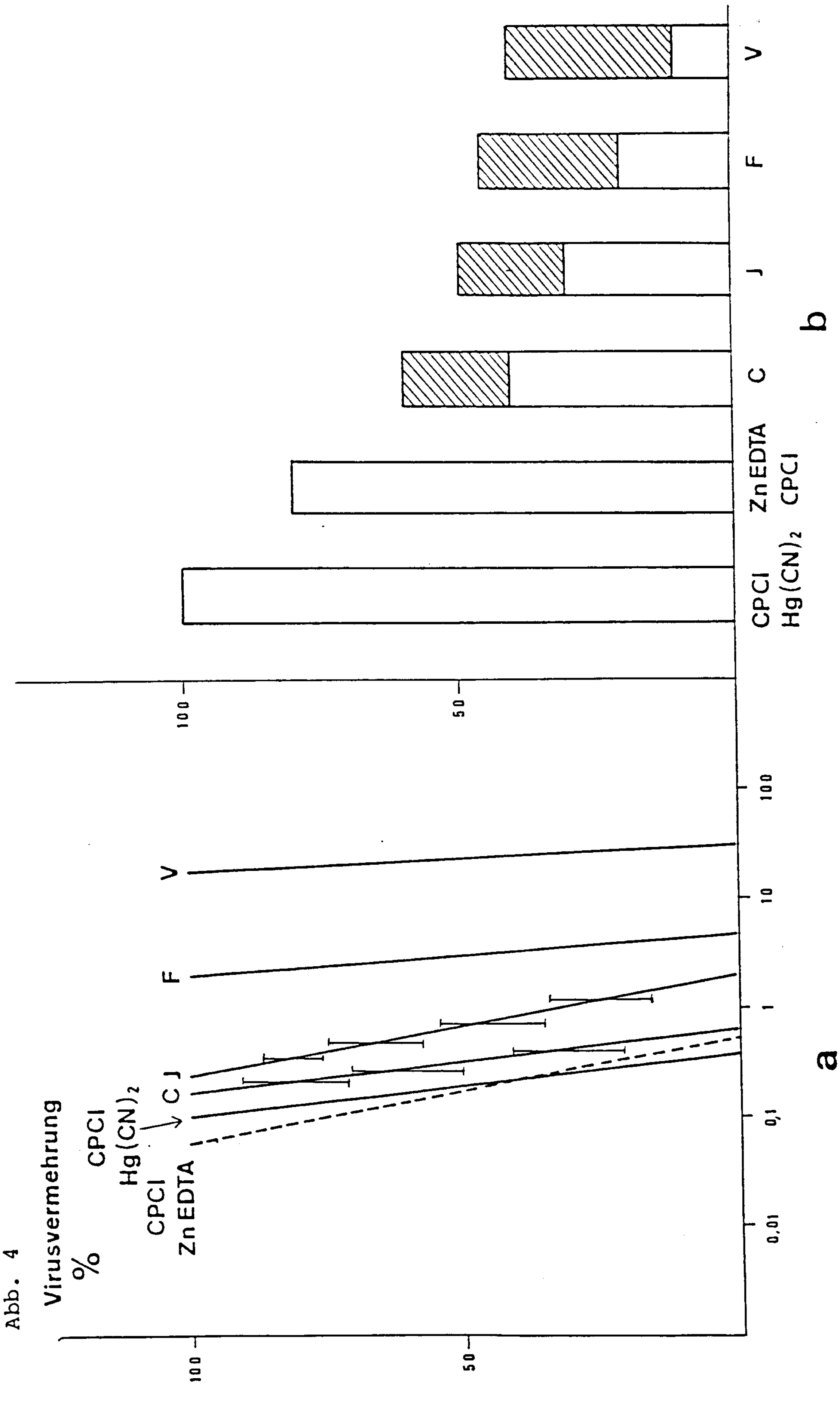
Virusvermehrung
%
CPCl
Hg(CN)$_2$
CPCl
ZnEDTA
C J
F
V
100
50
0,01
0,1
1
10
100
a
CPCl
Hg(CN)$_2$
ZnEDTA
CPCl
C
J
F
V
b

Abb. 5

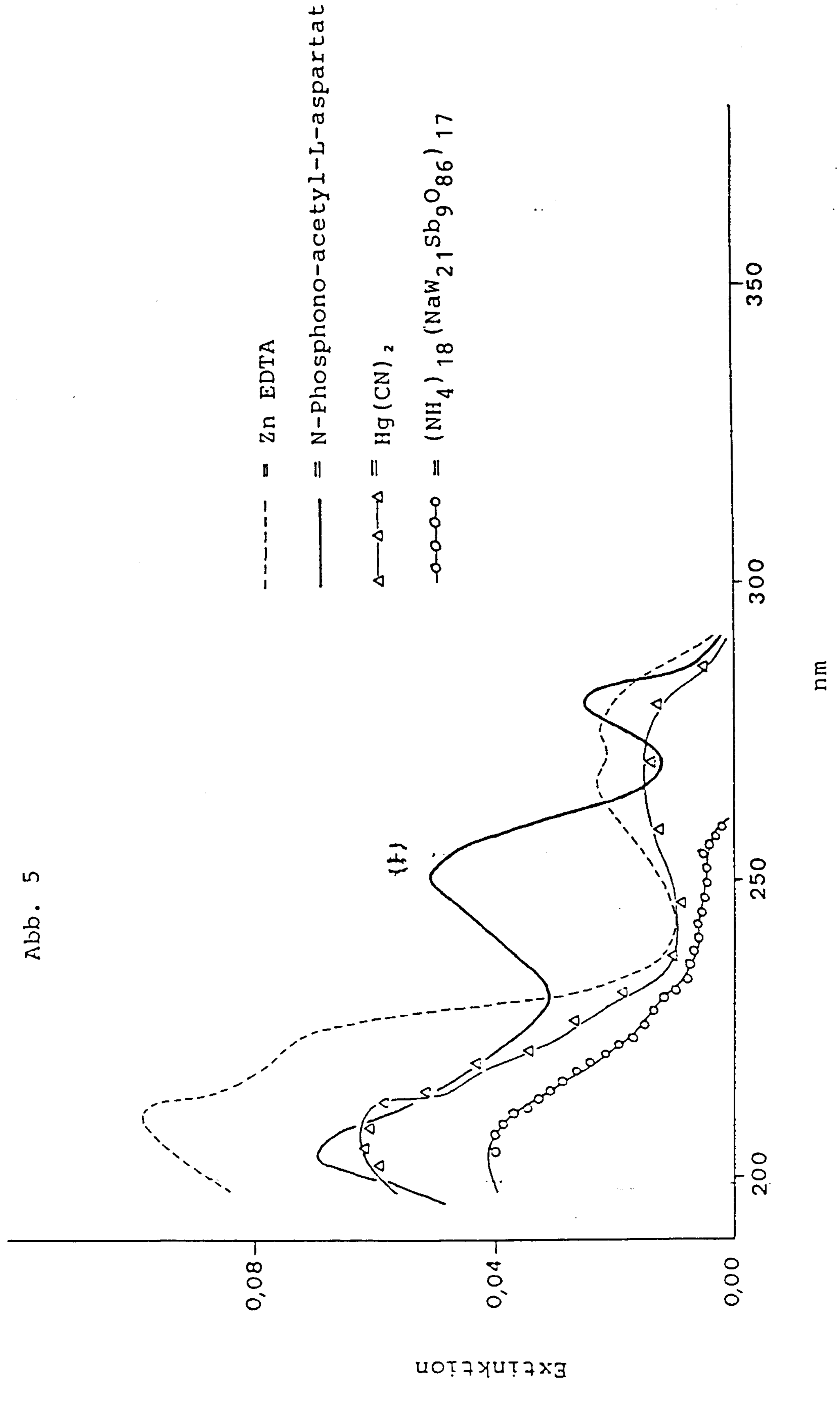
= Zn EDTA
= N-Phosphono-acetyl-L-aspartat
= Hg(CN)$_2$
= $(NH_4)_{18}(NaW_{21}Sb_9O_{86})_{17}$
(1)
Extinktion
0,08
0,04
0,00
200
250
300
350
nm

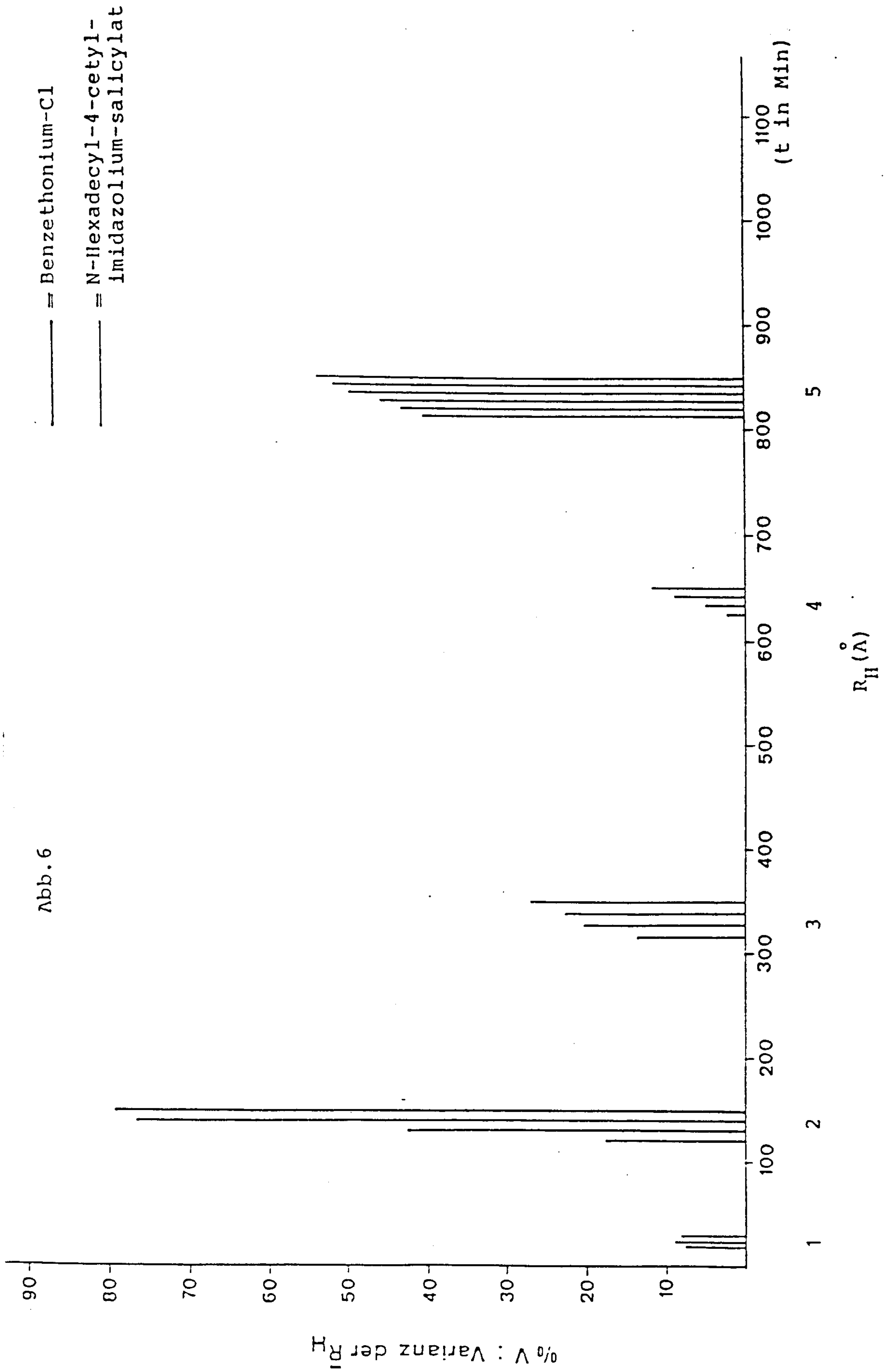
Abb.6
= Benzethonium-Cl
= N-Hexadecyl-4-cetyl-imidazolium-salicylat
% V : Varianz der R_H
10
20
30
40
50
60
70
80
90
1
2
3
4
5
100
200
300
400
500
600
700
800
900
1000
1100
(t in Min)
R_H (Å)

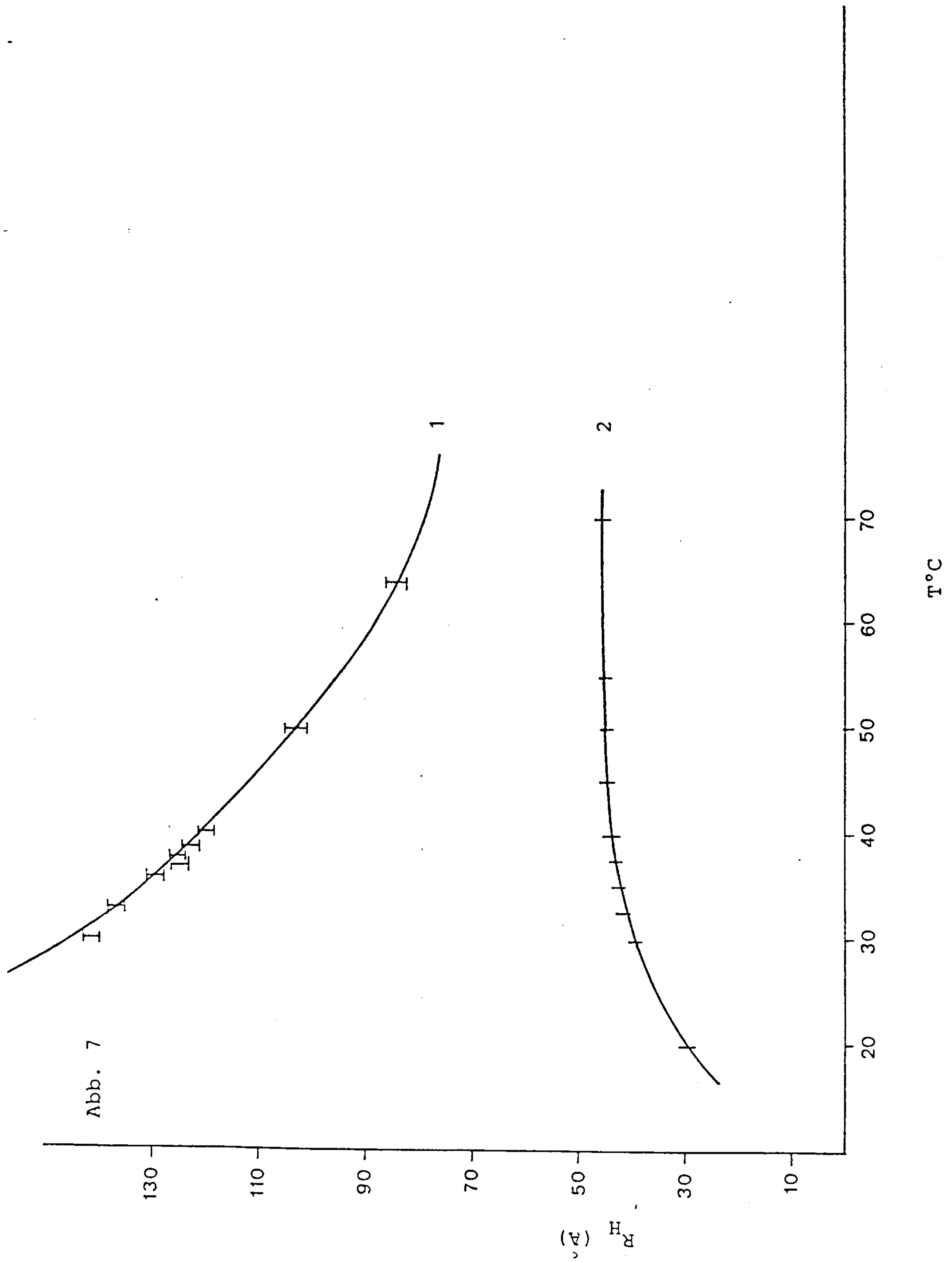
Abb. 7
1
2
130
110
90
70
50
30
10
R_H (Å)
20
30
40
50
60
70
T°C

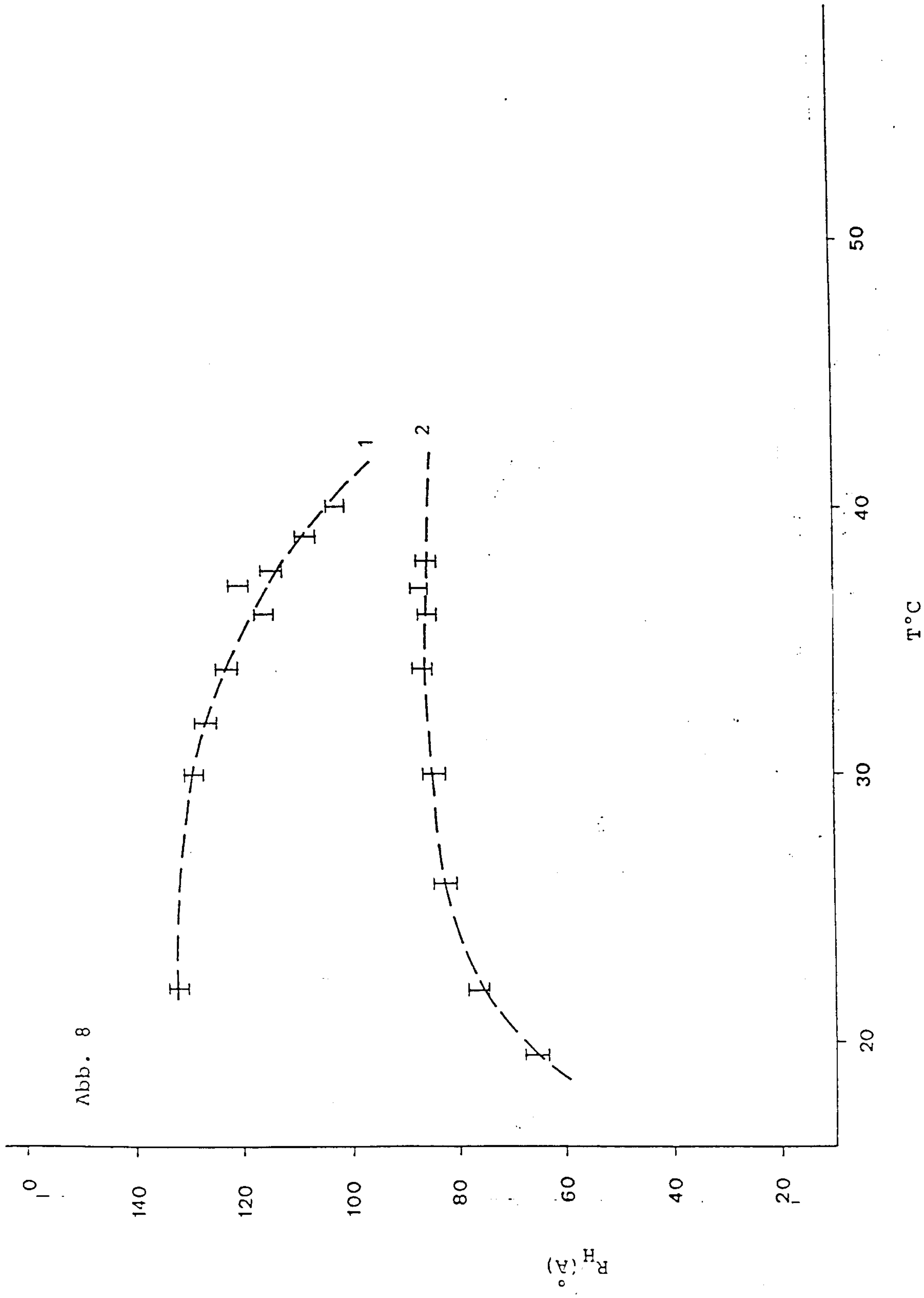
Abb. 8
1
2
T°C
20
30
40
50
R_H (Å)
0
140
120
100
80
60
40
20

Abb. 9

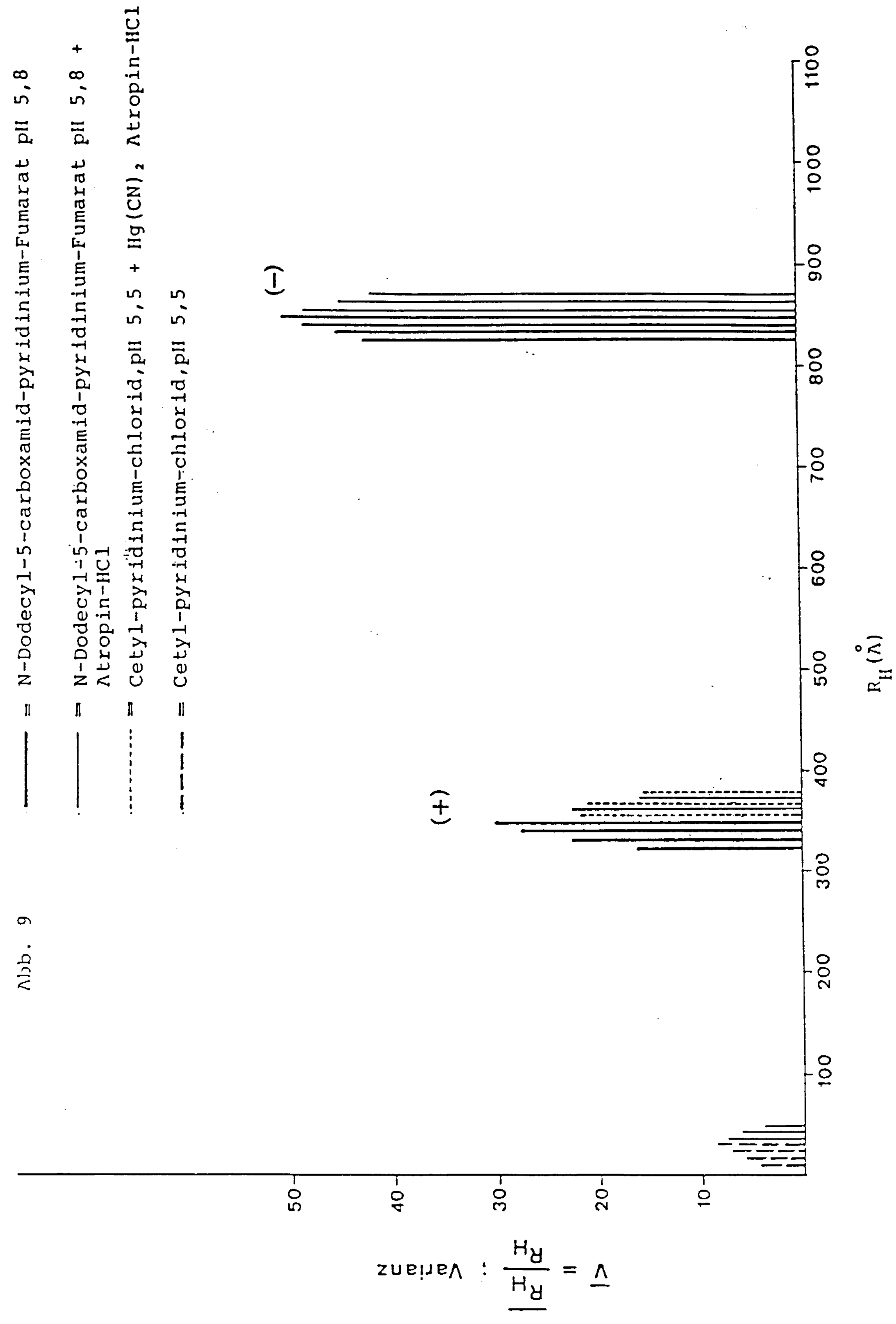

= N-Dodecyl-5-carboxamid-pyridinium-Fumarat pH 5,8
= N-Dodecyl-5-carboxamid-pyridinium-Fumarat pH 5,8 + Atropin-HCl
= Cetyl-pyridinium-chlorid, pH 5,5 + Hg(CN)₂ Atropin-HCl
= Cetyl-pyridinium-chlorid, pH 5,5
(-)
(+)
100
200
300
400
500
600
700
800
900
1000
1100
R_H (Å)
10
20
30
40
50
V = R_H / R_H : Varianz

Abb. 10

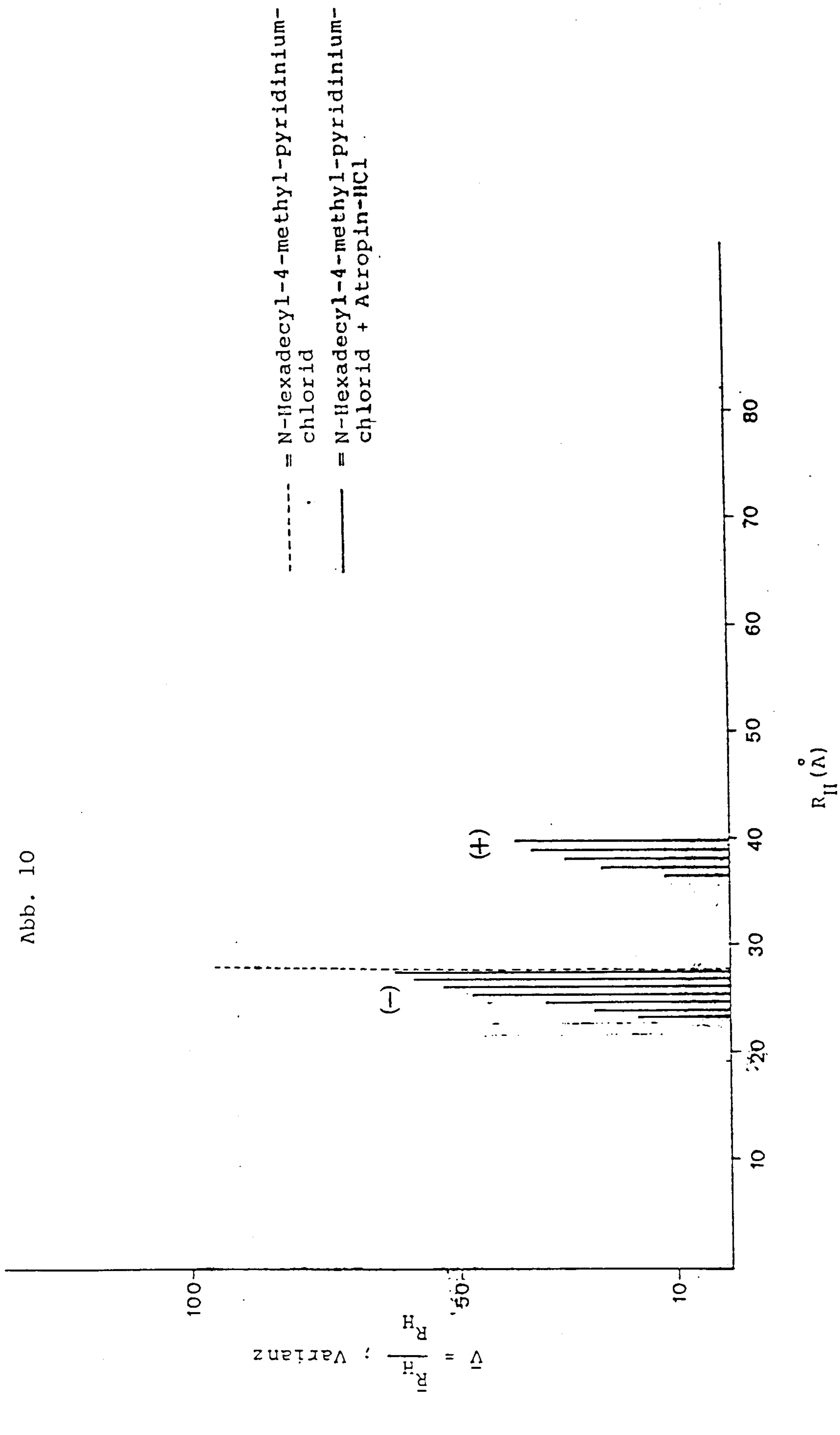

------- = N-Hexadecyl-4-methyl-pyridinium-chlorid
——— = N-Hexadecyl-4-methyl-pyridinium-chlorid + Atropin-HCl
(−)
(+)
$V = \frac{R_I}{R_H}$ ; Varianz
100
50
10
10
20
30
40
50
60
70
80
$R_{II}$ (Å)

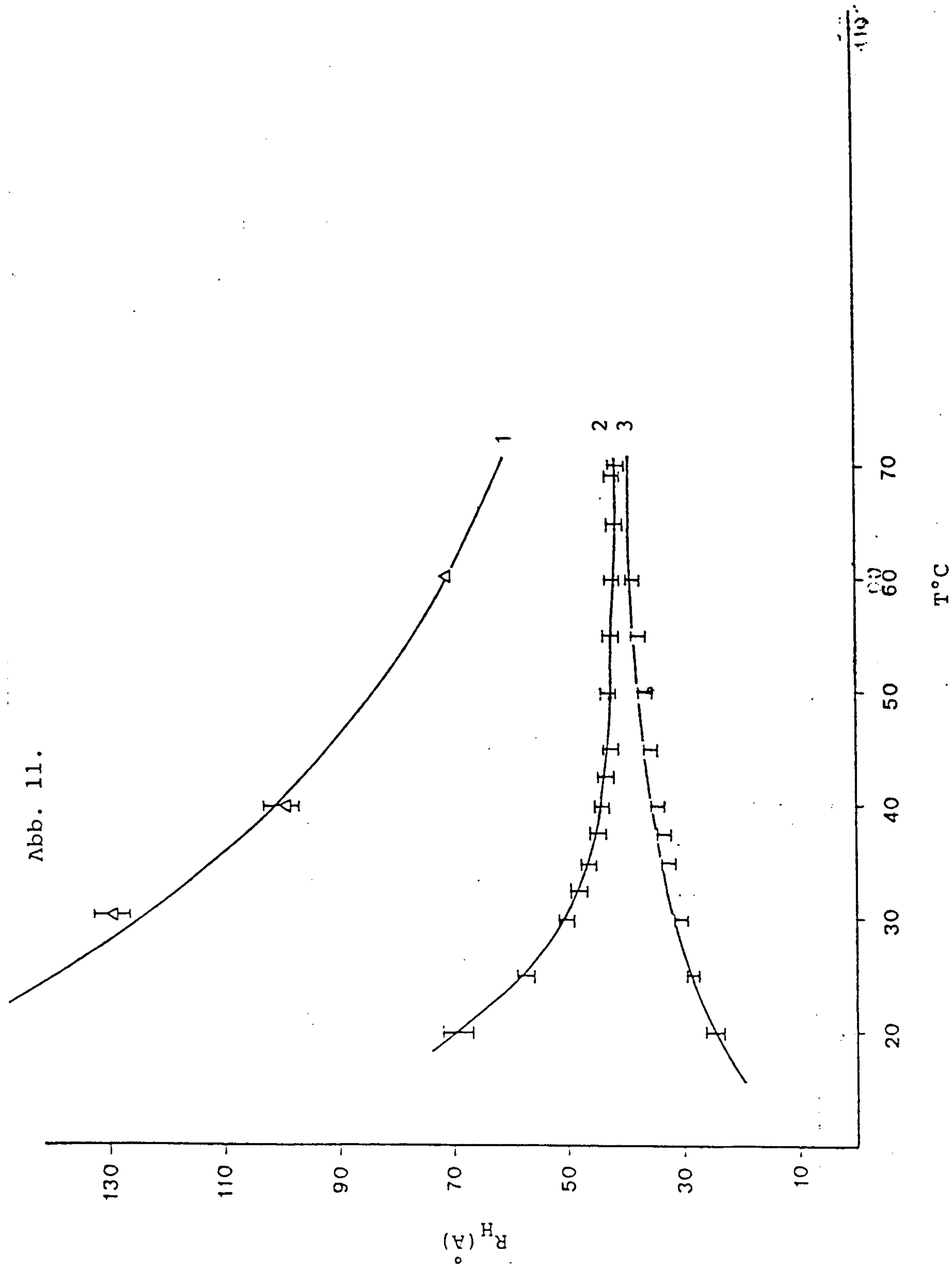

Abb. 11.

Abb.12

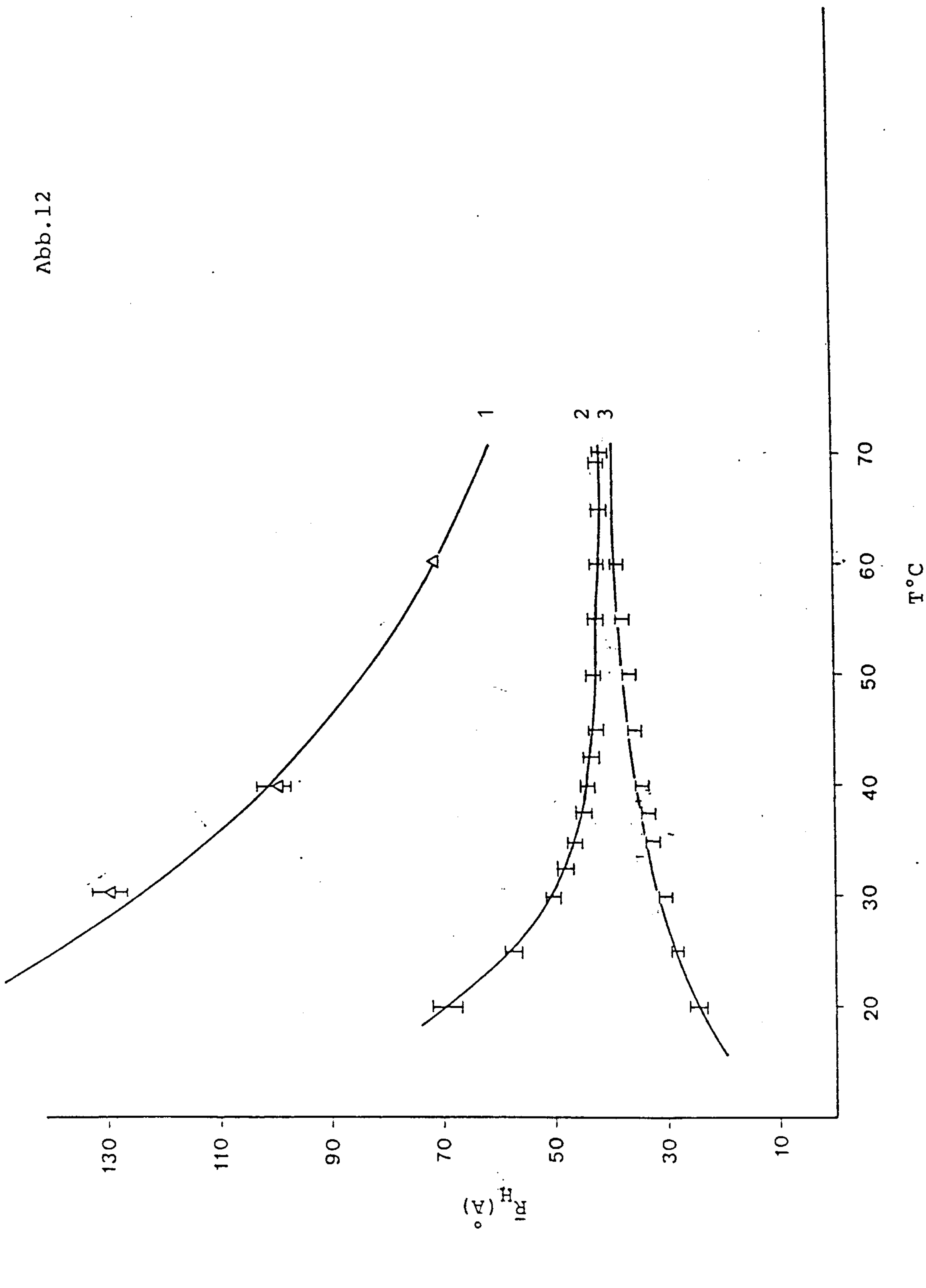

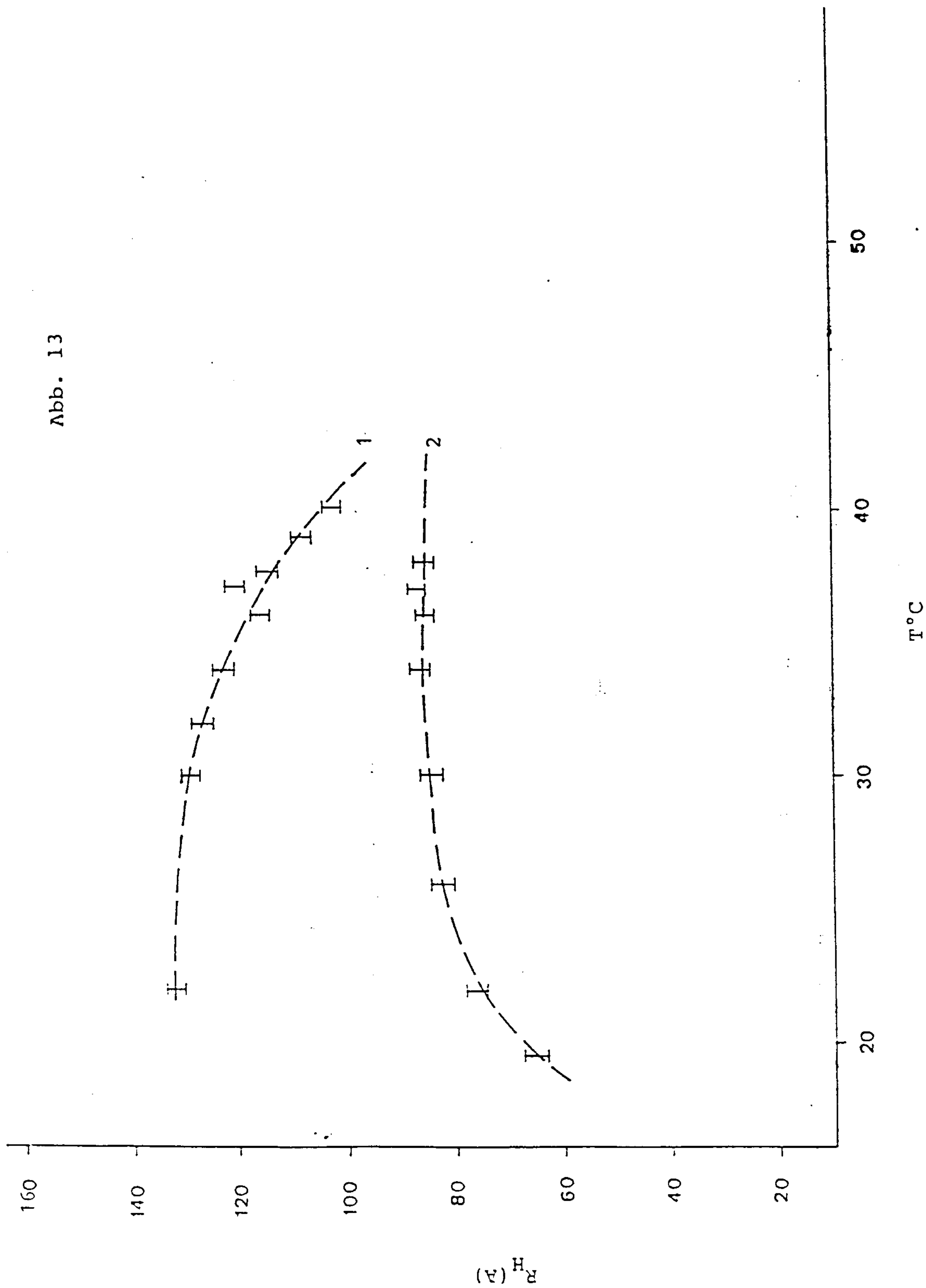


Abb. 13

Abb. 14

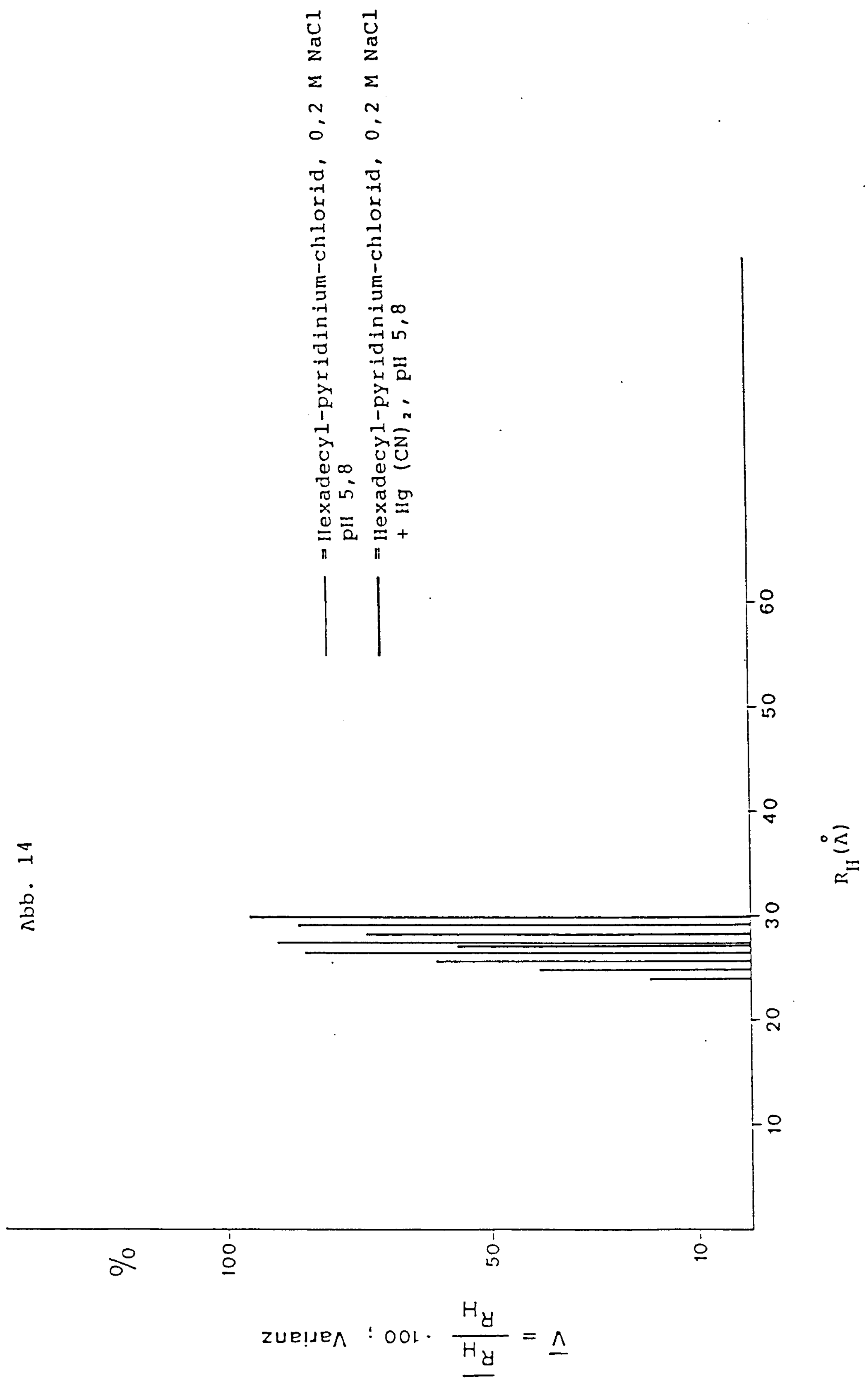

= Hexadecyl-pyridinium-chlorid, 0,2 M NaCl pH 5,8
= Hexadecyl-pyridinium-chlorid, 0,2 M NaCl + Hg (CN)₂, pH 5,8
10
20
30
40
50
60
R_H (Å)
%
100
50
10
V = |R_H| / R_H · 100 ; Varianz

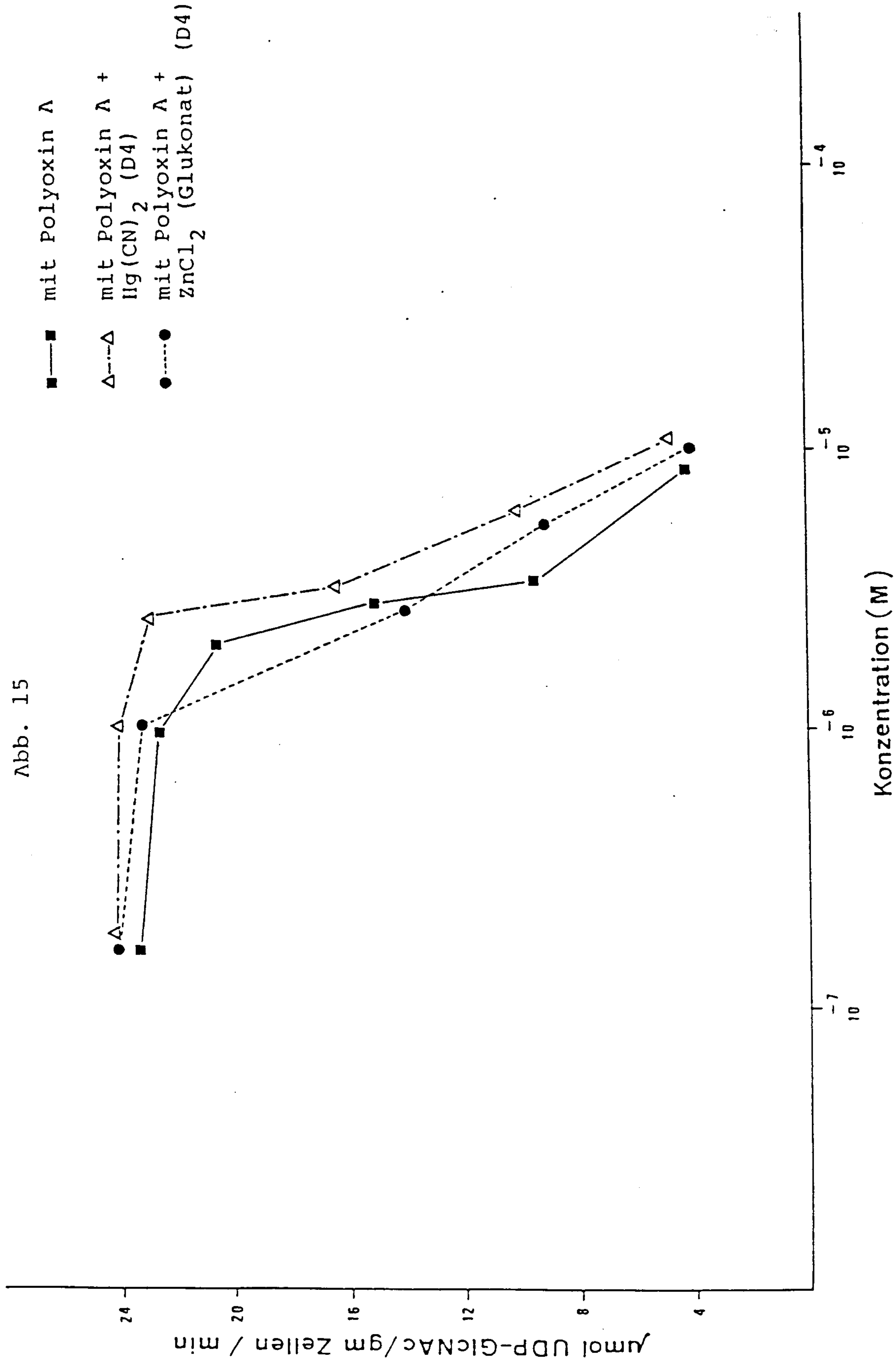
Abb. 15
mit Polyoxin A
mit Polyoxin A + Hg(CN)2 (D4)
mit Polyoxin A + ZnCl2 (Glukonat) (D4)
µmol UDP-GlcNAc/gm Zellen / min
24
20
16
12
8
4
10 -7
10 -6
10 -5
10 -4
Konzentration (M)

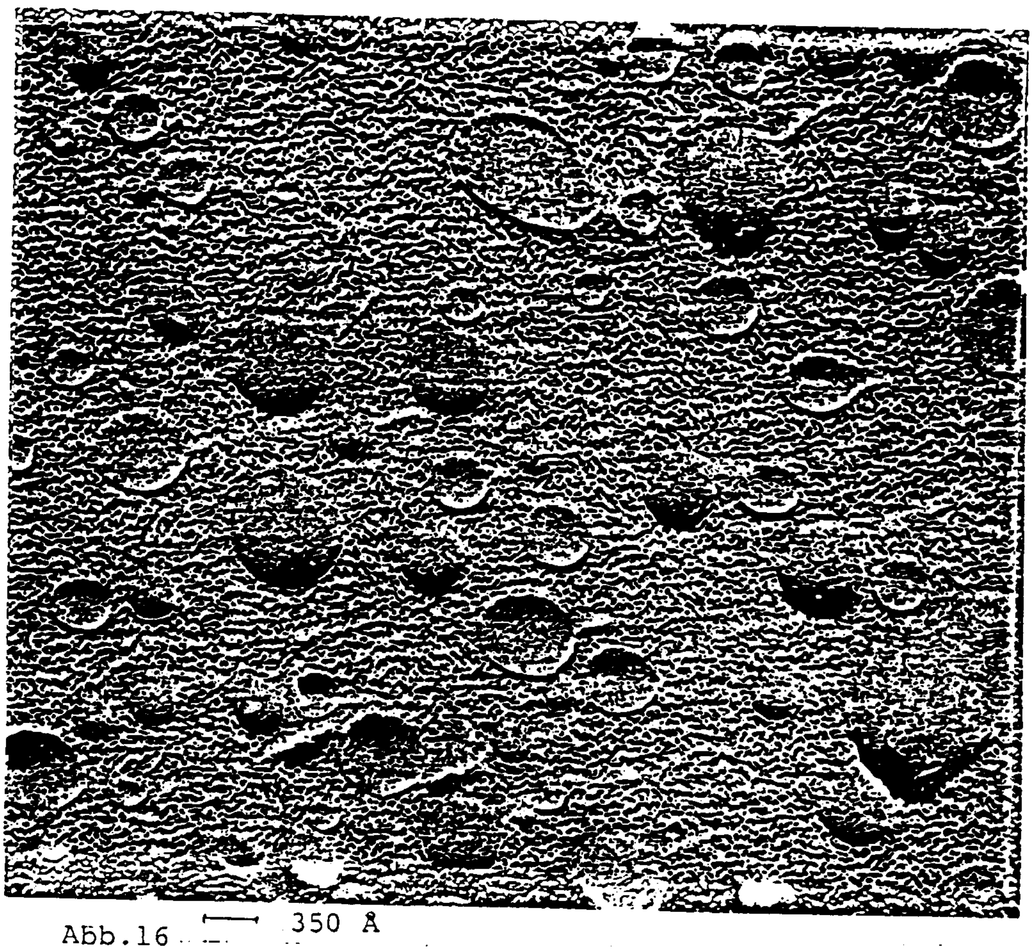

Abb.16 ⊢—⊣ 350 Å

"Freeze fracture" elektronenmikroskopische Aufnahme von Vesikeln des $N^+$-Tensides 3,5-bis (n-hexadecyloxy)carbonyl - N-methyl-pyridiniumchlorid einschließlich der eingeschlossenen Polyoxin A gemäß Arbeitsvorschrift.
Es zeigt die verschiedenen Größenordnungen der Vesikel, wenn nicht nach Größenordnung getrennt wird.